# FASCICULE DE BREVET EUROPEEN

(11) **EP 0 766 695 B1**
(45) Date de publication et mention de la délivrance du brevet: **22.07.1998**
(21) Numéro de dépôt: 95923397.4
(22) Date de dépôt: 16.06.1995
(51) Int. Cl.: C07K 5/06, A61K 31/40, C07D 207/12

(54) **DERIVES DE PYRROLIDINE POUR LE TRAITEMENT DES DESORDRES LIES A LA CCK ET LA GASTRINE**
PYRROLIDINDERIVATE ZUR BEHANDLUNG VON CCK UND GASTRIN-ABHÄNGIGEN ERKRANKUNGEN
PYRROLIDINE DERIVATIVES FOR THE TREATMENT OF CHOLECYSTOKININE AND GASTRINE-RELATED DISORDERS

(30) Priorité: 20.06.1994 FR 9407540
(43) Date de publication de la demande: 09.04.1997
(73) Titulaire: RHONE-POULENC RORER S.A., 92160 Antony (FR)
(72) Inventeur: CAPET, Marc, 91170 Viry Chaltillon (FR); DUBROEUCQ, Marie-Christine, 95880 Enghein-les-Bains (FR)
(74) Mandataire: Savina, Jacques
(86) Numéro de dépôt international: FR9500796
(87) Numéro de publication internationale: WO9535310

(56) Documents cités:
- EP-A- 0 160 436
- WO-A-93/01167

## Description

La présente invention concerne des dérivés de formule : 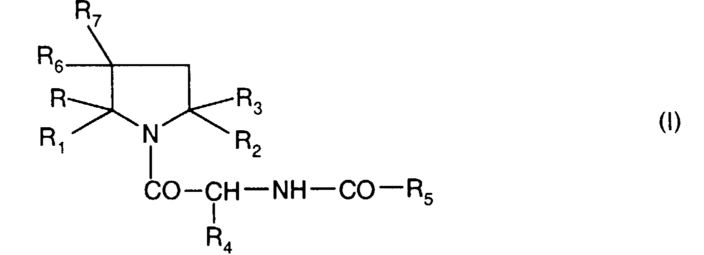 leurs sels, leur préparation et les médicaments les contenant.

De la demande de brevet WO93/01167, il est connu que des dérivés de pyrrolidine et de thiazolidine sont utiles comme antagonistes de la cholécystokinine. En outre de la demande de brevet EP160436, il est connu que des dérivés de N-(amino)alkyl-1-pyrrolidine, 1-pipéridine et 1-homopipéridinecarboxamides et thiocarboxamides sont utiles comme antiarrhytmiques.

Selon l'invention, dans la formule (I),
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₈R_{9,} -NH-CO-CH₃, trifluorométhyle ou trifluorométhoxy,
R₁ représente un atome d'hydrogène ou un radical alkyle,
R₂ représente une chaîne -(CH₂)ₙ-CO-R₁₀, -(CH₂)ₘ-O-CO-R₁₁, -(CH₂)ₘ-NR₁₂R₁₃ ou un radical oxazolinyle éventuellement substitué par un ou plusieurs radicaux alkyle ou alkyl-3 oxadiazolyle,
R₃ représente un atome d'hydrogène ou un radical alkyle,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 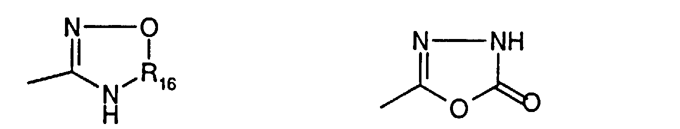 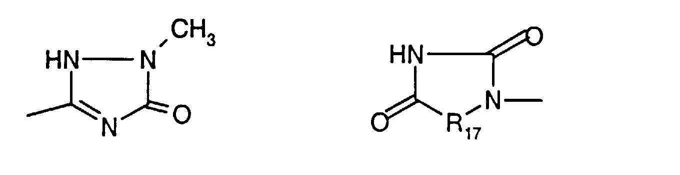 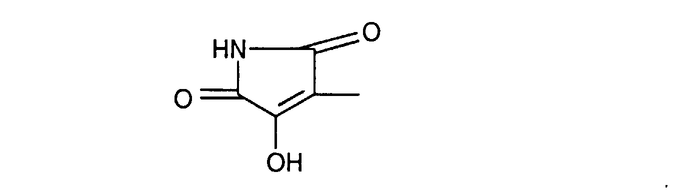 ou diméthyl-2,2 dioxo-4,6 dioxanne-1,3-yl-5,
R₆ représente un atome d'hydrogène ou un radical alkyle ou phénylalkyle,
R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy,
R₈ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₉ représente un radical alkyle, phénylalkyle ou phényle éventuellement substitué par un ou plusieurs substituants choisis parmi les atomes d'halogène et les radicaux alkyle, alcoxy et alkylthio,
ou bien R₈ et R₉ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₁₀ représente un radical hydroxy, alcoxy, cycloalkyloxy, cycloalkylalkyloxy, phényle ou -NR₁₂R₁₃,
R₁₁ représente un radical alcoxy, cycloalkyloxy, cycloalkylalkyloxy, phényle ou -NR₁₂R₁₃,
R₁₂ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₃ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₁₂ et R₁₃ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₄ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₅ représente un radical tétrazolyl-5,
R₁₆ représente C=O ou S=O,
R₁₇ représente O ou C=O,
n est égal à 0, 1 ou 2,
m est égal à 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R représente un radical alkyle insaturé, celui-ci est de préférence un radical isopropylidène.

Lorsque R représente un radical cycloalkyle, celui-ci est de préférence un radical cyclohexyle.

Lorsque R représente un radical cycloalkyle insaturé, celui-ci est de préférence un radical tétrahydrophényle, cyclopentadiène ou dihydrophényle.

Lorsque R représente un radical polycycloalkyle, celui-ci est de préférence un radical norbornyle ou adamantyle.

Lorsque R représente un radical polycycloalkyle insaturé, celui-ci est de préférence un radical norbornényle.

Lorsque R₈ et R₉ forment avec l'atome d'azote auquel ils sont rattachés un hétérocycle, celui-ci est de préférence un cycle pipéridino éventuellement substitué par un ou plusieurs radicaux alkyle, morpholino ou tétrahydro-1,2,3,4 quinoléine.

Lorsque R₁₂ et R₁₃ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle.

Les composés de formule (I) comportant un ou plusieurs centres asymétriques présentent des formes isomères. Ces isomères font également partie de l'invention.

Les composés de formule (I) pour lesquels R₅ représente un radical phénylamino dont le noyau phényle est éventuellement substitué peuvent être préparés par action d'un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 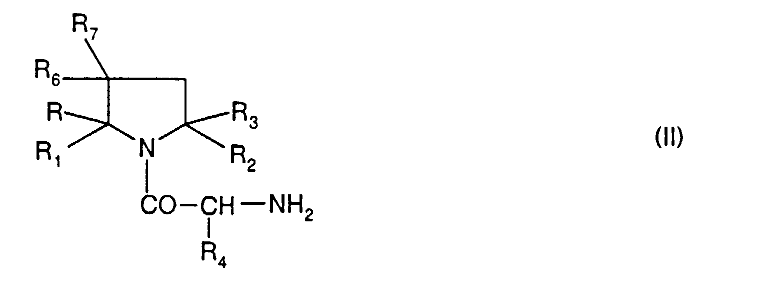 dans laquelle R, R₁, R₂, R₃, R₄, R₆ et R₇ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 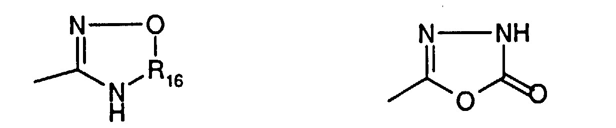 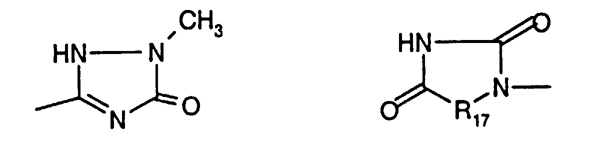 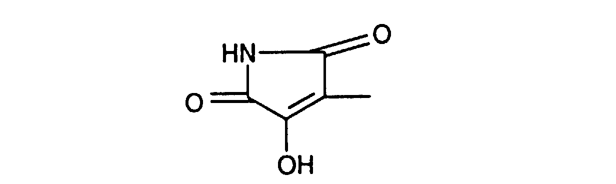 ou diméthyl-2,2 dioxo-4,6 dioxanne-1,3-yl-5, alk, alk', X, R₁₄, R₁₅, R₁₆ et R₁₇ ayant les mêmes significations que dans la formule (I).

Cette réaction s'effectue généralement au sein d'un solvant inerte tel que le tétrahydrofuranne, le N,N-diméthyl-formamide, un solvant chloré (chloroforme, dichloro-1,2 éthane par exemple) ou un solvant aromatique (benzène, toluène par exemple), à une température comprise entre 20°C et la température d'ébullition du solvant.

Le dérivé réactif de l'acide carbamique peut être obtenu dans les mêmes conditions de solvant et de température.

Les dérivés de formule (II) peuvent être obtenus par déprotection d'un dérivé de formule : 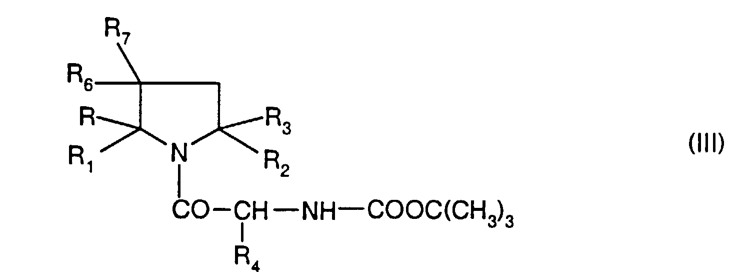 dans laquelle R, R₁, R₂, R₃, R₄, R₆ et R₇ ont les mêmes significations que dans la formule (I).

Cette déprotection s'effectue de préférence au moyen d'iodotriméthylsilane, au sein d'un solvant inerte tel qu'un solvant chloré (chloroforme, dichloro-1,2 éthane par exemple) ou l'acétonitrile, à une température comprise entre 15 et 40°C.

Les dérivés de formule (III) pour lesquels R₂ représente une chaîne -(CH₂)ₙ-CO-R₁₀, R₁₀ représente un radical alcoxy, cycloalkyloxy, cycloalkylalkyloxy, phényle ou -NR₁₂R₁₃ et R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, nitro, amino, monoalkylamino, dialkylamino, acylamino, trifluorométhyle, trifluorométhoxy peuvent être obtenus par action d'un dérivé de formule : 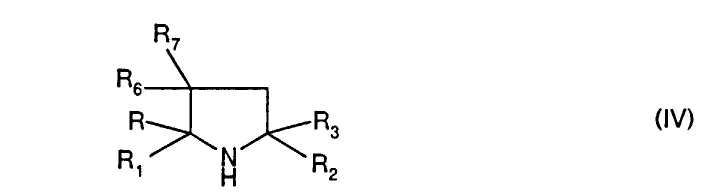 dans laquelle R, R₁, R₃ et R₆ ont les mêmes significations que dans la formule (I) et R₂ et R₇ ont les mêmes significations que précédemment, sur un acide de formule : 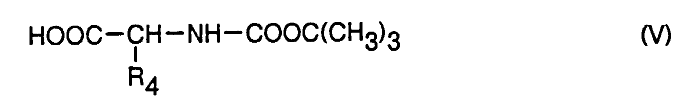 dans laquelle R₄ est défini comme dans la formule (I).

Cette réaction s'effectue au sein d'un solvant inerte tel que l'acétonitrile, le tétrahydrofuranne ou un solvant chloré, en présence d'un agent de condensation utilisé en chimie peptidique tel qu'un carbodiimide (N,N'-dicyclohexyl-carbodiimide par exemple) ou un chloroformiate d'alkyle, à une température comprise entre 10 et 40°C.

Les dérivés de formule (V) peuvent être obtenus selon les méthodes habituelles de protection des amino acides.

Les dérivés de formule (IV) pour lesquels R₂ représente une chaîne -(CH₂)ₙ-CO-R₁₀ et n est égal à 0 peuvent être obtenus par réaction d'un dérivé de formule : 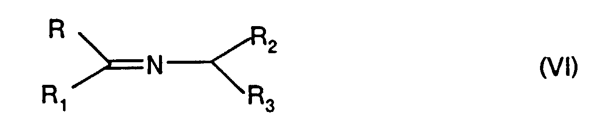 dans laquelle R, R₁ et R₃ ont les mêmes significations que dans la formule (I) et R₂ représente une chaîne -(CH₂)ₙ-CO-R₁₀, n est égal à 0, R₁₀ représente un radical alcoxy, cycloalkyloxy, cycloalkylalkyloxy, phényle ou -NR₁₂R₁₃ et R₁₂ et R₁₃ ont les mêmes significations que dans la formule (I), sur un dérivé de formule : 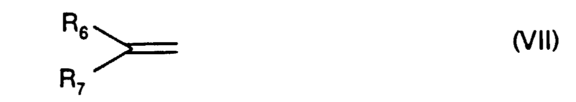 dans laquelle R₆ a les mêmes significations que dans la formule (I) et R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, nitro, amino, monoalkylamino, dialkylamino, acylamino, trifluorométhyle, trifluorométhoxy, R₈ et R₉ ont les mêmes significations que dans la formule (I).

Cette réaction s'effectue au sein d'un solvant inerte tel que l'acétonitrile, le tétrahydrofuranne, le toluène, en présence d'un sel métallique tel que l'acétate d'argent, le bromure de lithium, le bromure de magnésium, l'iodure de sodium, l'iodure de zinc, et d'une base azotée telle que la triéthylamine, à une température comprise entre 0°C et la température d'ébullition du milieu réactionnel.

Les dérivés de formule (VII) sont commercialisés ou peuvent être obtenus par application ou adaptation des méthodes décrites par N. O. BRACE, J. Org. Chem., 58, 4506 (1993), B.E. LOVE, Li Chao Syn. Commun., 23, 3073 (1993) ou J. CHANET-REY et coll., Heterocycles, 26, 101 (1987).

Les dérivés de formule (VI) peuvent être obtenus par action d'une cétone R-CO-R₁ dans laquelle R et R₁ ont les mêmes significations que dans la formule (I), sur une amine H₂N-CH(R₂)R₃ dans laquelle R₂ a les mêmes significations que dans la formule (VI) et R₃ a les mêmes significations que dans la formule (I).

Cette réaction s'effectue généralement soit au moyen d'un agent déshydratant tel que le tamis moléculaire 4 Å, au sein d'un solvant inerte tel que le dichlorométhane, à une température comprise entre 0°C et la température d'ébullition du milieu réactionnel ou bien par distillation azéotropique de l'eau dans un solvant aromatique tel que le toluène, en présence éventuellement d'un acide tel que l'acide paratoluènesulfonique.

Les dérivés de formule (IV) pour lesquels R₂ représente une chaîne -(CH₂)ₙ-CO-R₁₀, n est égal à 0, R₇ représente un radical phénylsulfonyle substitué peuvent également être obtenus par adaptation des méthodes décrites par S. KANEMASA et coll., Bull. Chem. Soc. Japan, 62, 869 (1982); D. A. BARR et coll., J. Chem. Soc. Perkin. Trans. 1, 1550 (1989) et O. TSUGE et coll., J. Org. Chem., 53, 1384 (1988).

Les dérivés de formule (IV) pour lesquels R₂ représente une chaîne -(CH₂)ₙ-CO-R₁₀ et n est égal à 1 ou 2 peuvent être obtenus par adaptation des méthodes décrites par S. ROSSET et coll., Tetrahedron Lett., 32, 7521 (1991); T. GALLAGHER et coll., J. Chem. Soc. Perkin Trans. 1, 2193 (1991) et J. F. W. KEANA, J. Org. Chem., 48, 2644 (1983).

Les dérivés de formule (IV) pour lesquels R₂ représente un radical -(CH₂)ₙ-CO-R₁₀ et R₁₀ représente un radical -NR₁₂R₁₃ peuvent également être obtenus par action d'une amine HNR₁₂R₁₃ dans laquelle R₁₂ et R₁₃ ont les mêmes significations que dans la formule (I) sur un dérivé de formule (IV) correspondant pour lequel R₂ représente un radical -(CH₂)ₙ-CO-R₁₀ et R₁₀ représente un radical hydroxy selon les méthodes décrites dans les exemples. On opère généralement en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel. Il peut être nécessaire d'introduire un groupe protecteur de la fonction amine du composé de formule (IV) tel que ceux décrits par T. W. GREENE, protective Groups in Organic Synthesis, John Wiley and Sons, New York.

Les dérivés de formule (IV) pour lesquels R₂ représente un radical -(CH₂)ₙ-CO-R₁₀ et R₁₀ représente un radical hydroxy peuvent également être obtenus par hydrolyse d'un dérivé de formule (IV) correspondant pour lequel R₂ représente un radical -(CH₂)ₙ-CO-R₁₀ et R₁₀ représente un radical alcoxy. Il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C.

Les dérivés de formule (III) pour lesquels R₂ représente une chaîne -(CH₂)ₙ-CO-R₁₀ et R₁₀ représente un radical hydroxy peuvent être obtenus par hydrolyse ou, selon le cas, hydrogénolyse des esters correspondants de formule (III).

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C. Lorsque l'on utilise des esters de phénylalkyle, il peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle.

Les dérivés de formule (III) pour lesquels R₂ représente une chaîne -(CH₂)ₘ-O-CO-R₁₁ peuvent être obtenus par réaction d'un dérivé de formule : 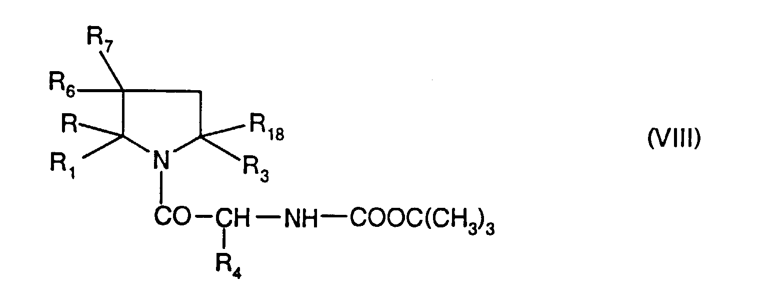 dans laquelle R, R₁, R₃, R₄, R₆ et R₇ ont les mêmes significations que dans la formule (I) et R₁₈ représente une chaîne -(CH₂)ₘ-OH, soit sur un halogénure de formule Hal-CO-R₁₁ dans laquelle Hal représente un atome d'halogène et R₁₁ a les mêmes significations que dans la formule (I) soit sur un anhydride de formule (R₁₁CO)₂O dans laquelle R₁₁ a les mêmes significations que dans la formule (I).

Cette réaction s'effectue au sein d'un solvant inerte tel qu'un solvant chloré, en présence d'une trialkylamine, à une température comprise entre 20°C et la température d'ébullition du milieu réactionnel.

Les dérivés de formule (VIII) peuvent être obtenus par réduction d'un dérivé de formule (III) correspondant pour lequel R₂ représente une chaîne -(CH₂)ₙ-CO-R₁₀, n est égal à 0 ou 1 et R₁₀ représente un radical hydroxy ou alcoxy.

Cette réaction s'effectue au sein d'un alcool (méthanol, éthanol, tertiobutanol), le tétrahydrofuranne ou un mélange de ces solvants, en présence de borohydrure de sodium ou de diborane, à une température comprise entre 20°C et la température d'ébullition du milieu réactionnel.

Les dérivés de formule (III) pour lesquels R₂ représente une chaîne -(CH₂)ₘ-O-CO-R₁₁, R₁₁ représente un radical -NR₁₂R₁₃ et R₁₂ représente un atome d'hydrogène peuvent également être obtenus par condensation d'un dérivé de formule (VIII) dans laquelle R₁₈ représente une chaîne -(CH₂)ₘ-OH sur un isocyanate de formule R₁₃NCO dans laquelle R₁₃ a les mêmes significations que dans la formule (I).

Cette réaction s'effectue au sein d'un solvant inerte tel qu'un solvant chloré, le tétrahydrofuranne ou le N,N-diméthyl-formamide, éventuellement en présence d'un quantité catalytique d'un alcoxyde de métal alcalin, à une température comprise entre 20°C et la température d'ébullition du milieu réactionnel.

Les dérivés de formule (III) pour lesquels R₂ représente un radical -(CH₂)ₘ-NR₁₂R₁₃ peuvent être obtenus par action d'une amine HNR₁₂R₁₃ dans laquelle R₁₂ et R₁₃ ont les mêmes significations que dans la formule (I) sur un dérivé de formule (VIII) dans laquelle R₁₈ représente un radical -(CH₂)ₘ-O-SO₂-CH₃.

Cette réaction s'effectue généralement soit en présence d'un large excès d'amine, à une température comprise entre 0 et 10°C soit, lorsque l'on utilise le chlorhydrate de l'amine, au sein d'un solvant chloré, en présence d'une trialkylamine, à une température comprise entre 20°C et la température d'ébullition du milieu réactionnel.

Les dérivés de formule (VIII) dans laquelle R₁₈ représente un radical -(CH₂)ₘ-O-SO₂-CH₃ peuvent être obtenus par réaction d'un dérivé de formule (VIII) correspondant pour lequel R₁₈ représente un radical -(CH₂)ₘ-OH avec le chlorure de méthanesulfonyle.

Cette réaction s'effectue généralement au sein d'un solvant inerte tel que l'acétonitrile ou le chlorure de méthylène, en présence de triéthylamine, à une température comprise entre 0°C et la température d'ébullition du milieu réactionnel.

Les dérivés de formule (III) pour lesquels R₂ représente un radical -(CH₂)ₙ-CO-R₁₀, R₁₀ représente un radical hydroxy peuvent être obtenus par saponification d'un dérivé de formule (III) correspondant pour lequel R₁₀ représente un radical alcoxy.

Cette réaction s'effectue au sein de solvants inertes tels que le méthanol, le dioxanne, le tétrahydrofuranne et l'eau ou un mélange de ces solvants, en présence d'une base telle que la soude, la potasse, l'hydroxyde de lithium, à une température comprise entre 0 et 25°C.

Les dérivés de formule (III) pour lesquels R₂ représente un radical -(CH₂)ₙ-CO-R₁₀ et R₁₀ représente un radical alcoxy, cycloalcoxy ou cycloalkylalkyloxy peuvent être obtenus par estérification des dérivés de formule (III) correspondants pour lesquels R₂ représente un radical -(CH₂)ₙ-CO-R₁₀ et R₁₀ représente un radical hydroxy.

Cette réaction s'effectue de préférence au moyen d'un alcool R₁₉-OH dans lequel R₁₉ représente un radical alkyle, cycloalkyle, cycloalkylalkyle, en présence de chlorure de tosyle, au sein de la pyridine, à une température comprise entre 0 et 25°C.

Les dérivés de formule (III) pour lesquels R₂ représente un radical -(CH₂)ₙ-CO-R₁₀ et R₁₀ représente un radical phényle peuvent être obtenus par action d'un dérivé de formule (III) correspondant pour lequel R₂ représente un radical -(CH₂)ₙ-CO-R₁₀ et R₁₀ représente un radical alcoxy avec le bromure de phénylmagnésium.

Cette réaction s'effectue de préférence au sein d'un solvant inerte tel que le tétrahydrofuranne ou l'éther éthylique, à une température comprise entre -70°C et la température d'ébullition du mélange réactionnel.

Les dérivés de formule (III) pour lesquels R₂ représente un radical oxazolinyle éventuellement substitué peuvent être obtenus par action d'un dérivé de formule (III) correspondant pour lequel R₂ représente un radical -(CH₂)ₙ-CO-R₁₀, n est égal à 0 et R₁₀ représente un radical hydroxy, sur l'amino-2 éthanol éventuellement substitué par un ou plusieurs radicaux alkyle.

Cette réaction s'effectue au sein d'un solvant inerte tel que le toluène en éliminant l'eau formée, à la température d'ébullition du milieu réactionnel.

Les dérivés de formule (III) pour lesquels R₂ représente un radical alkyl-3 oxadiazolyle peuvent être obtenus par action d'un dérivé de formule (III) correspondant pour lequel R₂ représente un radical -(CH₂)ₙ-CO-R₁₀, n est égal à 0 et R₁₀ représente un radical alcoxy sur une alkylamidoxime.

Cette réaction s'effectue au sein d'un solvant inerte tel que le tétrahydrofurane, en présence d'hydrure de sodium, à une température comprise entre 25°C et la température d'ébullition du milieu réactionnel.

Les dérivés de formule (III) pour lesquels R₂ représente une chaîne -(CH₂)ₙ-CO-R₁₀ et R₁₀ représente un radical -NR₁₂R₁₃ peuvent être obtenus par action d'un dérivé de formule (III) correspondant pour lequel R₁₀ représente un radical hydroxy ou un dérivé réactif de cet acide sur une amine de formule HNR₁₂R₁₃ dans laquelle R₁₂ et R₁₃ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (N,N-diméthyl-formamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. Fr, 1511 (1970); W.A. JACOBS et coll., J. Am. Chem. Soc., 39, 2438 (1917) et J. Am. Chem. Soc., 39, 1438 (1917) et dans les exemples.

Les composés de formule (I) pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle peuvent également être préparés par action d'un dérivé de formule (Il) dans laquelle R, R₁, R₂, R₃, R₄, R₆ et R₇ ont les mêmes significations que dans la formule (I), sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle et alk et alk' ont les mêmes significations que dans la formule (I).

Cette réaction s'effectue généralement au sein d'un solvant inerte tel que le tétrahydrofuranne, le N,N-diméthyl-formamide, un solvant chloré (chloroforme, dichloro-1,2 éthane par exemple), un solvant aromatique (benzène, toluène par exemple), à une température comprise entre 10°C et la température d'ébullition du solvant.

Les phénylisocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les composés de formule (I) pour lesquels R₅ représente un radical phényle éventuellement substitué, naphtyle, indolyle, quinolyle peuvent être préparés par action d'un dérivé de formule (II) dans laquelle R, R₁, R₂, R₃, R₄, R₆ et R₇ ont les mêmes significations que dans la formule (I), sur un acide de formule HOOC-R₅ dans laquelle R₅ a les mêmes significations que précédemment ou un dérivé réactif de cet acide.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (N,N-diméthyl-formamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo[5.4.0]undécène-7 ou diaza-1,5 bicyclo[4.3.0]nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalino-terreux à une température comprise entre 0 et 40°C.

Les composés de formule (I) pour lesquels R₅ représente un radical phénylamino dont le noyau phényle est substitué par un radical carboxy, -alk-COOX, -O-alk-COOX, -alk'-COOX, -CH=CH-COOX, -CO-COOX, -S-alk-COOX, -SO-alk-COOX, -SO₂-alk-COOX, -C(=NOH)-COOX, -O-CH₂-alk'-COOX, -CX=N-O-alk-COOX et X représente un atome d'hydrogène peuvent également être préparés par hydrolyse ou, selon le cas, hydrogénolyse des esters correspondants de formule (I) pour lesquels X représente un radical alkyle ou phénylalkyle.

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C. Lorsque l'on utilise des esters de phénylalkyle, il est peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle. Lorsqu'on utilise des esters de tert-butyle, il est avantageux d'effectuer l'hydrolyse au moyen d'un acide tel que l'acide trifluoroacétique.

Les composés de formule (I) pour lesquels R₅ représente un radical phénylamino dont le noyau phényle est substitué par un radical hydroxyiminoalkyle ou alcoxyiminoalkyle peuvent également être préparés par action du composé de formule (I) correspondant pour lequel R₅ représente un radical phénylamino dont le noyau phényle est substitué par un radical acyle sur un dérivé de formule :

H₂N-OR₂₀ (IX)

dans laquelle R₂₀ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les composés de formule (I) pour lesquels R₂ représente une chaîne -(CH₂)ₙ-CO-R₁₀, R₁₀ représente un radical alcoxy, cycloalkyloxy, cycloalkylalkyloxy, phényle ou -NR₁₂R₁₃,_{,} R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle peuvent également être préparés par action d'un dérivé de formule (IV) dans laquelle R₂ et R₇ ont les mêmes significations que précédemment et R, R₁, R₃ et R₆ ont les mêmes significations que dans la formule (I), sur un acide de formule : 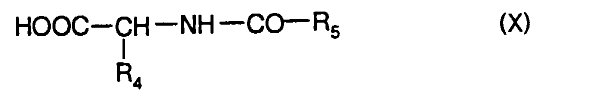 dans laquelle R₅ a les mêmes significations que ci-dessus ou un dérivé réactif de cet acide et R₄ a les mêmes significations que dans la formule (I).

Cette réaction s'effectue de préférence en présence d'un agent de condensation utilisé en chimie peptidique tel qu'un carbodimide au sein d'un solvant tel que l'acétonitrile, le tétrahydrofuranne ou un solvant chloré ou au moyen de chlorure de thionyle dans le dichlorométhane à une température comprise entre 10° C et la température d'ébullition du solvant.

Les acides de formule (X) peuvent être obtenus par application ou adaptation de la méthode décrite par J.R. JOHNSON et coll., J. Am. Chem. Soc., 69, 2370 (1947) ou, pour les composés pour lesquels R₅ représente un radical phénylamino éventuellement substitué, par action d'un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle, sur un dérivé de formule : 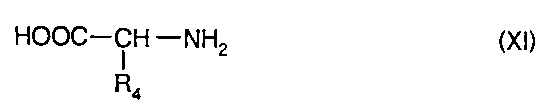 dans laquelle R₄ a les mêmes significations que dans la formule (I).

Cette réaction s'effectue généralement en solution aqueuse en présence d'une base telle qu'un bicarbonate de métal alcalin ou dans le dioxanne aqueux, à une température voisine de 20°C.

Les composés de formule (I) pour lesquels R₇ représente un radical phénylsulfonyle dont le phényle est substitué par un radical amino peuvent également être préparés par réduction des composés de formule (I) correspondants pour lesquels R₇ représente un radical phénylsulfonyle dont le phényle est substitué par un radical nitro.

Cette réduction s'effectue généralement sous pression d'hydrogène (130 KPa de péférence), en présence d'un catalyseur d'hydrogénation tel que le charbon palladié, au sein d'un solvant inerte tel qu'un alcool (éthanol par exemple), à une température voisine de 20°C.

Les composés de formule (I) pour lesquels R₇ représente un radical phénylsulfonyle dont le phényle est substitué par un radical monométhylamino ou diméthylamino peuvent également être préparés par méthylation des composés de formule (I) correspondants pour lesquels R₇ représente un radical phénylsulfonyle dont le phényle est substitué par un radical amino.

Cette réaction s'effectue au moyen de formaldéhyde, sous pression d'hydrogène (130 KPa de péférence), en présence d'un catalyseur d'hydrogénation tel que le charbon palladié, au sein d'un solvant inerte tel qu'un alcool (éthanol par exemple), à une température voisine de 20°C.

Il est entendu pour l'homme de métier que, la mise en oeuvre des procédés selon l'invention décrits précédemment, il peut être nécessaire afin d'éviter des réactions secondaires d'introduire des groupes protecteurs des fonctions amine, alcool, acide, cétone tels que ceux décrits par T. W. GREENE, protective groups in organic synthesis, John Wiley and Sons, New York. Par exemple les fonctions amine peuvent être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions alcool peuvent par exemple être bloquées sous forme de benzoate puis régénérées par hydrolyse en milieu alcalin après avoir mis en oeuvre le procédé selon l'invention. Les fonctions cétone peuvent être bloquées sous forme de dioxolanne-1,3 puis régénérées au moyen de d'un mélange acide chlorhydrique-acide acétique.

Les énantiomères des composés de formule (I) contenant au moins un site asymétrique peuvent être obtenus par dédoublement des racémiques par exemple par chromatographie sur colonne chirale ou par synthèse à partir des précurseurs chiraux.

Les composés de formule (I) peuvent être purifiés par les méthodes connues habituelles, par exemple par cristallisation, chromatographie ou extractions.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la Cl₅₀ des composés de formule (I) est généralement inférieure ou égale à 2000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989); REYFELD et coll., Am. J. Physiol., 240, G255-266 (1981); BEINFELD et coll., Neuropeptides, 3, 411-427 (1983).

Les composés de formule (I) présentent une toxicité faible. Leur DL₅₀ est généralement supérieure à 40 mg/kg par voie sous cutanée chez la souris.

Parmi les composés de formule (I) sont préférés ceux pour lesquels
R représente phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₈R_{9,} -NH-CO-CH₃, trifluorométhyle ou trifluorométhoxy,
R₁ représente un atome d'hydrogène,
R₂ représente une chaîne -(CH₂)ₙ-CO-R₁₀,
R₃ représente un atome d'hydrogène,
R₄ représente un atome d'hydrogène,
R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 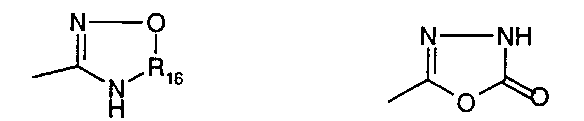 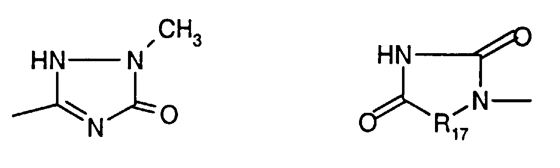 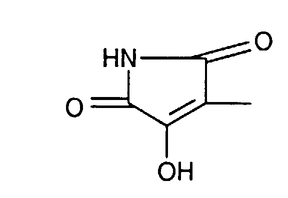 ou diméthyl-2,2 dioxo-4,6 dioxanne-1,3-yl-5,
R₆ représente un atome d'hydrogène,
R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy,
R₈ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₉ représente un radical alkyle, phénylalkyle ou phényle éventuellement substitué par un ou plusieurs substituants choisis parmi les atomes d'halogène et les radicaux alkyle, alcoxy et alkylthio,
ou bien R₈ et R₉ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₁₀ représente un radical hydroxy ou alcoxy,
R₁₄ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₅ représente un radical tétrazolyl-5,
R₁₆ représente C=O ou S=O,
R₁₇ représente O ou C=O,
n est égal à 0, 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène, leurs racémiques, leurs énantiomères et leurs sels.

Les composés préférés sont les suivants :
acide ({[tert-butoxycarbonyl-2 (fluoro-2 phényl)-5 (chloro-4 phényl)sulfonyl-4 pyrrolidinyl-1]-2 oxo-2 éthyl}-3 uréido)-3 phénylacétique-(2RS,4SR,5RS),
acide ({[tert-butoxycarbonyl-2 (fluoro-2 phényl)-5 (fluoro-2 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (méthoxy-3 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (méthyl-4 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (nitro-4 phényl)sulfonyl-4 pyrrolidinyl-1]-2 oxo-2 éthyl}-3 uréido)-3 benzoique-(2RS,4SR,5RS),
acide ({[(amino-4 phényl)sulfonyl-4 tert-butoxycarbonyl-2 (fluoro-2 phényl)-5 pyrrolidinyl-1]-2 oxo-2 éthyl}-3 uréido)-3 benzoïque-(2RS,4SR,5RS),
acide ({[(acétamido-4 phényl)sulfonyl-4 tert-butoxycarbonyl-2 (fluoro-2 phényl)-5 pyrrolidinyl-1]-2 oxo-2 éthyl}-3 uréido)-3 benzoïque-(2RS,4SR,5RS),
acide ({[tert-butoxycarbonyl-2 (diméthylamino-4 phényl)sulfonyl-4 (fluoro-2 phényl)-5 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 méthylsulfonyl-4 pyrrolidinyl-1]-2 oxo-2 éthyl}-3 uréido)-3 benzoïque-(2RS,4SR,5RS),
acide ({[(chloro-4 phényl)sulfonyl-4 (fluoro-2 phényl)-5 isobutylcarbamoyl-2 pyrrolidinyl-1]-2 oxo-2 éthyl}-3 uréido)-3 phénylacétique-(2RS,4SR,5RS),
acide ({[(fluoro-2 phényl)-5 (fluoro-2 phényl)sulfonyl-4 isobutylcarbamoyl-2 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (méthoxy-3 phényl)sulfonyl-4 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (méthyl-4 phényl)sulfonyl-4 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (nitro-4 phényl)sulfonyl-4 pyrrolidinyl-1]-2 oxo-2 éthyl}-3 uréido)-3 benzoïque-(2RS,4SR,5RS),
acide ({[(amino-4 phényl)sulfonyl-4 (fluoro-2 phényl)-5 isobutylcarbamoyl-2 pyrrolidinyl-1]-2 oxo-2 éthyl}-3 uréido)-3 phénylacétique-(2RS,4SR,5RS),
acide ({[(acétamido-4 phényl)sulfonyl-4 (fluoro-2 phényl)-5 isobutylcarbamoyl-2 pyrrolidinyl-1]-2 oxo-2 éthyl}-3 uréido)-3 benzoïque-(2RS,4SR,5RS),
acide ({[(tert-butoxycarbonyl-2 (fluoro-2 phényl)-5 morpholinosulfonyl-4 pyrrolidinyl-1]-2 oxo-2 éthyl}-3 uréido)-3 benzoïque-(2RS,4SR,5RS),
et leurs sels.

Les exemples suivants illustrent l'invention.

### Exemple 1

A A une solution de 2,5 g de {[(méthoxycarbonylméthyl-3 phényl)-3 uréido]-2 acétyl}-1 (fluoro-2 phényl)-5 (chloro-4 phényl)sulfonyl-4 pyrrolidinecarboxylate-2 de tert-butyle-(2RS,4SR,5RS) dans 60 cm³ de méthanol et 30 cm³ d'eau distillée, on ajoute, à une température voisine de 20°C, 0,2 g d'hydroxyde de potassium. Le mélange réactionnel est agité pendant vingt heures à une température voisine de 20°C, puis concentré sous pression réduite. Le résidu est repris dans 100 cm³ d'eau distillée, lavé par trois fois 75 cm³ d'oxyde de diéthyle, acidifié à un pH voisin de 1 par une solution aqueuse normale d'acide chlorhydrique et extrait par trois fois 75 cm³ d'acétate d'éthyle. Les extraits sont réunis, lavés par trois fois 50 cm³ d'une solution aqueuse saturée en chlorure de sodium, séchés sur sulfate de magnésium et concentrés sous pression réduite. Le résidu est purifié par chromatographie sur 80 g de silice contenus dans une colonne de 3,2 cm de diamètre [éluant : dichlorométhane-méthanol (95/5 en volumes)]. Les fractions contenant le produit attendu sont réunies et concentrées sous pression réduite. On obtient ainsi 0,95 g d'acide ({[tert-butoxycarbonyl-2 (fluoro-2 phényl)-5 (chloro-4 phényl)sulfonyl-4 pyrrolidinyl-1]-2 oxo-2 éthyl}-3 uréido)-3 phénylacétique-(2RS,4SR,5RS) [Rf = 0,5 ; éluant : chlorure de méthylène-méthanol (90/10); Spectre de R.M.N. ¹H (250 MHz, (CD₃)₂SO avec ajout de quelques gouttes de CD₃COOD, à une température de 393 K, δ en ppm) : 1,54 (s, 9H : C(CH₃)₃); de 2,40 à 2,60 et 2,85 (2 mts, 1H chacun : CH₂ en 3); 3,50 (s, 2H : ArCH₂COO); 3,70 et 3,97 (respectivement d large et d, J = 17 Hz, 1H chacun : COCH₂N); 4,09 (mt, 1H : H 4); 4,68 (t, J = 8 Hz, 1H : H 2); 5,73 (d, J = 3 Hz, 1H : H 5); 6,86 (d large, J = 7,5 Hz, 1H : H aromatique (H 4 : en ortho du CH₂)); de 7,00 à 7,40 (mt, 6H : H aromatiques); 7,68 (d large, J = 8,5 Hz, 2H : H de l'aromatique en 4 (H en ortho du Cl)); 7,82 (t large, J = 8 Hz, 1H : H de l'aromatique en 5 (H 6)); 7,96 (d large, J = 8,5 Hz, 2H : H aromatiques en 4 (H en méta du Cl))].

B Le {[(méthoxycarbonylméthyl-3 phényl)-3 uréido]-2 acétyl}-1 (fluoro-2 phényl)-5 (chloro-4 phényl)sulfonyl-4 pyrrolidinecarboxylate-2 de tert-butyle-(2RS,4SR,5RS) peut être préparé de la manière suivante : à une solution de 2,4 g de chlorhydrate de (fluoro-2 phényl)-5 (chloro-4 phényl)sulfonyl-4 pyrrolidinecarboxylate-2 de tert-butyle-(2RS,4SR,5RS) dans 100 cm³ de dichlorométhane, on ajoute 0,7 cm³ de triéthylamine. Le mélange est lavé par trois fois 50 cm³ d'eau distillée et la phase organique séchée sur sulfate de magnésium. Le résidu est dissous dans 75 cm³ d'acétonitrile. On ajoute ensuite 1,35 g d'acide [(méthoxycarbonylméthyl-3 phényl)-3 uréido]-2 acétique puis 1,1 g de N,N'-dicyclohexylcarbodiimide. Le mélange réactionnel est agité pendant vingt heures à une température voisine de 20°C. Le mélange réactionnel est concentré sous pression réduite, repris par 50 cm³ d'acétate d'éthyle, filtré, rincé par deux fois 25 cm³ d'acétate d'éthyle et concentré sous pression réduite. Le résidu est purifié par chromatographie sur 120 g de silice contenus dans une colonne de 3,2 cm de diamètre [éluant : cyclo-hexane-acétate d'éthyle (50/50 en volumes)]. Les fractions contenant le produit attendu sont réunies et concentrées sous pression réduite. On obtient ainsi 3 g de {[(méthoxycarbonylméthyl-3 phényl)-3 uréido]-2 acétyl}-1 (fluoro-2 phényl)-5 (chloro-4 phényl)sulfonyl-4 pyrrolidinecarboxylate-2 de tert-butyle-(2RS,4SR,5RS) sous forme d'une meringue utilisée telle quelle dans les synthèses ultérieures.

C Le chlorhydrate de (fluoro-2 phényl)-5 (chloro-4 phényl)sulfonyl-4 pyrrolidinecarboxylate-2 de tert-butyle-(2RS,4SR,5RS) peut être préparé de la manière suivante : à une solution de 7,2 g de (fluoro-2 benzylidèneamino)acétate de tert-butyle dans 200 cm³ d'acétonitrile, on ajoute successivement 6,1 g de (chloro-4 phényl)vinylsulfone, 7,52 g d'acétate d'argent et 5,1 cm³ de triéthylamine. Le mélange réactionnel est agité pendant quatre heures à une température voisine de 20°C, puis versé dans 200 cm³ d'une solution aqueuse saturée en chlorure d'ammonium. La suspension est filtrée, le précipité lavé par deux fois 50 cm³ d'eau distillée et le filtrat extrait par trois fois 100 cm³ d'acétate d'éthyle. Les extraits sont réunis, lavés par deux fois 100 cm³ d'eau distillée, séchés sur sulfate de magnésium et concentrés sous pression réduite. Le résidu est purifié par chromatographie sur 150 g de silice contenus dans une colonne de 4,2 cm de diamètre [éluant: cyclohexane-acétate d'éthyle (70/30 en volumes)]. Les fractions contenant le produit attendu sont réunies et concentrées sous pression réduite. Le résidu est dissous dans 20 cm³ d'acétate d'éthyle et traité par 20 cm³ d'une solution aqueuse normale d'acide chlorhydrique. Le précipité qui se forme est filtré et lavé successivement à l'eau et l'acétate d'éthyle. On obtient ainsi 4 g de chlorhydrate de (fluoro-2 phényl)-5 (chloro-4 phényl)sulfonyl-4 pyrrolidinecarboxylate-2 de tert-butyle-(2RS,4SR,5RS) fondant à 138°C. A partir d'autres fractions, on obtient aussi, après recristallisation dans l'acétonitrile, 3 g de (fluoro-2 phényl)-5 (chloro-4 phényl)sulfonyl-4 pyrrolidinecarboxylate-2 de tert-butyle-(2RS,4RS,5RS) fondant à 174°C.

D Le (fluoro-2 benzylidèneamino)acétate de tert-butyle peut être préparé de la manière suivante : à une suspension de 3 g de tamis moléculaire 4Å dans une solution contenant 5,01 g de chlorhydrate de glycinate de tert-butyle, de 3,15 cm³ de fluoro-2 benzaldéhyde dans 45 cm³ de dichlorométhane, on ajoute goutte à goutte 4,2 cm³ de triéthylamine à une température voisine de 20°C. Le mélange réactionnel est agité pendant soixante-douze heures à une température voisine de 20°C et concentré sous pression réduite. Le résidu est repris dans 50 cm³ d'oxyde de diéthyle, filtré et le précipité rincé par deux fois 25 cm³ d'oxyde de diéthyle. Le filtrat est concentré sous pression réduite. On obtient ainsi 7,2 g de (fluoro-2 benzylidèneamino)acétate de tert-butyle sous forme d'une huile utilisée telle quelle dans les synthèses ultérieures.

E La (chloro-4 phényl)vinylsulfone peut être préparée de la manière suivante : à une solution de 18,5 g de (chloro-2 éthyl)(chloro-4 phényl)sulfone dans 100 cm³ de tétrahydrofuranne, on ajoute en cinq minutes à une température voisine de 30°C une solution de 16,3 cm³ de triéthylamine dans 50 cm³ de tétrahydrofuranne. Le mélange réactionnel est agité pendant vingt heures à une température voisine de 20°C. La suspension est filtrée et le précipité rincé par trois fois 25 cm³ de tétrahydrofuranne. Le filtrat est concentré sous pression réduite. On obtient ainsi 16 g de (chloro-4 phényl)vinylsulfone sous forme d'une huile jaune utilisée telle quelle dans les synthèses ultérieures.

F La (chloro-2 éthyl)(chloro-4 phényl)sulfone peut être préparée de la manière suivante : à une solution de 18 g de (chloro-2 éthyl)(chloro-4 phényl)sulfure dans 87 cm³ d'acide acétique, on ajoute en trente minutes, à une température voisine de 48°C, un mélange de 24,8 cm³ d'une solution aqueuse à 30% de peroxyde d'hydrogène et de 40 cm³ d'acide acétique. Le milieu réactionnel est agité pendant quatre heures à une température voisine de 70°C, pendant huit heures à une température voisine de 90°C, puis pendant vingt heures à une température voisine de 20°C. On ajoute ensuite 200 cm³ d'eau distillée au milieu réactionnel et on filtre. Le précipité est lavé par cinq fois 75 cm³ d'eau distillée et séché sous pression réduite à une température voisine de 40°C. On obtient ainsi 18,5 g de (chloro-2 éthyl)(chloro-4 phényl)sulfone fondant à 91°C.

G Le (chloro-2 éthyl)(chloro-4 phényl)sulfure peut être préparé de la manière suivante : à une solution de 14,5 g de chloro-4 thiophénol et de 0,24 cm³ d'aliquat dans 114 cm³ de dichloro-1,2 éthane, on ajoute en deux heures, à une température voisine de 20°C, une solution de 4,94 g d'hydroxyde de sodium dans 62 cm³ d'eau distillée. Le milieu réactionnel est agité pendant vingt heures à une température voisine de 20°C puis est décanté. La phase organique est lavée par 60 cm³ d'une solution aqueuse 0,1 N d'acide chlorhydrique puis deux fois 50 cm³ d'une solution aqueuse saturée en chlorure de sodium, séchée sur sulfate de magnésium et concentrée sous pression réduite. Le résidu est purifié par chromatographie sur 200 g de silice contenus dans une colonne de 6 cm de diamètre (éluant: cyclohexane). Les fractions contenant le produit attendu sont réunies et concentrées sous pression réduite. On obtient ainsi 18 g de (chloro-2 éthyl)(chloro-4 phényl)sulfure sous forme d'une huile incolore utilisée telle quelle dans les synthèses ultérieures.

H L'acide [(méthoxycarbonylméthyl-3 phényl)-3 uréido]-2 acétique peut être préparé de la manière suivante : à une solution de 9,42 g de glycine et de 34,69 g de carbonate de potassium dans 220 cm³ d'eau, on ajoute, à une température voisine de 5°C, une solution de 24 g d'isocyanato-3 phénylacétate de méthyle dans 170 cm³ de dioxanne-1,4, Le mélange réactionnel est agité pendant vingt heures à une température voisine de 20°C puis acidifié à un pH voisin de 1 avec une solution aqueuse 4N d'acide chlorhydrique. Le produit insoluble est séparé par filtration, lavé par trois fois 50 cm³ d'eau et séché à l'air. On obtient ainsi après recristallisation dans l'acétate d'éthyle, 46,85 g d'acide [(méthoxycarbonylméthyl-3 phényl)-3 uréido]-2 acétique fondant à 136°C.

I L'isocyanato-3 phénylacétate de méthyle peut être préparé de la manière suivante : à une suspension de 1 g de charbon et 6 cm³ de chloroformiate de trichlorométhyle dans 70 cm³ de toluène, on ajoute, à une température voisine de -20°C et sous argon, 8,25 g d'amino-3 phénylacétate de méthyle en solution dans 100 cm³ de toluène. Le mélange réactionnel est agité et maintenu à une température voisine de -20°C pendant quinze minutes, puis, après retour à une température voisine de 20°C, chauffé à reflux pendant deux heures trente minutes. Le mélange est alors dégazé par barbotage d'argon pendant trente minutes, filtré sur célite, rincé par 50 cm³ de dichlorométhane et concentré sous pression réduite à une température voisine de 50°C. On obtient ainsi 9,30 g d'isocyanato-3 phénylacétate de méthyle sous forme d'une huile utilisée telle quelle dans les synthèses ultérieures.

L'amino-3 phénylacétate de méthyle peut être préparé selon la méthode décrite par W.A. JACOBS et coll. J. Amer. Chem. Soc., 34, 2420 (1917).

### Exemple 2

A A une solution de 4,1 g de {[(benzyloxycarbonyl-3 phényl)-3 uréido]-2 acétyl}-1 (fluoro-2 phényl)-5 (fluoro-2 phényl)sulfonyl-4 pyrrolidinecarboxylate-2 de tert-butyle-(2RS,4SR,5RS) dans 100 cm³ d'éthanol, on ajoute 0,4 g de palladium à 10% sur charbon. La suspension est agitée pendant vingt heures à une température voisine de 20°C sous atmosphère d'hydrogène (130 kPa). Le catalyseur est séparé par filtration sur célite et le filtrat est concentré à sec sous pression réduite. Le résidu est repris dans un mélange acétate d'isopropyle-oxyde de diisopropyle (10/90 en volumes), filtré et le précipité est rincé par un mélange acétate d'isopropyle-oxyde de diisopropyle (10/90 en volumes) puis par de l'oxyde de diisopropyle et séché sous pression réduite à une température voisine de 40°C. On obtient ainsi 3 g d'acide ({[tert-butoxycarbonyl-2 (fluoro-2 phényl)-5 (fluoro-2 phényl)sulfonyl-4 pyrrolidinyl-1]-2 oxo-2 éthyl}-3 uréido)-3 benzoïque-(2RS,4SR,5RS) [Rf = 0,3; éluant : chlorure de méthylène-méthanol (90/10); Spectre de R.M.N. ¹H (20 MHz, (CD₃)₂SO avec ajout de quelques gouttes de CD₃COOD, à une température de 383 K, δ en ppm) : 1,55 (s, 9H : C(CH₃)₃); de 2,45 à 2,65 et 2,88 (2 mts, 1H chacun : CH₂ en 3); 3,75 et 4,00 (respectivement d large et d, J = 17 Hz, 1H chacun : COCH₂N); 4,15 (mt, 1H : H 4); 4,76 (t, J = 8 Hz, 1H : H 2); 5,73 (d, J = 3,5 Hz, 1H : H 5); de 6,95 à 8,05 (mt, 12H : H aromatiques)].

B Le {[(benzyloxycarbonyl-3 phényl)-3 uréido]-2 acétyl}-1 (fluoro-2 phényl)-5 (fluoro-2 phényl)sulfonyl-4 pyrrolidinecarboxylate-2 de tert-butyle-(2RS,4SR,5RS) peut être préparé comme décrit à l'exemple 1B, mais à partir de 3 g de (fluoro-2 phényl)-5 (fluoro-2 phényl)sulfonyl-4 pyrrolidinecarboxylate-2 de tert-butyle-(2RS,4SR,5RS), de 2,33 g d'acide [(benzyloxycarbonyl-3 phényl)-3 uréido]-2 acétique et de 1,46 g de N,N'-dicyclohexylcarbodiimide dans 75 cm³ d'acétonitrile. Après traitement, on obtient 4,1 g de {[(benzyloxycarbonyl-3 phényl)-3 uréido]-2 acétyl}-1 (fluoro-2 phényl)-5 (fluoro-2 phényl)sulfonyl-4 pyrrolidinecarboxylate-2 de tert-butyle-(2RS,4SR,5RS) sous forme d'une meringue utilisée telle quelle dans les synthèses ultérieures.

C Le (fluoro-2 phényl)-5 (fluoro-2 phényl)sulfonyl-4 pyrrolidinecarboxylate-2 de tert-butyle-(2RS,4SR,5RS) peut être préparé comme décrit à l'exemple 1C, mais à partir de 4,75 g de (fluoro-2 benzylidèneamino)acétate de tert-butyle, de 3,73 g de (fluoro-2 phényl)vinylsulfone, de 5 g d'acétate d'argent et de 3,4 cm³ de triéthylamine dans 200 cm³ d'acétonitrile. Après traitement, on obtient 3 g de (fluoro-2 phényl)-5 (fluoro-2 phényl)sulfonyl-4 pyrrolidinecarboxylate-2 de tert-butyle-(2RS,4SR,5RS) fondant à 126°C et 2,55 g de (fluoro-2 phényl)-5 (fluoro-2 phényl)sulfonyl-4 pyrrolidinecarboxylate-2 de tert-butyle-(2RS,4RS,5RS) fondant à 202°C.

D La (fluoro-2 phényl)vinylsulfone peut être préparée comme décrit à l'exemple 1E, mais à partir de 11,5 g de (chloro-2 éthyl)(fluoro-2 phényl)sulfone, de 10,6 cm³ de triéthylamine et de 150 cm³ de tétrahydrofuranne. Après traitement, on obtient 9 g de (fluoro-2 phényl)vinylsulfone sous forme d'une huile jaune utilisée telle quelle dans les synthèses ultérieures.

E La (chloro-2 éthyl)(fluoro-2 phényl)sulfone peut être préparée comme décrit à l'exemple 1F, mais à partir de 10 g de (chloro-2 éthyl)(fluoro-2 phényl)sulfure, de 14,3 cm³ d'une solution aqueuse à 30% de peroxyde d'hydrogène et de 75 cm³ d'acide acétique. Après traitement, on obtient 11,5g de (chloro-2 éthyl)(fluoro-2 phényl)sulfone sous forme d'une huile orangée utilisée telle quelle dans les synthèses ultérieures.

F Le (chloro-2 éthyl)(fluoro-2 phényl)sulfure peut être préparé comme décrit à l'exemple 1G, mais à partir de 6,4 g de fluoro-2 thiophénol, de 0,12cm³ d'aliquat, de 2,47 g d'hydroxyde de sodium, de 31 cm³ d'eau distillée et de 57 cm³ de dichloro-1,2 éthane. Après traitement, on obtient 10 g de (chloro-2 éthyl)(fluoro-2 phényl)sulfure sous forme d'une huile jaune utilisée telle quelle dans les synthèses ultérieures.

G L'acide [(benzyloxycarbonyl-3 phényl)-3 uréido]-2 acétique peut être préparé comme décrit à l'exemple 1H, mais à partir de 36,7 g d'isocyanato-3 benzoate de benzyle, de 10,9 g de glycine et de 40,1 g de carbonate de potassium dans un mélange de 245 cm³ d'eau distillée et de 195 cm³ de dioxanne-1,4. Après traitement, on obtient 38,5 g d'acide [(benzyloxycarbonyl-3 phényl)-3 uréido]-2 acétique fondant à 168°C.

H L'isocyanato-3 benzoate de benzyle peut être préparé comme décrit à l'exemple 1l, mais à partir de 33 g d'amino-3 benzoate de benzyle, de 2,9 g de charbon et de 28,7 g de carbonate de bis-trichlorométhyle dans 500 cm³ de toluène. Après traitement on obtient 38 g d'isocyanato-3 benzoate de benzyle sous forme d'une huile utilisée telle quelle dans les synthèses ultérieures.

L'amino-3 benzoate de benzyle peut être préparé selon la méthode décrite par H. Bredereck Ber., 91, 215 (1948).

### Exemple 3

A On opère comme décrit à l'exemple 2A, mais à partir de 5,3 g de {([(benzyloxycarbonyl-3 phényl)-3 uréido]-2 acétyl}-1 (fluoro-2 phényl)-5 (méthoxy-3 phényl)sulfonyl-4 pyrrolidinecarboxylate-2 de tert-butyle-(2RS,4SR,5RS), de 0,4 g de palladium à 10% sur charbon et de 100 cm³ d'éthanol sous atmosphère d'hydrogène (130 kPa). Après traitement, on obtient 3,5 g d'acide ({[tert-butoxycarbonyl-2 (fluoro-2 phényl)-5 (méthoxy-3 phényl)sulfonyl-4 pyrrolidinyl-1]-2 oxo-2 éthyl}-3 uréido)-3 benzoïque-(2RS,4SR,5RS) [Rf = 0,3 ; éluant : chlorure de méthylène-méthanol (90/10); Spectre de R.M.N. 1H (250 MHz, (CD₃)₂SO avec ajout de quelques gouttes de CD₃COOD, à une température de 393 K, δ en ppm) : 1,54 (s, 9H : C(CH₃)₃); de 2,40 à 2,60 et 2,82 (2 mts, 1H chacun : CH₂ en 3); 3,68 et 3,98 (respectivement d large et d, J = 17 Hz, 1H chacun : COCH₂N); 3,88 (s, 3H : OCH₃); 4,11 (mt, 1H : H 4); 4,67 (t, J = 8 Hz, 1H : H 2); 5,72 (d, J = 3 Hz, 1H: H 5); de 7,00 à 7,65 (mt, 10H : H aromatiques); 7,80 (t large, J = 8 Hz, 1H : H de l'aromatique en 5 (H 6)); 8,00 (mt, 1H : H aromatique (H 2 : en ortho du COOH))].

B Le {[(benzyloxycarbonyl-3 phényl)-3 uréido]-2 acétyl}-1 (fluoro-2 phényl)-5 (méthoxy-3 phényl)sulfonyl-4 pyrrolidinecarboxylate-2 de tert-butyle-(2RS,4SR,5RS) peut être préparé comme décrit à l'exemple 1B, mais à partir de 3,7 g de (fluoro-2 phényl)-5 (méthoxy-3 phényl)sulfonyl-4 pyrrolidinecarboxylate-2 de tert-butyle-(2RS,4SR,5RS), de 2,73 g d'acide [(benzyloxycarbonyl-3 phényl)-3 uréido]-2 acétique et de 1,75 g de N,N'-dicyclohexylcarbodiimide dans 75 cm³ d'acétonitrile. Après traitement, on obtient 4,3 g de {[(benzyloxycarbonyl-3 phényl)-3 uréido]-2 acétyl}-1 (fluoro-2 phényl)-5 (méthoxy-3 phényl)sulfonyl-4 pyrrolidinecarboxylate-2 de tert-butyle-(2RS,4SR,5RS) sous forme d'une meringue utilisée telle quelle dans les synthèses ultérieures.

C Le (fluoro-2 phényl)-5 (méthoxy-3 phényl)sulfonyl-4 pyrrolidinecarboxylate-2 de tert-butyle-(2RS,4SR,5RS) peut être préparé comme décrit à l'exemple 1C, mais à partir de 4,75 g de (fluoro-2 benzylidèneamino)acétate de tert-butyle, de 3,73 g de (méthoxy-3 phényl)vinylsulfone, de 5 g d'acétate d'argent et de 3,4 cm³ de triéthylamine dans 200 cm³ d'acétonitrile. Après traitement, on obtient 3,9 g de (fluoro-2 phényl)-5 (méthoxy-3 phényl)sulfonyl-4 pyrrolidinecarboxylate-2 de tert-butyle-(2RS,4SR,5RS) sous forme d'une huile orangée utilisée telle quelle dans les synthèses ultérieures et 1,1 g de (fluoro-2 phényl)-5 (méthoxy-3 phényl)sulfonyl-4 pyrrolidinecarboxylate-2 de tert-butyle-(2RS,4RS,5RS) fondant à 140°C.

D La (méthoxy-3 phényl)vinylsulfone peut être préparée comme décrit à l'exemple 1E, mais à partir de 11,8 g de (chloro-2 éthyl)(méthoxy-3 phényl)sulfone, de 10,6 cm³ de triéthylamine et de 150 cm³ de tétrahydrofuranne. Après traitement, on obtient 9 g de (méthoxy-3 phényl)vinylsulfone sous forme d'une huile jaune utilisée telle quelle dans les synthèses ultérieures.

E La (chloro-2 éthyl)(méthoxy-3 phényl)sulfone peut être préparée comme décrit à l'exemple 1F, mais à partir de 10,5 g de (chloro-2 éthyl)(méthoxy-3 phényl)sulfure, de 14,3 cm³ d'une solution aqueuse à 30% de peroxyde d'hydrogène et de 75 cm³ d'acide acétique. Après traitement, on obtient 11,8 g de (chloro-2 éthyl)(méthoxy-3 phényl)sulfone sous forme d'une huile orangée utilisée telle quelle dans les synthèses ultérieures.

F Le (chloro-2 éthyl)(méthoxy-3 phényl)sulfure peut être préparé comme décrit à l'exemple 1G, mais à partir de 7 g de méthoxy-3 thiophénol, de 0,12 cm³ d'aliquat, de 2,47 g d'hydroxyde de sodium, de 31 cm³ d'eau distillée et de 57 cm³ de dichloro-1,2 éthane. Après traitement, on obtient 10,5 g de (chloro-2 éthyl)(méthoxy-3 phényl)sulfure sous forme d'une huile jaune utilisée telle quelle dans les synthèses ultérieures.

### Exemple 4

A On opère comme décrit à l'exemple 1A, mais à partir de 2,2 g de (fluoro-2 phényl)-5 {[(méthoxycarbonyl-3 phényl)-3 uréido]-2 acétyl}-1 (méthyl-4 phényl)sulfonyl-4 pyrrolidinecarboxylate-2 de tert-butyle-(2RS,4SR,5RS) et de 0,19 g d'hydroxyde de potassium dans un mélange de 60 cm³ de méthanol et de 20 cm³ d'eau distillée. Après traitement, on obtient 0,27 g d'acide ({[tert-butoxycarbonyl-2 (fluoro-2 phényl)-5 (méthyl-4 phényl)sulfonyl-4 pyrrolidinyl-1]-2 oxo-2 éthyl}-3 uréido)-3 benzoïque-(2RS,4SR,5RS) [Rf = 0,5 ; éluant : chlorure de méthylène-méthanol (90/10); Spectre de R.M.N. ¹H (200 MHz, (CD₃)₂SO avec ajout de quelques gouttes de CD₃COOD, à une température de 373 K, δ en ppm) : 1,52 (s, 9H : C(CH₃)₃); de 2,35 à 2,55 et 2,79 (2 mts, 1H chacun : CH₂ en 3); 2,45 (s, 3H: ArCH₃); 3,66 et 3,96 (respectivement d large et d, J = 17 Hz, 1H chacun: COCH₂N); 4,05 (mt, 1H : H 4); 4,62 (t, J = 8 Hz, 1H : H 2); 5,70 (d, J = 3 Hz, 1H : H 5); de 7,00 à 7,55 (mt, 8H : H aromatiques); 7,80 (mt, 1H : H de l'aromatique en 5 (H 6)); 7,85 (d, J = 8 Hz, 2H : H de l'aromatique en 4 (en ortho du CH₃)); 8,00 (t, J = 1,5 Hzt, 1H : H aromatique (H 2 : en ortho du COOH))].

B Le (fluoro-2 phényl)-5 {[(méthoxycarbonyl-3 phényl)-3 uréido]-2 acétyl}-1 (méthyl-4 phényl)sulfonyl-4 pyrrolidinecarboxylate-2 de tert-butyle-(2RS,4SR,5RS) peut être préparé comme décrit à l'exemple 1B, mais à partir de 2,1 g de (fluoro-2 phényl)-5 (méthyl-4 phényl)sulfonyl-4 pyrrolidinecarboxylate-2 de tert-butyle-(2RS,4SR,5RS), de 1,26 g d'acide [(méthoxycarbonyl-3 phényl)-3 uréido]-2 acétique et de 1,1 g de N,N'-dicyclohexylcarbodiimide dans 100 cm³ d'acétonitrile. Après traitement, on obtient 2,2 g de (fluoro-2 phényl)-5 {[(méthoxycarbonyl-3 phényl)-3 uréido]-2 acétyl}-1 (méthyl-4 phényl)sulfonyl-4 pyrrolidinecarboxylate-2 de tert-butyle-(2RS,4SR,5RS) sous forme d'une meringue utilisée telle quelle dans les synthèses ultérieures.

C Le (fluoro-2 phényl)-5 (méthyl-4 phényl)sulfonyl-4 pyrrolidinecarboxylate-2 de tert-butyle-(2RS,4SR,5RS) peut être préparé comme décrit à l'exemple 1C, mais à partir de 4,75 g de (fluoro-2 benzylidèneamino)acétate de tert-butyle, de 3,65 g de (méthyl-4 phényl)vinylsulfone, de 5 g d'acétate d'argent et de 3,65 cm³ de triéthylamine dans 150 cm³ d'acétonitrile. Après traitement, on obtient 4 g de (fluoro-2 phényl)-5 (méthyl-4 phényl)sulfonyl-4 pyrrolidinecarboxylate-2 de tert-butyle-(2RS,4SR,5RS) sous forme d'une huile orangée utilisée telle quelle dans les synthèses ultérieures et 1,1 g de (fluoro-2 phényl)-5 (méthyl-4 phényl)sulfonyl-4 pyrrolidinecarboxylate-2 de tert-butyle-(2RS,4RS,5RS) fondant à 146°C.

D La (méthyl-4 phényl)vinylsulfone peut être préparée comme décrit à l'exemple 1E, mais à partir de 21 g de (chloro-2 éthyl)(méthyl-4 phényl)sulfone, de 20,2 cm³ de triéthylamine et de 250 cm³ de tétrahydrofuranne. Après traitement, on obtient 13,2 g de (méthyl-4 phényl)vinylsulfone fondant à 66°C.

E La (chloro-2 éthyl)(méthyl-4 phényl)sulfone peut être préparée comme décrit à l'exemple 1F, mais à partir de 13 g de (chloro-2 éthyl)(méthyl-4 phényl)sulfure, de 28,6 cm³ d'une solution aqueuse à 30% de peroxyde d'hydrogène et de 150 cm³ d'acide acétique. Après traitement, on obtient 21 g de (chloro-2 éthyl)(méthyl-4 phényl)sulfone sous forme d'une huile orangée utilisée telle quelle dans les synthèses ultérieures.

F Le (chloro-2 éthyl)(méthyl-4 phényl)sulfure peut être préparé comme décrit à l'exemple 1G, mais à partir de 12,42 g de méthyl-4 thiophénol, de 0,24 cm³ d'aliquat, de 4,94 g d'hydroxyde de sodium, de 62 cm³ d'eau distillée et de 114 cm³ de dichloro-1,2 éthane. Après traitement, on obtient 19 g de (chloro-2 éthyl)(méthyl-4 phényl)sulfure sous forme d'une huile jaune utilisée telle quelle dans les synthèses ultérieures.

G L'acide [(méthoxycarbonyl-3 phényl)-3 uréido]-2 acétique peut être préparé comme décrit à l'exemple 1H, mais à partir de 5,88 g d'isocyanato-3 benzoate de méthyle, de 2,5 g de glycine et de 9,2 g de carbonate de potassium dans un mélange de 90 cm³ d'eau distillée et de 75 cm³ de dioxanne-1,4. Après traitement, on obtient 5,27 g d'acide [(méthoxycarbonyl-3 phényl)-3 uréido]-2 acétique fondant à 220°C.

### Exemple 5

A On opère comme décrit à l'exemple 1A, mais à partir de 2,9 g de (fluoro-2 phényl)-5 {[(méthoxycarbonyl-3 phényl)-3 uréido]-2 acétyl}-1 (nitro-4 phényl)sulfonyl-4 pyrrolidinecarboxylate-2 de tert-butyle-(2RS,4SR,5RS) et de 0,24 g d'hydroxyde de potassium dans un mélange de 60 cm³ de méthanol et de 20 cm³ d'eau distillée. Après traitement, on obtient 0,25 g d'acide ({[tert-butoxycarbonyl-2 (fluoro-2 phényl)-5 (nitro-4 phényl)sulfonyl-4 pyrrolidinyl-1]-2 oxo-2 éthyl}-3 uréido)-3 benzoïque-(2RS,4SR,5RS) [Rf = 0,3; éluant : chlorure de méthylène-méthanol (90/10); Spectre de R.M.N. ¹H (200 MHz, (CD₃)₂SO avec ajout de quelques gouttes de CD₃COOD, à une température de 393 K, δ en ppm) : 1,55 (s, 9H : C(CH₃)₃); de 2,40 à 2,60 et 2,88 (2 mts, 1H chacun : CH₂ en 3); 3,71 et 4,02 (respectivement d large et d, J = 17 Hz, 1H chacun : COCH₂N); 4,28 (mt, 1H : H 4); 4,72 (t, J = 8 Hz, 1H: H 2); 5,76 (d, J = 3,5 Hz, 1H : H 5); de 6,95 à 7,65 (mt, 6H : H aromatiques); 7,81 (mt, 1H : H de l'aromatique en 5 (H 6)); 8,00 (t, J = 1,5 Hz, 1H : H aromatique (H 2 : en ortho du COOH)); 8,27 et 8,42 (2d, J = 8 Hz, 2H chacun : H de l'aromatique en 4)].

B Le (fluoro-2 phényl)-5 {[(méthoxycarbonyl-3 phényl)-3 uréido]-2 acétyl}-1 (nitro-4 phényl)sulfonyl-4 pyrrolidinecarboxylate-2 de tert-butyle-(2RS,4SR,5RS) peut être préparé comme décrit à l'exemple 1B, mais à partir de 2,25 g de (fluoro-2 phényl)-5 (nitro-4 phényl)sulfonyl-4 pyrrolidinecarboxylate-2 de tert-butyle-(2RS,4SR,5RS), de 1,26 g d'acide [(méthoxycarbonyl-3 phényl)-3 uréido]-2 acétique et de 1,1 g de N,N'-dicyclohexylcarbodiimide dans 100 cm³ d'acétonitrile. Après traitement, on obtient 2,9 g de (fluoro-2 phényl)-5 {[(méthoxycarbonyl-3 phényl)-3 uréido]-2 acétyl}-1 (nitro-4 phényl)sulfonyl-4 pyrrolidinecarboxylate-2 de tert-butyle-(2RS,4SR,5RS) sous forme d'une meringue utilisée telle quelle dans les synthèses ultérieures.

C Le (fluoro-2 phényl)-5 (nitro-4 phényl)sulfonyl-4 pyrrolidinecarboxylate-2 de tert-butyle-(2RS,4SR,5RS) peut être préparé comme décrit à l'exemple 1C, mais à partir de 9,5 g de (fluoro-2 benzylidèneamino)acétate de tert-butyle, de 8,5 g de (nitro-4 phényl)vinylsulfone, de 10 g d'acétate d'argent et de 7,3 cm³ de triéthylamine dans 200 cm³ d'acétonitrile. Après traitement, on obtient 6 g de (fluoro-2 phényl)-5 (nitro-4 phényl)sulfonyl-4 pyrrolidinecarboxylate-2 de tert-butyle-(2RS,4SR,5RS) sous forme d'une huile orangée utilisée telle quelle dans les synthèses ultérieures et 5,5 g de (fluoro-2 phényl)-5 (nitro-4 phényl)sulfonyl-4 pyrrolidinecarboxylate-2 de tert-butyle-(2RS,4RS,5RS) fondant à 218°C.

D La (nitro-4 phényl)vinylsulfone peut être préparée comme décrit à l'exemple 1E, mais à partir de 19 g de (chloro-2 éthyl)(nitro-4 phényl)sulfone, de 16,1 cm³ de triéthylamine et de 250 cm³ de tétrahydrofuranne. Après traitement, on obtient 12,7 g de (nitro-4 phényl)vinylsulfone fondant à 114°C.

E La (chloro-2 éthyl)(nitro-4 phényl)sulfone peut être préparée comme décrit à l'exemple 1F, mais à partir de 20 g de (chloro-2 éthyl)(nitro-4 phényl)sulfure, de 26,4 cm³ d'une solution aqueuse à 30% de peroxyde d'hydrogène et de 150 cm³ d'acide acétique. Après traitement, on obtient 19 g de (chloro-2 éthyl)(nitro-4 phényl)sulfone fondant à 130°C.

F Le (chloro-2 éthyl)(nitro-4 phényl)sulfure peut être préparé comme décrit à l'exemple 1G, mais à partir de 15,52 g de nitro-4 thiophénol, de 0,24 cm³ d'aliquat, de 4,94 g d'hydroxyde de sodium, de 62 cm³ d'eau distillée et de 114 cm³ de dichloro-1,2 éthane. Après traitement, on obtient 20 g de (chloro-2 éthyl)(nitro-4 phényl)sulfure sous forme d'un solide jaune fondant en dessous de 50°C.

### Exemple 6

A A une solution de 1,7 g de {([(benzyloxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) dans 100 cm³ d'éthanol, on ajoute 0,2 g de palladium à 10% sur charbon. La suspension est agitée pendant vingt heures à une température voisine de 20°C sous atmosphère d'hydrogène (130 kPa). Le catalyseur est séparé par filtration sur célite et le filtrat est concentré à sec sous pression réduite. Le résidu est purifié par chromatographie sur 40 g de silice contenus dans une colonne de 2,2 cm de diamètre [éluant : dichlorométhane-méthanol (90/10 en volumes)]. Les fractions contenant le produit attendu sont réunies et concentrées sous pression réduite. On obtient ainsi 0,95 g d'acide ({[(amino-4 phényl)sulfonyl-4 tert-butoxycarbonyl-2 (fluoro-2 phényl)-5 pyrrolidinyl-1]-2 oxo-2 éthyl}-3 uréido)-3 benzolque-(2RS,4SR,5RS) [Rf = 0,2 ; éluant : chlorure de méthylène-méthanol (90/10); Spectre de R.M.N. ¹H (200 MHz, (CD₃)₂SO avec ajout de quelques gouttes de CD₃COOD, à une température de 393 K, δ en ppm) : 1,55 (s, 9H : C(CH₃)₃); de 2,30 à 2,55 et 2,76 (2 mts, 1H chacun : CH₂ en 3); 3,68 et 4,00 (respectivement d large et d, J = 17 Hz, 1H chacun : COCH₂N); 3,84 (mt, 1H : H4); 4,62 (t, J = 8Hz, 1H : H2); 5,72 (d, J = 3 Hz, 1H : H 5); 6,76 (d,J= 8 Hz, 2H : H de l'aromatique en 4 (H en ortho du NH₂)); de 7,00 à 7,70 (mt, 6H : H aromatiques); 7,58 (d, J = 8 Hz, 2H : H de l'aromatique en 4 (H en méta du NH₂)); 7,82 (t large, J = 8 Hz, 1H : H de l'aromatique en 5 (H 6)); 8,00 (t, J = 1,5 Hz, 1H : H aromatique (H 2 : en ortho du COOH))].

B Le {[(benzyloxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) peut être préparé comme décrit à l'exemple 1B, mais à partir de 1,35 g de (fluoro-2 phényl)-5 (nitro-4 phényl)sulfonyl-4 pyrrolidinecarboxylate-2 de tert-butyle-(2RS,4SR,5RS), de 0,99 g d'acide [(benzyloxycarbonyl-3 phényl)-3 uréido]-2 acétique et de 0,62 g de N,N'-dicyclohexylcarbodiimide dans 50 cm³ d'acétonitrile. Après traitement, on obtient 1,7 g de {[(benzyloxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) sous forme d'une meringue utilisée telle quelle dans les synthèses ultérieures.

### Exemple 7

A On opère comme décrit à l'exemple 2A, mais à partir de 1,6 g d'(acétamido-4 phényl)sulfonyl-4 {([(benzyloxycarbonyl-3 phényl)-3 uréido]-2 acétyl}-1 (fluoro-2 phényl)-5 pyrrolidinecarboxylate-2 de tert-butyle-(2RS,4SR,5RS), de 0,2 g de palladium à 10% sur charbon et de 100 cm³ d'éthanol sous atmosphère d'hydrogène (130 kPa). Après traitement, on obtient 0,9 g d'acide ({[(acétamido-4 phényl)sulfonyl-4 tert-butoxycarbonyl-2 (fluoro-2 phényl)-5 pyrrolidinyl-1]-2 oxo-2 éthyl}-3 uréido)-3 benzoïque-(2RS,4SR,5RS) [Rf = 0,3 ; éluant: chlorure de méthylène-méthanol (90/10); Spectre de R.M.N. ^{1H} (300 MHz, (CD₃)₂SO, à une température de 393 K, δ en ppm) : 1,55 (s, 9H : C(CH₃)₃); 2,12 (s, 3H : COCH₃); 2,44 et 2,79 (2 mts, 1H chacun : CH₂ en 3); 3,68 et 3,99 (respectivement mt et dd (J = 17 et 4,5 Hz), 1H chacun : COCH₂N); 4,03 (mt, 1H : H 4); 4,65 (t large, J = 7 Hz, 1H : H 2); 5,72 (d, J = 3 Hz, 1H : H 5); 6,20 (t, J = 4,5 Hz, 1H : NHCO); de 7,05 à 7,65 (mt, 6H : H aromatiques); 7,80 (t large, J = 8 Hz, 1H : H de l'aromatique en 5 (H 6)); 7,88 (AB limite, J = 7,5 Hz, 4H : H de l'aromatique en 4); 8,00 (mt, 1H : H aromatique (H 2 : en ortho du COOH)); 8,65 (s, 1H : CONHAr); 10,03 (s, 1H : ArNH)].

B L'(acétamido-4 phényl)sulfonyl-4 {[(benzyloxycarbonyl-3 phényl)-3 uréido]-2 acétyl}-1 (fluoro-2 phényl)-5 pyrrolidinecarboxylate-2 de tert-butyle-(2RS,4SR,5RS) peut être préparé comme décrit à l'exemple 1B, mais à partir de 1,16 g d'(acétamido-4 phényl)sulfonyl-4 (fluoro-2 phényl)-5 pyrrolidinecarboxylate-2 de tert-butyle-(2RS,4SR,5RS), de 0,82 g d'acide [(benzyloxycarbonyl-3 phényl)-3 uréido]-2 acétique et de 0,52 g de N,N'-dicyclohexylcarbodiimide dans 100 cm3 d'acétonitrile. Après traitement, on obtient 1,6 g d'(acétamido-4 phényl)sulfonyl-4 {[(benzyloxycarbonyl-3 phényl)-3 uréido]-2 acétyl}-1 (fluoro-2 phényl)-5 pyrrolidinecarboxylate-2 de tert-butyle-(2RS,4SR,5RS) sous forme d'une meringue utilisée telle quelle dans les synthèses ultérieures.

C L'(acétamido-4 phényl)sulfonyl-4 (fluoro-2 phényl)-5 pyrrolidinecarboxylate-2 de tert-butyle-(2RS,4SR,5RS) peut être préparé comme décrit à l'exemple 1C, mais à partir de 4,75 g de (fluoro-2 benzylidèneamino)acétate de tert-butyle, de 9,5 g d'(acétamido-4 phényl)vinylsulfone, de 5 g d'acétate d'argent et de 3,65 cm³ de triéthylamine dans 200 cm³ d'acétonitrile. Après traitement, on obtient 4 g d'(acétamido-4 phényl)sulfonyl-4 (fluoro-2 phényl)-5 pyrrolidinecarboxylate-2 de tert-butyle-(2RS,4SR,5RS) sous forme d'une meringue utilisée telle quelle dans les synthèses ultérieures et 1,6 g d'(acétamido-4 phényl)sulfonyl-4 (fluoro-2 phényl)-5 pyrrolidinecarboxylate-2 de tert-butyle-(2RS,4RS,5RS) fondant à 160°C.

D L'(acétamido-4 phényl)vinylsulfone peut être préparée comme décrit à l'exemple 1E, mais à partir de 22 g d'(acétamido-4 phényl)(chloro-2 éthyl)sulfone, de 17,8 cm³ de triéthylamine et de 260 cm³ de tétrahydrofuranne. Après traitement, on obtient 12,7 g d'(acétamido-4 phényl)vinylsulfone fondant à 122°C.

E L'(acétamido-4 phényl)(chloro-2 éthyl)sulfone peut être préparée comme décrit à l'exemple 1F, mais à partir de 23 g d'(acétamido-4 phényl)(chloro-2 éthyl)sulfure, de 28,6 cm³ d'une solution aqueuse à 30% de peroxyde d'hydrogène et de 150 cm³ d'acide acétique. Après traitement, on obtient 22 g d'(acétamido-4 phényl)(chloro-2 éthyl)sulfone fondant à 188°C.

F L'(acétamido-4 phényl)(chloro-2 éthyl)sulfure peut être préparé comme décrit à l'exemple 1G, mais à partir de 17,6 g d'acétamido-4 thiophénol, de 0,24 cm³ d'aliquat, de 4,94 g d'hydroxyde de sodium, de 62 cm³ d'eau distillée et de 114 cm³ de dichloro-1,2 éthane. Après traitement, on obtient 23 g d'(acétamido-4 phényl)(chloro-2 éthyl)sulfure sous forme d'un solide fondant à 146°C.

### Exemple 8

A On opère comme décrit à l'exemple 1A, mais à partir de 1,5 g de (diméthylamino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) et de 0,12 g d'hydroxyde de potassium dans un mélange de 60 cm³ de méthanol et de 20 cm³ d'eau distillée. Après traitement, on obtient 0,12 g d'acide ({[tert-butoxycarbonyl-2 (diméthylamino-4 phényl)sulfonyl-4 (fluoro-2 phényl)-5 pyrrolidinyl-1]-2 oxo-2 éthyl}-3 uréido)-3 benzoïque-(2RS,4SR,5RS) [Rf = 0,4 ; éluant : chlorure de méthylène-méthanol (90/10); Spectre de R.M.N. ¹H (250 MHz, (CD₃)₂SO avec ajout de quelques gouttes de CD₃COOD, à une température de 393 K, δ en ppm) : 1,52 (s, 9H : C(CH₃)₃); 2,43 et 2,77 (2 mts, 1H chacun : CH₂ en 3); 3,04 (s, 6H : N(CH₃)₂); 3,63 et 3,99 (respectivement d large et d, J = 17 Hz, 1H chacun : COCH₂N); 3,86 (mt, 1H : H 4); 4,56 (t large, J = 8 Hz, 1H : H 2); 5,71 (d, J = 3 Hz, 1H : H 5); 6,81 (d, J = 8,5 Hz, 2H : H de l'aromatique en 4 (H en ortho du N(CH₃)₂)); de 7,00 à 7,70 (mt, 6H : H aromatiques); 7,68 (d, J = 8,5 Hz, 2H : H de l'aromatique en 4 (H en méta du N(CH₃)₂)); 7,80 (mt 1H : H de l'aromatique en 5 (H 6)); 8,00 (mt, 1H : H aromatque (H 2 : en ortho du COOH))].

B Le (diméthylamino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) peut être préparé de la manière suivante : à une solution de 1,8 g d'(amino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) dans 50 cm³ d'éthanol, on ajoute successivement 4 cm³ de formaldéhyde et 0,2 g de palladium à 10% sur charbon. La suspension est agitée pendant vingt heures à une température voisine de 20°C sous atmosphère d'hydrogène (130 kPa). Le catalyseur est séparé par filtration sur célite et le filtrat est concentré à sec sous pression réduite. Le résidu est purifié par chromatographie sur 60 g de silice contenus dans une colonne de 3,2 cm de diamètre [éluant : acétate d'éthyle-cyclohexane (70/30 en volumes)]. Les fractions contenant le produit attendu sont réunies et concentrées sous pression réduite. On obtient ainsi 1,5 g de (diméthylamino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) sous forme d'une huile jaune utilisée telle quelle dans les synthèses ultérieures.

C L'(amino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) peut être préparé de la manière suivante : à une solution de 1,9 g d'{[(éthoxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) dans 100 cm³ d'éthanol, on ajoute 0,2 g de palladium à 10% sur charbon. La suspension est agitée pendant vingt heures à une température voisine de 20°C sous atmosphère d'hydrogène (130 kPa). Le catalyseur est séparé par filtration sur célite et le filtrat est concentré à sec sous pression réduite. On obtient ainsi 1,8 g d'(amino-4 phényl)sulfonyl-4 {[(éthoxycarbonyl-3 phényl)-3 uréido]-2 acétyl}-1 (fluoro-2 phényl)-5 pyrrolidinecarboxylate-2 de tert-butyle-(2RS,4SR,5RS) sous forme d'une huile brune utilisée telle quelle dans les synthèses ultérieures.

D L'{[(éthoxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) peut être préparé comme décrit à l'exemple 1B, mais à partir de 1,2 g de (fluoro-2 phényl)-5 (nitro-4 phényl)sulfonyl-4 pyrrolidinecarboxylate-2 de tert-butyle-(2RS,4SR,5RS), de 0,71 g d'acide [(éthoxycarbonyl-3 phényl)-3 uréido]-2 acétique et de 0,55 g de N,N'-dicyclohexylcarbodiimide dans 50 cm³ d'acétonitrile. Après traitement, on obtient 1,9 g d'{[(éthoxycarbonyl-3 phényl)-3 uréido]-2 acétyl}-1 (fluoro-2 phényl)-5 (nitro-4 phényl)sulfonyl-4 pyrrolidinecarboxylate-2 de tert-butyle-(2RS,4SR,5RS) sous forme d'une meringue utilisée telle quelle dans les synthèses ultérieures.

E L'acide [(éthoxycarbonyl-3 phényl)-3 uréido]-2 acétique peut être préparé comme décrit à l'exemple 1H, mais à partir de 10 g d'isocyanato-3 benzoate d'éthyle, de 3,95 g de glycine et de 4,4 g d'hydrogénocarbonate de sodium dans 60 cm³ d'eau distillée. Après traitement, on obtient 5,3 g d'acide [(éthoxycarbonyl-3 phényl)-3 uréido]-2 acétique fondant à 174°C.

### Exemple 9

A On opère comme décrit à l'exemple 2A, mais à partir de 3,4 g de {([(benzyloxycarbonyl-3 phényl)-3 uréido]-2 acétyl}-1 (fluoro-2 phényl)-5 méthylsulfonyl-4 pyrrolidinecarboxylate-2 de tert-butyle-(2RS,4SR,5RS), de 0,3 g de palladium à 10% sur charbon et de 100 cm³ d'éthanol sous atmosphère d'hydrogène (130 kPa). Après traitement, on obtient 2,4 g d'acide ({[tert-butoxycarbonyl-2 (fluoro-2 phényl)-5 méthylsulfonyl-4 pyrrolidinyl-1]-2 oxo-2 éthyl)-3 uréido)-3 benzoïque-(2RS,4SR,5RS) [Rf = 0,3 ; éluant : chlorure de méthylène-méthanol (90/10); Spectre de R.M.N. ¹H (200 MHz, (CD₃)₂SO avec ajout de quelques gouttes de CD₃COOD, à une température de 373 K, δ en ppm) : 1,53 (s, 9H : C(CH₃)₃); de 2,45 et 2,88 (2 mts, 1H chacun : CH₂ en 3); 3,10 (s, 3H : SO₂CH₃); 3,70 et 4,02 (respectivement d large et d, J = 17 Hz, 1H chacun : COCH₂N); 3,96 (mt, 1H : H 4); 4,67 (t, J = 8 Hz, 1H : H 2); 5,83 (d, J = 3 Hz, 1H : H 5); de 7,10 à 7,65 (mt, 6H : H aromatiques); 7,92 (t large, J = 8 Hz, 1H : H de l'aromatique en 5 (H 6)); 7,98 (t, J = 1,5 Hz, 1H : H aromatique (H 2 en ortho du COOH))].

B Le {[(benzyloxycarbonyl-3 phényl)-3 uréido]-2 acétyl}-1 (fluoro-2 phényl)-5 méthylsulfonyl-4 pyrrolidinecarboxylate-2 de tert-butyle-(2RS,4SR,5RS) peut être préparé comme décrit à l'exemple 1B, mais à partir de 2,2 g de (fluoro-2 phényl)-5 méthylsulfonyl-4 pyrrolidinecarboxylate-2 de tert-butyle-(2RS,4SR,5RS), de 2,1 g d'acide [(benzyloxycarbonyl-3 phényl)-3 uréido]-2 acétique et de 1,33 g de N,N'-dicyclohexylcarbodiimide dans 100 cm³ d'acétonitrile. Après traitement, on obtient 3,4 g de {[(benzyloxycarbonyl-3 phényl)-3 uréido]-2 acétyl}-1 (fluoro-2 phényl)-5 méthylsulfonyl-4 pyrrolidinecarboxylate-2 de tert-butyle-(2RS,4SR,5RS) sous forme d'une meringue utilisée telle quelle dans les synthèses ultérieures.

C Le (fluoro-2 phényl)-5 méthylsulfonyl-4 pyrrolidinecarboxylate-2 de tert-butyle-(2RS,4SR,5RS) peut être préparé comme décrit à l'exemple 1C, mais à partir de 4,8 g de (fluoro-2 benzylidèneamino)acétate de tert-butyle, de 1,8 cm³ de méthylvinylsulfone, de 5 g d'acétate d'argent et de 3,5 cm³ de triéthylamine dans 200 cm³ d'acétonitrile. Après traitement, on obtient 2,2 g de (fluoro-2 phényl)-5 méthylsulfonyl-4 pyrrolidinecarboxylate-2 de tert-butyle-(2RS,4SR,5RS) sous forme d'une huile orangée utilisée telle quelle dans les synthèses ultérieures et 1,35 g de (fluoro-2 phényl)-5 méthylsulfonyl-4 pyrrolidinecarboxylate-2 de tert-butyle-(2RS,4RS,5RS) fondant à 176°C.

### Exemple 10

A On opère comme décrit à l'exemple 1A, mais à partir de 3,35 g de (chloro-4 phényl)sulfonyl-4 (fluoro-2 phényl)-5 isobutylcarbamoyl-2 {[(méthoxycarbonylméthyl-3 phényl)-3 uréido]-2 acétyl}-1 pyrrolidine-(2RS,4SR,5RS) et de 0,36 g d'hydroxyde de potassium dans un mélange de 60 cm³ de méthanol et de 20 cm³ d'eau distillée. Après traitement, on obtient 1,65 g d'acide ({[(chloro-4 phényl)sulfonyl-4 (fluoro-2 phényl)-5 isobutyl-carbamoyl-2 pyrrolidinyl-1]-2 oxo-2 éthyl}-3 uréido)-3 phénylacétique-(2RS,4SR,5RS) [Rf = 0,45 ; éluant: chlorure de méthylène-méthanol (90/10); Spectre de R.M.N. ¹H (200 MHz, (CD₃)₂SO avec ajout de quelques gouttes de CD₃COOD, une température de 373 K, δ en ppm) : 0.94 (d, J = 6,5 Hz, 6H : C(CH₃)₂); 1,85 (mt, 1H : CH); de 2,30 à 2,55 et 2,84 (2 mts, 1H chacun : CH₂ en 3); 3,06 (mt, 2H : NCH₂); 3,50 (s, 2H : ArCH₂COO); 3,67 et 3,88 (respectivement d large et d, J = 17 Hz, 1H chacun : COCH₂N); 4,08 (mt, 1H: H 4); 4,78 (t, J = 8 Hz, 1H : H 2); 5,68 (d, J = 3 Hz, 1H : H 5); 6,12 (t résiuduel: NHCO); 6,85 (d large, J = 7,5 Hz, 1H : H aromatique (H 4 : en ortho du CH₂)); de 6,95 à 7,30 (mt, 6H : H aromatiques); 7,69 (d large, J = 8,5 Hz, 2H : H de l'aromatique en 4 (H en ortho du Cl)); 7,96 (d large, J = 8,5 Hz, 2H : H aromatiques en 4 (H en méta du Cl)); 8,2,0 (t large, J = 8 Hz, 1H : H de l'aromatique en 5 (H 6)); 8,40 (s résiduel: CONHAr)].

B La (chloro-4 phényl)sulfonyl-4 (fluoro-2 phényl)-5 isobutylcarbamoyl-2 {([(méthoxycarbonylméthyl-3 phényl)-3 uréido]-2 acétyl}-1 pyrrolidine-(2RS,4SR,5RS) peut être préparée comme décrit à l'exemple 1B, mais à partir de 2,2 g de (chloro-4 phényl)sulfonyl-4 (fluoro-2 phényl)-5 isobutylcarbamoyl-2 pyrrolidine-(2RS,4SR,5RS), de 1,31 g d'acide [(méthoxycarbonylméthyl-3 phényl)-3 uréido]-2 acétique et de 1,1 g de N,N'-dicyclohexylcarbodiimide dans 50 cm³ d'acétonitrile. Après traitement, on obtient 3,35 g de (chloro-4 phényl)sulfonyl-4 (fluoro-2 phényl)-5 isobutylcarbamoyl-2 {[(méthoxycarbonylméthyl-3 phényl)-3 uréido]-2 acétyl}-1 pyrrolidine-(2RS,4SR,5RS) sous forme d'une meringue utilisée telle quelle dans les synthèses ultérieures.

C La (chloro-4 phényl)sulfonyl-4 (fluoro-2 phényl)-5 isobutylcarbamoyl-2 pyrrolidine-(2RS,4SR,5RS) peut être préparée comme décrit à l'exemple 1C, mais à partir de 7,6 g de (fluoro-2 benzylidène)amino-2 N-isobutyl-acétamide, de 6,1 g de (chloro-4 phényl)vinylsulfone, de 7,52 g d'acétate d'argent et de 5,1 cm³ de triéthylamine dans 200 cm³ d'acétonitrile. Après traitement, on obtient 2,2 g de (chloro-4 phényl) sulfonyl-4 (fluoro-2 phényl)-5 isobutylcarbamoyl-2 pyrrolidine-(2RS,4SR,5RS) sous forme d'une huile utilisée telle quelle dans les synthèses ultérieures et 1,3 g de (chloro-4 phényl)sulfonyl-4 (fluoro-2 phényl)-5 isobutylcarbamoyl-2 pyrrolidine-(2RS,4RS,5RS) fondant à 136°C.

D La (fluoro-2 benzylidène)amino-2 N-isobutyl-acétamide peut être préparée comme décrit à l'exemple 1D, mais à partir de 3,2 cm³ de fluoro-2 benzaldéhyde, de 5 g de chlorhydrate d'amino-2 N-isobutyl-acétamide, de 6 g de tamis 4Å et de 4,2 cm³ de triéthylamine dans 90 cm³ de dichlorométhane. Après traitement, on obtient 7,6 g de (fluoro-2 benzylidène)amino-2 N-isobutyl-acétamide sous forme d'une huile utilisée telle quelle dans les synthèses ultérieures.

E Le chlorhydrate d'amino-2 N-isobutyl-acétamide peut être préparé de la manière suivante : une solution de 15 g de chloro-2 N-isobutyl-acétamide dans 145 cm³ d'une solution méthanolique 7N d'ammoniac est agitée pendant soixante-douze heures à une température voisine de 20°C. Le milieu réactionnel est ensuite concentré sous pression réduite et le résidu cristallisé dans 50 cm³ d'acétonitrile. Le solide est séparé par filtration et séché sous pression réduite. On obtient ainsi 9,3 g de chlorhydrate d'amino-2 N-isobutyl-acétamide fondant à 154°C.

F La chloro-2 N-isobutyl-acétamide peut être préparée de la manière suivante : à une solution de 10,2 cm³ d'isobutylamine dans 50 cm³ de dichloro-1,2 éthane, on ajoute 25 cm³ d'une solution aqueuse à 20% d'hydroxyde de sodium. Le mélange est refroidi à une température voisine de - 20°C puis on ajoute goutte à goutte 9,9 cm³ de chlorure de chloracétyle. Le milieu réactionnel est agité pendant deux heures à une température voisine de -20°C puis pendant vingt heures à une température voisine de 20°C. On ajoute ensuite 100 cm³ d'eau distillée et on extrait par trois fois 100 cm³ de dichlorométhane. Les phases organiques sont réunies, lavées par deux fois 100 cm³ d'une solution aqueuse à 5% d'acide chlorhydrique, deux fois 100 cm³ d'une solution aqueuse à 10% d'hydrogénocarbonate de sodium et deux fois 100 cm³ d'une solution aqueuse saturée en chlorure de sodium puis séchées sur sulfate de magnésium et concentrées sous pression réduite. On obtient ainsi 15 g de chloro-2 N-isobutyl-acétamide sous forme d'une huile jaune utilisée telle quelle dans les synthèses ultérieures.

### Exemple 11

A On opère comme décrit à l'exemple 2A, mais à partir de 3,2 g de {([(benzyloxycarbonyl-3 phényl)-3 uréido]-2 acétyl}-1 (fluoro-2 phényl)-5 (fluoro-2 phényl)sulfonyl-4 isobutylcarbamoyl-2 pyrrolidine-(2RS,4SR,5RS), de 0,35 g de palladium à 10% sur charbon et de 100 cm³ d'éthanol sous atmosphère d'hydrogène (130 kPa). Après traitement, on obtient 2,3 g d'acide ({[(fluoro-2 phényl)-5 (fluoro-2 phényl)sulfonyl-4 isobutylcarbamoyl-2 pyrrolidinyl-1]-2 oxo-2 éthyl}-3 uréido)-3 benzoïque-(2RS,4SR,5RS) [Rf = 0,3; éluant : chlorure de méthylène-méthanol (90/10); Spectre de R.M.N. ¹H (200 MHz, (CD₃)₂SO avec ajout de quelques gouttes de CD₃COOD, à une température de 383 K, δ en ppm) : 0.96 (d, J = 6,5 Hz, 6H : C(CH₃)₂); 1,88 (mt, 1H : CH); de 2,45 à 2,65 et 2,90 (2 mts, 1H chacun : CH₂ en 3); 3,09 (mt, 2H : NCH₂); 3,70 et 3,90 (respectivement d large et d, J = 17 Hz, 1H chacun : COCH₂N); 4,14 (mt, 1H : H 4); 4,86 (t, J = 8 Hz, 1H : H 2); 5,70 (d, J = 3 Hz, 1H : H 5); de 6,90 à 8,00 (mt, 10H : H aromatiques); 8,00 (mt, 1H: H Aromatique (H 2 : en ortho du COOH)); 8,2,0 (t large, J = 8 Hz, 1H : H de l'aromatique en 5 (H 6))].

B La {[(benzyloxycarbonyl-3 phényl)-3 uréido]-2 acétyl}-1 (fluoro-2 phényl)-5 (fluoro-2 phényl)sulfonyl-4 isobutylcarbamoyl-2 pyrrolidine-(2RS,4SR,5RS) peut être préparée comme décrit à l'exemple 1B, mais à partir de 2,7 g de (fluoro-2 phényl)-5 (fluoro-2 phényl)sulfonyl-4 isobutylcarbamoyl-2 pyrrolidine-(2RS,4SR,5RS), de 2,1 g d'acide [(benzyloxycarbonyl-3 phényl)-3 uréido]-2 acétique et de 1,32 g de N,N'-dicyclohexylcarbodiimide dans 75 cm³ d'acétonitrile. Après traitement, on obtient 3,2 g de {[(benzyloxycarbonyl-3 phényl)-3 uréido]-2 acétyl}-1 (fluoro-2 phényl)-5 (fluoro-2 phényl)sulfonyl-4 isobutylcarbamoyl-2 pyrrolidine-(2RS,4SR,5RS) sous forme d'une meringue utilisée telle quelle dans les synthèses ultérieures.

C La (fluoro-2 phényl)-5 (fluoro-2 phényl)sulfonyl-4 isobutylcarbamoyl-2 pyrrolidine-(2RS,4SR,5RS) peut être préparée comme décrit à l'exemple 1C, mais à partir de 6,3 g de (fluoro-2 benzylidène)amino-2 N-isobutyl-acétamide, de 4,7 g de (fluoro-2 phényl)vinylsulfone, de 6,32 g d'acétate d'argent et de 4,2 cm³ de triéthylamine dans 200 cm³ d'acétonitrile. Après traitement, on obtient 2,7 g de (fluoro-2 phényl)-5 (fluoro-2 phényl)sulfonyl-4 isobutylcarbamoyl-2 pyrrolidine-(2RS,4SR,5RS) sous forme d'une huile utilisée telle quelle dans les synthèses ultérieures et 1,55 g de (fluoro-2 phényl)-5 (fluoro-2 phényl)sulfonyl-4 isobutylcarbamoyl-2 pyrrolidine-(2RS,4RS,5RS) fondant à 128°C.

### Exemple 12

A On opère comme décrit à l'exemple 1A, mais à partir de 3 g de (fluoro-2 phényl)-5 isobutylcarbamoyl-2 {[(méthoxycarbonyl-3 phényl)-3 uréido]-2 acétyl}-1 (méthoxy-3 phényl)sulfonyl-4 pyrrolidine-(2RS,4SR,5RS) et de 0,245 g d'hydroxyde de potassium dans un mélange de 60 cm³ de méthanol et de 20 cm³ d'eau distillée. Après traitement, on obtient 0,5 g d'acide ({[(fluoro-2 phényl)-5 isobutylcarbamoyl-2 (méthoxy-3 phényl)sulfonyl-4 pyrrolidinyl-1]-2 oxo-2 éthyl}-3 uréido)-3 benzoïque-(2RS,4SR,5RS) [Rf = 0,4 ; éluant : chlorure de méthylène-méthanol (90/10); Spectre de R.M.N. ¹H (200 MHz, (CD₃)₂SO avec ajout de quelques gouttes de CD₃COOD, à une température de 383 K, δ en ppm) : 0.95 (d, J = 6,5 Hz, 6H : C(CH₃)₂); 1,86 (mt, 1H : CH); de 2,35 à 2,55 et 2,86 (2 mts, 1H chacun : CH₂ en 3); 3,08 (mt, 2H : NCH₂); de 3,60 à 3,80 et 3,90 (respectivement mt et d (J = 17 Hz), 1H chacun : COCH₂N); 3,91 (s, 3H : OCH₃); 4,09 (mt, 1H : H 4); 4,81 (t, J = 7 Hz, 1H : H 2); 5,72 (s très large, 1H : H 5); de 6,95 à 7,65 (mt, 10H : H aromatiques); 8,00 (mt, 1H : H Aromatique (H 2 : en ortho du COOH)); 8,20 (t large, J = 8 Hz, 1H : H de l'aromatique en 5 (H 6))].

B La (fluoro-2 phényl)-5 isobutylcarbamoyl-2 1(méthoxycarbonyl-3 phényl)-3 uréido]-2 acétyl}-1 (méthoxy-3 phényl)sulfonyl-4 pyrrolidine-(2RS,4SR,5RS) peut être préparée comme décrit à l'exemple 1B, mais à partir de 2,2 g de (fluoro-2 phényl)-5 isobutylcarbamoyl-2 (méthoxy-3 phényl)sulfonyl-4 pyrrolidine-(2RS,4SR,5RS), de 1,35 g d'acide [(méthoxycarbonyl-3 phényl)-3 uréido]-2 acétique et de 1,1 g de N,N'-dicyclohexylcarbodiimide dans 100 cm³ d'acétonitrile. Après traitement, on obtient 3 g de (fluoro-2 phényl)-5 isobutylcarbamoyl-2 {[(méthoxycarbonyl-3 phényl)-3 uréido]-2 acétyl}-1 (méthoxy-3 phényl)sulfonyl-4 pyrrolidine-(2RS,4SR,5RS) sous forme d'une meringue utilisée telle quelle dans les synthèses ultérieures.

C La (fluoro-2 phényl)-5 isobutylcarbamoyl-2 (méthoxy-3 phényl)sulfonyl-4 pyrrolidine-(2RS,4SR,5RS) peut être préparée comme décrit à l'exemple 1C, mais à partir de 6,3 g de (fluoro-2 benzylidène)amino-2 N-isobutyl-acétamide, de 5 g de (méthoxy-3 phényl)vinylsulfone, de 6,3 g d'acétate d'argent et de 4,6 cm³ de triéthylamine dans 150 cm³ d'acétonitrile. Après traitement, on obtient 3,8 g de (fluoro-2 phényl)-5 isobutylcarbamoyl-2 (méthoxy-3 phényl)sulfonyl-4 pyrrolidine-(2RS,4SR,5RS) sous forme d'une huile jaune utilisée telle quelle dans les synthèses ultérieures et 1,7 g de chlorhydrate de (fluoro-2 phényl)-5 isobutylcarbamoyl-2 (méthoxy-3 phényl)sulfonyl-4 pyrrolidine-(2RS,4RS,5RS) fondant à 182°C.

### Exemple 13

A On opère comme décrit à l'exemple 1A, mais à partir de 3 g d'[(éthoxycarbonyl-3 phényl)-3 uréido]-2 acétyl}-1 (fluoro-2 phényl)-5 isobutylcarbamoyl-2 (méthyl-4 phényl)sulfonyl-4 pyrrolidine-(2RS,4SR,5RS) et de 0,252 g d'hydroxyde de potassium dans un mélange de 80 cm³ de méthanol et de 20 cm³ d'eau distillée. Après traitement, on obtient 0,18 g d'acide ({[(fluoro-2 phényl)-5 isobutylcarbamoyl-2 (méthyl-4 phényl)sulfonyl-4 pyrrolidinyl-1]-2 oxo-2 éthyl}-3 uréido)-3 benzoique-(2RS,4SR,5RS) [Rf = 0,6 ; éluant : chlorure de méthylène-méthanol (90/10); Spectre de R.M.N. ¹H (200 MHz, (CD₃)₂SO avec ajout de quelques gouttes de CD₃COOD, à une température de 393 K, δ en ppm) : 0,96 (d, J = 6,5 Hz, 6H : C(CH₃)₂); 1,85 (mt, 1H : CH); de 2,30 à 2,55 et 2,82 (2 mts, 1H chacun : CH₂ en 3); 2,48 (s, 3H : CH₃); 3,07 (mt, 2H : NCH₂); de 3,60 à 3,80 et 3,88 (respectivement mt et d (J = 17 Hz), 1H chacun : COCH₂N); 4,00 (mt, 1H : H 4); 4,78 (t, J = 7 Hz, 1H : H 2); 5,71 (s très large, 1H : H 5); de 6,95 à 7,65 (mt, 6H : H aromatiques); 7,49 (d, J = 7,5 Hz, 2H : H de l'aromatique en 4 (H en ortho du CH₃)); 7,86 (d, J = 7,5 Hz, 2H : H de l'aromatique en 4 (H en méta du CH₃)); 8,02 (mt, 1H: H Aromatique (H 2: en ortho du COOH)); 8,22 (t large, J = 8 Hz, 1H : H de l'aromatique en 5 (H 6))].

B L'[(éthoxycarbonyl-3 phényl)-3 uréido]-2 acétyl}-1 (fluoro-2 phényl)-5 isobutylcarbamoyl-2 (méthyl-4 phényl)sulfonyl-4 pyrrolidine-(2RS,4SR,5RS) peut être préparée comme décrit à l'exemple 1B, mais à partir de 2,1 g de (fluoro-2 phényl)-5 isobutylcarbamoyl-2 (méthyl-4 phényl)sulfonyl-4 pyrrolidine-(2RS,4SR,5RS), de 1,35 g d'acide [(éthoxycarbonyl-3 phényl)-3 uréido]-2 acétique et de 1,1 g de N,N'-dicyclohexylcarbodiimide dans 100 cm³ d'acétonitrile. Après traitement, on obtient 3 g d'[(éthoxycarbonyl-3 phényl)-3 uréido]-2 acétyl}-1 (fluoro-2 phényl)-5 isobutylcarbamoyl-2 (méthyl-4 phényl)sulfonyl-4 pyrrolidine-(2RS,4SR,5RS) sous forme d'une meringue utilisée telle quelle dans les synthèses ultérieures.

C La (fluoro-2 phényl)-5 isobutylcarbamoyl-2 (méthyl-4 phényl) sulfonyl-4 pyrrolidine-(2RS,4SR,5RS) peut être préparée comme décrit à l'exemple 1C, mais à partir de 10,1 g de (fluoro-2 benzylidène)amino-2 N-isobutyl-acétamide, de 7,3 g de (méthyl-4 phényl)vinylsulfone, de 10,12 g d'acétate d'argent et de 7,3 cm³ de triéthylamine dans 200 cm³ d'acétonitrile. Après traitement, on obtient 5 g de (fluoro-2 phényl)-5 isobutylcarbamoyl-2 (méthyl-4 phényl)sulfonyl-4 pyrrolidine-(2RS,4SR,5RS) sous forme d'une huile jaune utilisée telle quelle dans les synthèses ultérieures et 1,9 g de chlorhydrate de (fluoro-2 phényl)-5 isobutylcarbamoyl-2 (méthyl-4 phényl)sulfonyl-4 pyrrolidine-(2RS,4RS,5RS) fondant à 234°C.

### Exemple 14

A On opère comme décrit à l'exemple 1A, mais à partir de 2 g de (fluoro-2 phényl)-5 isobutylcarbamoyl-2 {[(méthoxycarbonyl-3 phényl)-3 uréido]-2 acétyl}-1 (nitro-4 phényl)sulfonyl-4 pyrrolidine-(2RS,4SR,5RS) et de 0,16 g d'hydroxyde de potassium dans un mélange de 60 cm³ de méthanol et de 20 cm³ d'eau distillée. Après traitement, on obtient 0,9 g d'acide ({[(fluoro-2 phényl)-5 isobutylcarbamoyl-2 (nitro-4 phényl)sulfonyl-4 pyrrolidinyl-1]-2 oxo-2 éthyl}-3 uréido)-3 benzoïque-(2RS,4SR,5RS) [Rf = 0,2; éluant : chlorure de méthylène-méthanol (90/10); Spectre de R.M.N. ¹H (200 MHz, (CD₃)₂SO avec ajout de quelques gouttes de CD₃COOD, à une température de 383 K, δ en ppm) : 0,94 (d, J = 6,5 Hz, 6H : C(CH₃)₂); 1,89 (mt, 1H : CH); de 2,65 à 3,00 (2 mts, 1H chacun : CH₂ en 3); 3,08 (mt, 2H : NCH₂); 3,72 et 3,91 (respectivement d large et d, J = 17 Hz, 1H chacun : COCH₂N); 4,21 (mt, 1H: H 4); 4,83 (t, J = 7,5 Hz, 1H: H 2); 5,71 (d, J = 3 Hz, 1H : H 5); de 6,95 à 7,65 (mt, 6H : H aromatiques); 8,02 (mt, 1H: H Aromatique (H 2: en ortho du COOH)); 8,18 (t large, J = 8 Hz, 1H: H de l'aromatique en 5 (H 6)); 8,25 (d, J = 8,5 Hz, 2H : H de l'aromatique en 4 (H en méta du NO₂)); 8,42 (d, J = 8,5 Hz, 2H : H de l'aromatique en 4 (H en ortho du NO₂))].

B La (fluoro-2 phényl)-5 isobutylcarbamoyl-2 {[(méthoxycarbonyl-3 phényl)-3 uréido]-2 acétyl}-1 (nitro-4 phényl)sulfonyl-4 pyrrolidine-(2RS,4SR,5RS) peut être préparée comme décrit à l'exemple 1B, mais à partir de 1,35 g de (fluoro-2 phényl)-5 isobutylcarbamoyl-2 (nitro-4 phényl)sulfonyl-4 pyrrolidine-(2RS,4SR,5RS), de 0,76 g d'acide [(méthoxycarbonyl-3 phényl)-3 uréido]-2 acétique et de 0,62 g de N,N'-dicyclohexylcarbodiimide dans 50 cm³ d'acétonitrile. Après traitement, on obtient 2 g de (fluoro-2 phényl)-5 isobutylcarbamoyl-2 {[(méthoxycarbonyl-3 phényl)-3 uréido]-2 acétyl}-1 (nitro-4 phényl)sulfonyl-4 pyrrolidine-(2RS,4SR,5RS) sous forme d'une meringue utilisée telle quelle dans les synthèses ultérieures.

C La (fluoro-2 phényl)-5 isobutylcarbamoyl-2 (nitro-4 phényl)sulfonyl-4 pyrrolidine-(2RS,4SR,5RS) peut être préparée comme décrit à l'exemple 1C, mais à partir de 5,05 g de (fluoro-2 benzylidène)amino-2 N-isobutyl-acétamide, de 4,9 g de (nitro-4 phényl)vinylsulfone, de 5,01 g d'acétate d'argent et de 3,65 cm3 de triéthylamine dans 150 cm³ d'acétonitrile. Après traitement, on obtient 2,7 g de (fluoro-2 phényl)-5 isobutylcarbamoyl-2 (nitro-4 phényl)sulfonyl-4 pyrrolidine-(2RS,4SR,5RS) fondant à 156°C et 1,85 g de (fluoro-2 phényl)-5 isobutylcarbamoyl-2 (méthyl-4 phényl)sulfonyl-4 pyrrolidine-(2RS,4RS,5RS) fondant à 218°C.

### Exemple 15

A On opère comme décrit à l'exemple 6A, mais à partir de 2 g de {[(benzyloxycarbonyl-3 phényl)-3 uréido]-2 acétyl}-1 (fluoro-2 phényl)-5 isobutylcarbamoyl-2 (nitro-4 phényl)sulfonyl-4 pyrrolidine-(2RS,4SR,5RS), de 0,2 g de palladium à 10% sur charbon et de 100 cm³ d'éthanol sous atmosphère d'hydrogène (130 kPa). Après traitement, on obtient 1,15 g d'acide ({[(amino-4 phényl)sulfonyl-4 (fluoro-2 phényl)-5 isobutylcarbamoyl-2 pyrrolidinyl-1]-2 oxo-2 éthyl}-3 uréido)-3 phénylacétique-(2RS,4SR,5RS) [Rf = 0,3; éluant : chlorure de méthylène-méthanol (90/10); Spectre de R.M.N. ¹H (200 MHz, (CD₃)₂SO , à une température de 383 K, δ en ppm) : 0,97 (d, J = 6,5 Hz, 6H : C(CH₃)₂); 1,87 (mt, 1H: CH); 2,40 et 2,79 (2 mts, 1H chacun : CH₂ en 3); 3,09 (mt, 2H : NCH₂); 3,70 et 3,92 (respectivement d large et dd, J = 17 Hz et J = 17 et 5,5 Hz: 1H chacun : COCH₂N); 3,82 (mt, 1H: H 4); 4,78 (t, J = 7,5 Hz, 1H : H 2); 5,71 (d, J = 3 Hz, 1H : H 5); de 5,60 à 5,90 (mf étalé, 2H : NH₂); 6,15 (t, J = 5,5 Hz, 1H : NHCO); 6,77 (d, J = 8,5 Hz, 2H : H de l'aromatique en 4 (H en ortho du NH₂)); de 7,00 à 7,70 (mt, 6H : H aromatiques); 7,58 (d, J = 8,5 Hz, 2H : H de l'aromatique en 4 (H en méta du NH₂)); 7,90 (mf, 1H: NH); 8,02 (mt, 1H: H Aromatique (H 2 : en ortho du COOH)); 8,20 (t large, J = 8 Hz, 1H : H de l'aromatique en 5 (H 6)); 8,60 (s, 1H : CONHAr)].

B La {[(benzyloxycarbonyl-3 phényl)-3 uréido]-2 acétyl}-1 (fluoro-2 phényl)-5 isobutylcarbamoyl-2 (nitro-4 phényl)sulfonyl-4 pyrrolidine-(2RS,4SR,5RS) peut être préparée comme décrit à l'exemple 1B, mais à partir de 1,31 g de (fluoro-2 phényl)-5 isobutylcarbamoyl-2 (nitro-4 phényl)sulfonyl-4 pyrrolidine-(2RS,4SR,5RS), de 0,97 g d'acide [(benzyloxycarbonyl-3 phényl)-3 uréido]-2 acétique et de 0,62 g de N,N'-di-cyclohexylcarbodiimide dans 50 cm³ d'acétonitrile. Après traitement, on obtient 2 g de {[(benzyloxycarbonyl-3 phényl)-3 uréido]-2 acétyl}-1 (fluoro-2 phényl)-5 isobutylcarbamoyl-2 (nitro-4 phényl)sulfonyl-4 pyrrolidine-(2RS,4SR,5RS) sous forme d'une meringue utilisée telle quelle dans les synthèses ultérieures.

### Exemple 16

A On opère comme décrit à l'exemple 2A, mais à partir de 1,6 g d'(acétamido-4 phényl)sulfonyl-4 {[(benzyloxycarbonyl-3 phényl)-3 uréido]-2 acétyl}-1 (fluoro-2 phényl)-5 isobutylcarbamoyl-2 pyrrolidine-(2RS,4SR,5RS), de 0,2 g de palladium à 10% sur charbon et de 100 cm³ d'éthanol sous atmosphère d'hydrogène (130 kPa). Après traitement, on obtient 1,05 g d'acide ({[(acétamido-4 phényl)sulfonyl-4 (fluoro-2 phényl)-5 isobutylcarbamoyl-2 pyrrolidinyl-1]-2 oxo-2 éthyl}-3 uréido)-3 benzoïque-(2RS,4SR,5RS) [Rf = 0,2; éluant : chlorure de méthylène-méthanol (90/10); Spectre de R.M.N. ¹H (300 MHz, (CD₃)₂SO , à une température de 383 K, δ en ppm) : 0,97 (d, J = 6,5 Hz, 6H : C(CH₃)₂); 1,85 (mt, 1H : CH); 2,15 (s, 3H : COH₃); 2,41 et 2,82 (2 mts, 1H chacun : CH₂ en 3); 3,08 (mt, 2H : NCH₂); 3,70 et 3,89 (respectivement mt et dd (J = 17 et 5,5 Hz), 1H chacun : COCH₂N); 3,98 (mt, 1H: H 4); 4,79 (t, J = 7,5 Hz, 1H: H 2); 5,71 (mt, 1H: H 5); 6,18 (t, J = 5,5 Hz, 1H: NHCO); de 7,00 à 7,65 (mt, 6H : H aromatiques); 7,86 (AB limite, 4H: H de l'aromatique en 4); 8,00 (mt, 1H : H Aromatique (H 2 : en ortho du COOH)); 8,03 (mf, 1H: NH); 8,23 (t large, J = 8 Hz, 1H: H de l'aromatique en 5 (H 6)); 8,67 (s, 1H : CONHAr); 10,03 (s, 1H :ArNH)].

B L'(acétamido-4 phényl)sulfonyl-4 {[(benzyloxycarbonyl-3 phényl)-3 uréido]-2 acétyl}-1 (fluoro-2 phényl)-5 isobutylcarbamoyl-2 pyrrolidine-(2RS,4SR,5RS) peut être préparée comme décrit à l'exemple 1B, mais à partir de 1,16 g d'(acétamido-4 phényl)sulfonyl-4 (fluoro-2 phényl)-5 isobutylcarbamoyl-2 pyrrolidine-(2RS,4SR,5RS), de 0,82 g d'acide [(benzyloxycarbonyl-3 phényl)-3 uréido]-2 acétique et de 0,52 g de N,N'-di-cyclohexylcarbodiimide dans 100 cm³ d'acétonitrile. Après traitement, on obtient 1,6 g d'(acétamido-4 phényl)sulfonyl-4 {[(benzyloxycarbonyl-3 phényl)-3 uréido]-2 acétyl}-1 (fluoro-2 phényl)-5 isobutylcarbamoyl-2 pyrrolidine-(2RS,4SR,5RS) sous forme d'une meringue utilisée telle quelle dans les synthèses ultérieures.

C L'(acétamido-4 phényl)sulfonyl-4 (fluoro-2 phényl)-5 isobutylcarbamoyl-2 pyrrolidine-(2RS,4SR,5RS) peut être préparée comme décrit à l'exemple 1C, mais à partir de 2,55 g de (fluoro-2 benzylidène)amino-2 N-isobutyl-acétamide, de 2,25 g d'(acétamido-4 phényl)vinylsulfone, de 2,5 g d'acétate d'argent et de 1,85 cm³ de triéthylamine dans 100 cm³ d'acétonitrile. Après traitement, on obtient 1,2 g d'(acétamido-4 phényl)sulfonyl-4 (fluoro-2 phényl)-5 isobutylcarbamoyl-2 pyrrolidine-(2RS,4SR,5RS) sous forme d'une meringue utilisée telle quelle dans les synthèses ultérieures et 0,9 g d'(acétamido-4 phényl)sulfonyl-4 (fluoro-2 phényl)-5 isobutylcarbamoyl-2 pyrrolidine-(2RS,4RS,5RS).

### Exemple 17

A On opère comme décrit à l'exemple 2A, mais à partir de 2,5 g de {[(benzyloxycarbonyl-3 phényl)-3 uréido]-2 acétyl}-1 tert-butoxycarbonyl-2 (fluoro-2 phényl)-5 morpholinosulfonyl-4 pyrrolidine-(2RS,4SR,5RS), de 0,25 g de palladium à 10% sur charbon et de 100 cm³ d'éthanol sous atmosphère d'hydrogène (130 kPa). Après traitement, on obtient 1,3 g d'acide ({[tert-butoxycarbonyl-2 (fluoro-2 phényl)-5 morpholinosulfonyl-4 pyrrolidinyl-1]-2 oxo-2 éthyl}-3 uréido)-3 benzoïque-(2RS,4SR,5RS) [Rf = 0,43, éluant : chlorure de méthylène-méthanol (90/10)].

B Le {[(benzyloxycarbonyl-3 phényl)-3 uréido]-2 acétyl}-1 tert-butoxycarbonyl-2 (fluoro-2 phényl)-5 morpholinosulfonyl-4 pyrrolidine-(2RS,4SR,5RS) peut être préparé comme décrit à l'exemple 1B, mais à partir de 2 g de tert-butoxycarbonyl-2 (fluoro-2 phényl)-5 morpholinosulfonyl-4 pyrrolidine-(2RS,4SR,5RS), de 1,58 g d'acide [(benzyloxycarbonyl-3 phényl)-3 uréido]-2 acétique et de 0,99 g de N,N'-dicyclohexylcarbodiimide dans 50 cm³ d'acétonitrile. Après traitement, on obtient 2,5 g de {[(benzyloxycarbonyl-3 phényl)-3 uréido]-2 acétyl}-1 tert-butoxycarbonyl-2 (fluoro-2 phényl)-5 morpholinosulfonyl-4 pyrrolidine-(2RS,4SR,5RS) sous forme d'un solide amorphe utilisé tel quel dans les synthèses ultérieures.

C Le tert-butoxycarbonyl-2 (fluoro-2 phényl)-5 morpholinosulfonyl-4 pyrrolidine-(2RS,4SR,5RS) peut être préparée comme décrit à l'exemple 1C, mais à partir de 4,8 g de (fluoro-2 benzylidèneamino)acétate de tert-butyle, de 3,75 g de vinylsulfonyl-1 morpholine, de 5 g d'acétate d'argent et de 3,6 cm³ de triéthylamine dans 200 cm³ d'acétonitrile. Après traitement, on obtient 2 g de tert-butoxycarbonyl-2 (fluoro-2 phényl)-5 morpholinosulfonyl-4 pyrrolidine-(2RS,4SR,5RS) sous forme d'une huile utilisée telle quelle dans les synthèses ultérieures et 1 g de tert-butoxycarbonyl-2 (fluoro-2 phényl)-5 morpholinosulfonyl-4 pyrrolidine-(2RS,4RS,5RS) fondant à 130°C.

La vinylsulfonyl-1 morpholine peut être préparée selon la méthode décrite par J. CHANET-REY et coll. Heterocycles, 26, 101 (1987).

### Exemple 18

A On opère comme décrit à l'exemple 2A, mais à partir de 3 g de {[(benzyloxycarbonyl-3 phényl)-3 uréido]-2 acétyl}-1 (fluoro-2 phényl)-5 morpholinosulfonyl-4 (diméthyl-3,3 butyl)carbamoyl-2 pyrrolidine-(2RS,4SR,5RS), de 0,3 g de palladium à 10% sur charbon et de 100 cm³ d'éthanol sous atmosphère d'hydrogène (130 kPa). Après traitement, on obtient 2,2 g d'acide ({[(diméthyl-3,3 butyl)carbamoyl-2 (fluoro-2 phényl)-5 morpholinosulfonyl-4 pyrrolidinyl-1]-2 oxo-2 éthyl}-3 uréido)-3 benzoïque-(2RS,4SR,5RS) [Rf = 0,25 ; éluant : chlorure de méthylène-méthanol (90/10)].

B La {[(benzyloxycarbonyl-3 phényl)-3 uréido]-2 acétyl}-1 (fluoro-2 phényl)-5 morpholinosulfonyl-4 (diméthyl-3,3 butyl)carbamoyl-2 pyrrolidine-(2RS,4SR,5RS) peut être préparée comme décrit à l'exemple 1B, mais à partir de 2,4 g de (fluoro-2 phényl)-5 morpholinosulfonyl-4 (diméthyl-3,3 butyl)carbamoyl-2 pyrrolidine-(2RS,4SR,5RS), de 1,78 g d'acide [(benzyloxycarbonyl-3 phényl)-3 uréido]-2 acétique et de 1,15 g de N,N'-dicyclohexylcarbodiimide dans 100 cm³ d'acétonitrile. Après traitement, on obtient 3 g de {[(benzyloxycarbonyl-3 phényl)-3 uréido]-2 acétyl}-1 (fluoro-2 phényl)-5 morpholinosulfonyl-4 (diméthyl-3,3 butyl)carbamoyl-2 pyrrolidine-(2RS,4SR,5RS) sous forme d'une meringue utilisée telle quelle dans les synthèses ultérieures.

C La (fluoro-2 phényl)-5 morpholinosulfonyl-4 (diméthyl-3,3 butyl)carbamoyl-2 pyrrolidine-(2RS,4SR,5RS) peut être préparée de la manière suivante : à une solution de 3 g de tert-butoxycarbonyl-1 (fluoro-2 phényl)-5 morpholinosulfonyl-4 (diméthyl-3,3 butyl)carbamoyl-2 pyrrolidine-(2RS,4SR,5RS) dans 100 cm³ de chloroforme, on ajoute 0,79 cm³ d'iodotriméthylsilane. Le mélange réactionnel est agité pendant vingt heures à une température voisine de 20°C, puis versé sur 100 cm³ d'une solution saturée en bicarbonate de sodium. La phase organique est séparée par décantation, lavée par 75 cm³ d'eau, séchée sur sulfate de magnésium et concentrée sous pression réduite. On obtient ainsi 2,4 g de (fluoro-2 phényl)-5 morpholinosulfonyl-4 (diméthyl-3,3 butyl)carbamoyl-2 pyrrolidine-(2RS,4SR,5RS) sous forme d'une huile utilisée telle quelle dans les synthèses ultérieures.

D La tert-butoxycarbonyl-1 (fluoro-2 phényl)-5 morpholinosulfonyl-4 (diméthyl-3,3 butyl)carbamoyl-2 pyrrolidine-(2RS,4SR,5RS) peut être préparée de la manière suivante : à une solution de 2,6 g d'acide tert-butoxycarbonyl-1 (fluoro-2 phényl)-5 morpholinosulfonyl-4 pyrrolidinecarboxylique-2-(2RS,4SR,5RS) dans 50 cm³ de dichloro-1,2 éthane, on ajoute successivement 1,01 g de N,N'-diimidazole carbonyle et 0,05 g de diméthylamino-4 pyridine. Le mélange réactionnel est agité pendant 2 heures à une température voisine de 20°C, puis on ajoute goutte à goutte une solution de 0,77 cm³ diméthyl-3,3 butylamine dans 25 cm³ de dichloro-1,2 éthane. Le mélange réactionnel est encore agité pendant 20 heures à une température voisine de 20°C, puis lavé successivement par 75 cm³ d'eau, deux fois 75 cm³ d'une solution aqueuse décinormale d'acide chlorhydrique, 25 cm³ d'eau, deux fois 75 cm³ d'une solution aqueuse décinormale d'hydroxyde de sodium, 75 cm³ d'une solution aqueuse saturée en chlorure de sodium. La phase organique est séchée sur sulfate de magnésium et concentrée sous pression réduite. Le résidu est repris par 50 cm³ de pentane et filtré. On obtient ainsi 3 g de tert-butoxycarbonyl-1 (fluoro-2 phényl)-5 morpholinosulfonyl-4 (diméthyl-3,3 butyl)carbamoyl-2 pyrrolidine-(2RS,4SR,5RS) sous forme d'une meringue utilisée telle quelle dans les synthèses ultérieures.

E L'acide tert-butoxycarbonyl-1 (fluoro-2 phényl)-5 morpholinosulfonyl-4 pyrrolidinecarboxylique-2-(2RS,4SR,5RS) peut être préparé de la manière suivante : à une solution de 2,6 g du chlorhydrate d'acide (fluoro-2 phényl)-5 morpholinosulfonyl-4 pyrrolidinecarboxylique-2-(2RS,4SR,5RS) et de 1,4 g de carbonate de sodium dans 60 cm³ d'eau distillée, on ajoute goutte à goutte une solution de 1,6 g de dicarbonate de di-tert-butyle dans 30 cm³ de dioxanne-1,4. Le milieu réactionnel est agité pendant 20 heures à une température voisine de 20°C, puis on ajoute 50 cm³ d'eau distillée. La phase aqueuse est lavée par deux fois 50 cm³ d'acétate d'éthyle puis amenée à un pH voisin de 1 par addition d'une solution aqueuse tétranormale d'acide chlorhydrique, extraite par trois fois 75 cm3 d'acétate d'éthyle. Les extraits organiques sont réunis et lavés par 50 cm3 d'une solution aqueuse saturée en chlorure de sodium, séchés sur sulfate de magnésium et concentrés sous pression réduite. On obtient ainsi 2,6 g d'acide tert-butoxycarbonyl-1 (fluoro-2 phényl)-5 morpholinosulfonyl-4 pyrrolidinecarboxylique-2-(2RS,4SR,5RS) sous forme d'une meringue utilisée telle quelle dans les synthèses ultérieures.

F Le chlorhydrate d'acide (fluoro-2 phényl)-5 morpholinosulfonyl-4 pyrrolidinecarboxylique-2-(2RS,4SR,5RS) peut être préparé de la manière suivante : à une solution de 2,7 g de (fluoro-2 phényl)-5 méthoxycarbonyl-2 morpholinosulfonyl-4 pyrrolidine-(2RS,4SR,5RS) dans un mélange de 6 0cm³ de méthanol et de 30 cm³ d'eau distillée, on ajoute 0,4 g d'hydroxyde de potassium. Le milieu réactionnel est agité pendant 8 heures à une température voisine de 20°C, puis concentré sous pression réduite. Le résidu est dissous dans 150 cm³ d'eau distillée et la phase aqueuse lavée par deux fois 50 cm3 d'oxyde de diéthyle, amenée à un pH voisin de 1 par addition d'une solution aqueuse tetranormale d'acide chlorhydrique et concentrée sous pression réduite. Le résidu est repris par 75 cm³ d'un mélange dichlorométhane-méthanol (80/20 en volumes). Le produit insoluble est séparé par filtration et extrait par deux fois 50 cm³ d'un mélange dichlorométhane-méthanol (80/20 en volumes). Le filtrat est concentré sous pression réduite. On obtient ainsi 2,6 g de chlorhydrate d'acide (fluoro-2 phényl)-5 morpholinosulfonyl-4 pyrrolidinecarboxylique-2-(2RS,4SR,5RS) sous forme d'une huile utilisée telle quelle dans les synthèses ultérieures.

G La (fluoro-2 phényl)-5 méthoxycarbonyl-2 morpholinosulfonyl-4 pyrrolidine-(2RS,4SR,5RS) peut être préparée comme décrit à l'exemple 1C, mais à partir de 7,9 g de (fluoro-2 benzylidèneamino)acétate de méthyle, de 7,5 g de vinylsulfonyl-1 morpholine, de 10 g d'acétate d'argent et de 6,8 cm3 de triéthylamine dans 300 cm³ d'acétonitrile. Après traitement, on obtient 2,7 g de (fluoro-2 phényl)-5 méthoxycarbonyl-2 morpholinosulfonyl-4 pyrrolidine-(2RS,4SR,5RS) sous forme d'une huile utilisée telle quelle dans les synthèses ultérieures et 1,7 g de (fluoro-2 phényl)-5 méthoxycarbonyl-2 morpholinosulfonyl-4 pyrrolidine-(2RS,4RS,5RS) fondant à 156°C.

H Le (fluoro-2 benzylidèneamino)acétate de méthyle peut être préparé comme décrit à l'exemple 1D, mais à partir de 4,25 cm³ de fluoro-2 benzaldéhyde, de 5 12 g de chlorhydrate de glycinate de méthyle, de 12 g de tamis 4Å et de 5,6 cm³ de triéthylamine dans 100 cm³ de dichlorométhane. Après traitement, on obtient 7,9 g de(fluoro-2 benzylidèneamino)acétate de méthyle sous forme d'une huile utilisée telle quelle dans les synthèses ultérieures.

Les médicaments selon l'invention sont constitués par un composé de formule (I) sous forme libre ou sous forme d'un sel pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, des troubles de la mémoire, dans le sevrage aux traitements chroniques et abus d'alcool ou de médicaments, comme constricteurs de la pupille de l'oeil, comme analgésiques, comme potentialisateurs de l'activité analgésique des médicaments analgésiques narcotiques et non narcotiques et comme régulateurs de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm³

## Revendications

1. Composés de formule : 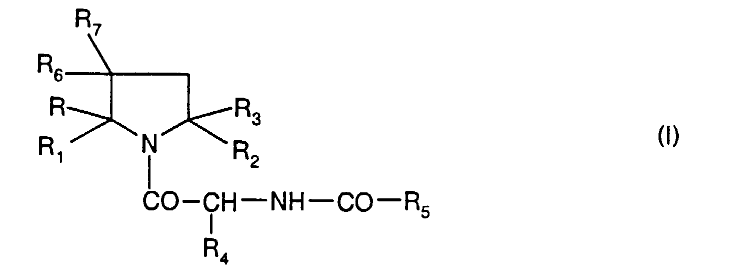 dans laquelle
R représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₈R_{9,} -NH-CO-CH₃, trifluorométhyle ou trifluorométhoxy,
R₁ représente un atome d'hydrogène ou un radical alkyle,
R₂ représente une chaîne -(CH₂)ₙ-CO-R₁₀, -(CH₂)ₘ-O-CO-R₁₁, -(CH₂)ₘ-NR₁₂R₁₃ ou un radical oxazolinyle éventuellement substitué par un ou plusieurs radicaux alkyle ou alkyl-3 oxadiazolyle,
R₃ représente un atome d'hydrogène ou un radical alkyle,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 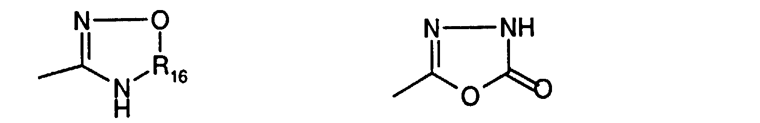 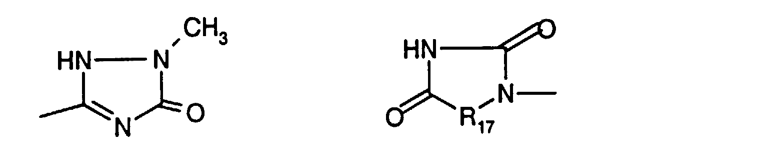 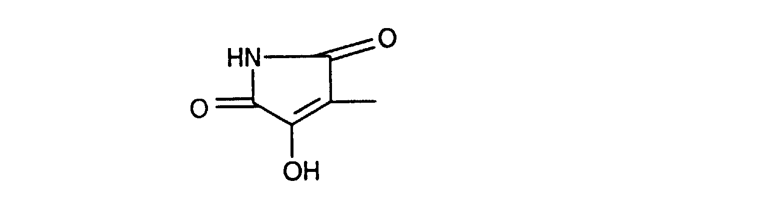 ou diméthyl-2,2 dioxo-4,6 dioxanne-1,3-yl-5,
R₆ représente un atome d'hydrogène ou un radical alkyle ou phénylalkyle,
R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy,
R₈ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₉ représente un radical alkyle, phénylalkyle ou phényle éventuellement substitué par un ou plusieurs substituants choisis parmi les atomes d'halogène et les radicaux alkyle, alcoxy et alkylthio,
ou bien R₈ et R₉ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₁₀ représente un radical hydroxy, alcoxy, cycloalkyloxy, cycloalkylalkyloxy, phényle ou -NR₁₂R₁₃,
R₁₁ représente un radical alcoxy, cycloalkyloxy, cycloalkylalkyloxy, phényle ou -NR₁₂R₁₃,
R₁₂ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₃ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₁₂ et R₁₃ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₄ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₅ représente un radical tétrazolyl-5,
R₁₆ représente C=O ou S=O,
R₁₇ représente O ou C=O,
n est égal à 0, 1 ou 2,
m est égal à 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène,
étant entendu que, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone,
les racémiques et les énantiomères de ces composés et leurs sels.

2. Composés de formule (I) selon la revendication 1 pour lesquels R représente un radical isopropylidène, cyclohexyle, tétrahydrophényle, cyclopentadiène, dihydrophényle, norbomyle, adamantyle ou norbomènyle, les racémiques et les énantiomères de ces composés et leurs sels.

3. Composés de formule (I) selon la revendication 1 pour lesquels lorsque R₈ et R₉ forment avec l'atome d'azote auquel ils sont rattachés un hétérocycle celui-ci est un cycle pipéridino éventuellement substitué par un ou plusieurs radicaux alkyle, morpholino ou tétrahydro-1,2,3,4 quinoléine, les racémiques et les énantiomères de ces composés et leurs sels.

4. Composés de formule (I) selon la revendication 1 pour lesquels lorsque R₁₂ et R₁₃ forment avec l'atome d'azote auquel ils sont rattachés un hétérocycle celui-ci est un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle, les racémiques et les énantiomères de ces composés et leurs sels.

5. Composés de formule (I) selon la revendication 1 pour lesquels
R représente phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₈R_{9,} -NH-CO-CH₃, trifluorométhyle ou trifluorométhoxy,
R₁ représente un atome d'hydrogène,
R₂ représente une chaîne -(CH₂)ₙ-CO-R₁₀,
R₃ représente un atome d'hydrogène,
R₄ représente un atome d'hydrogène,
R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 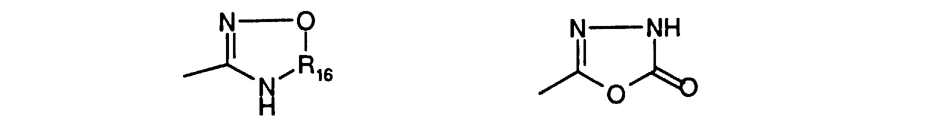 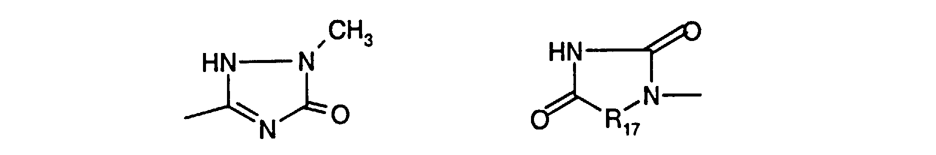 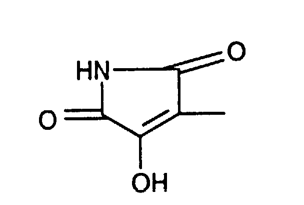 ou diméthyl-2,2 dioxo-4,6 dioxanne-1,3-yl-5,
R₆ représente un atome d'hydrogène,
R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy,
R₈ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₉ représente un radical alkyle, phénylalkyle ou phényle éventuellement substitué par un ou plusieurs substituants choisis parmi les atomes d'halogène et les radicaux alkyle, alcoxy et alkylthio,
ou bien R₈ et R₉ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₁₀ représente un radical hydroxy ou alcoxy,
R₁₄ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₅ représente un radical tétrazolyl-5,
R₁₆ représente C=O ou S=O,
R₁₇ représente O ou C=O,
n est égal à 0, 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène
les racémiques et les énantiomères de ces composés ainsi que leurs sels.

6. Composés choisis parmi
acide ({[tert-butoxycarbonyl-2 (fluoro-2 phényl)-5 (chloro-4 phényl)sulfonyl-4 pyrrolidinyl-1]-2 oxo-2 éthyl}-3 uréido)-3 phénylacétique-(2RS,4SR,5RS),
acide ({[tert-butoxycarbonyl-2 (fluoro-2 phényl)-5 (fluoro-2 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (méthoxy-3 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (méthyl-4 phényl)sulfonyl-4 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 (nitro-4 phényl)sulfonyl-4 pyrrolidinyl-1]-2 oxo-2 éthyl}-3 uréido)-3 benzoïque-(2RS,4SR,5RS),
acide ({[(amino-4 phényl)sulfonyl-4 tert-butoxycarbonyl-2 (fluoro-2 phényl)-5 pyrrolidinyl-1]-2 oxo-2 éthyl}-3 uréido)-3 benzoïque-(2RS,4SR,5RS),
acide ({[(acétamido-4 phényl)sulfonyl-4 tert-butoxycarbonyl-2 (fluoro-2 phényl)-5 pyrrolidinyl-1]-2 oxo-2 éthyl}-3 uréido)-3 benzoïque-(2RS,4SR,5RS),
acide ({[tert-butoxycarbonyl-2 (diméthylamino-4 phényl)sulfonyl-4 (fluoro-2 phényl)-5 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 méthylsulfonyl-4 pyrrolidinyl-1]-2 oxo-2 éthyl}-3 uréido)-3 benzoique-(2RS,4SR,SRS),
acide ({[(chloro-4 phényl)sulfonyl-4 (fluoro-2 phényl)-5 isobutylcarbamoyl-2 pyrrolidinyl-1]-2 oxo-2 éthyl}-3 uréido)-3 phénylacétique-(2RS,4SR,5RS),
acide ({[(fluoro-2 phényl)-5 (fluoro-2 phényl)sulfonyl-4 isobutylcarbamoyl-2 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (méthoxy-3 phényl)sulfonyl-4 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (méthyl-4 phényl)sulfonyl-4 pyrrolidinyl-1]-2 oxo-2 éthyl}-3 uréido)-3 benzoïque-(2RS,4SR,5RS),
acide ({[(fluoro-2 phényl)-5 isobutylcarbamoyl-2 (nitro-4 phényl)sulfonyl-4 pyrrolidinyl-1]-2 oxo-2 éthyl}-3 uréido)-3 benzoïque-(2RS,4SR,5RS),
acide ({[(amino-4 phényl)sulfonyl-4 (fluoro-2 phényl)-5 isobutylcarbamoyl-2 pyrrolidinyl-1]-2 oxo-2 éthyl}-3 uréido)-3 phénylacétique-(2RS,4SR,5RS),
acide ({[(acétamido-4 phényl)sulfonyl-4 (fluoro-2 phényl)-5 isobutylcarbamoyl-2 pyrrolidinyl-1]-2 oxo-2 éthyl}-3 uréido)-3 benzoïque-(2RS,4SR,5RS),
acide ({[tert-butoxycarbonyl-2 (fluoro-2 phényl)-5 morpholinosulfonyl-4 pyrrolidinyl-1]-2 oxo-2 éthyl}-3 uréido)-3 benzoïque-(2RS,4SR,5RS),
ainsi que leurs sels.

7. Procédé de préparation des composés de formule (I) selon la revendication 1 pour lesquels R₅ représente un radical phénylamino dont le noyau phényle est éventuellement substitué caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 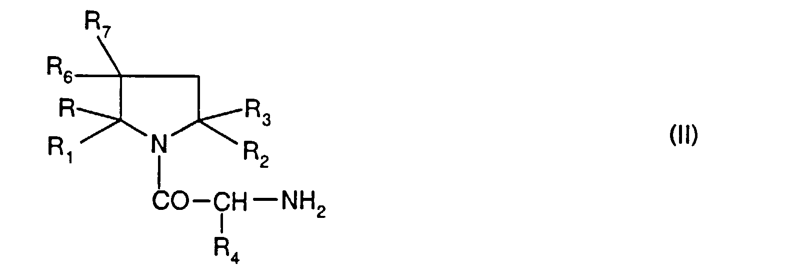 dans laquelle R, R₁, R₂, R₃, R₄, R₆ et R₇ ont les mêmes significations que dans la revendication 1, sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 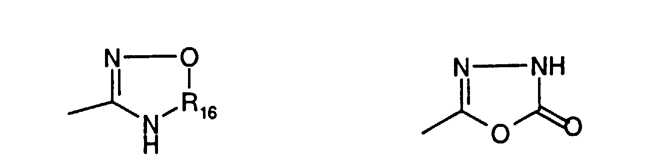 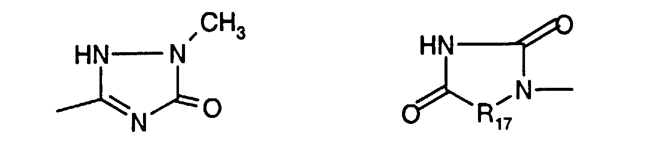 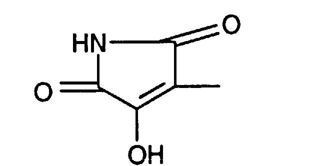 ou diméthyl-2,2 dioxo-4,6 dioxanne-1,3-yl-5, alk, alk', X, R₁₄, R₁₅, R₁₆ et R₁₇ ayant les mêmes significations que dans la revendication 1, isole le produit et le transforme éventuellement en sel.

8. Procédé de préparation des composés de formule (I) selon la revendication 1 pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle caractérisé en ce que l'on fait réagir un dérivé de formule : 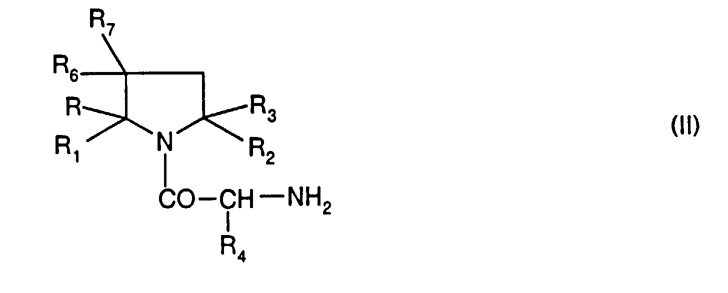 dans laquelle R, R₁, R₂, R₃, R₄, R₆ et R₇ ont les mêmes significations que dans la revendication 1, sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est un radical alkyle ou phénylalkyle et alk et alk' ont les mêmes significations que dans la formule (I), isole le produit et le transforme éventuellement en sel.

9. Procédé de préparation des composés de formule (I) selon la revendication 1 pour lesquels R₅ représente un radical phényle éventuellement substitué, naphtyle, indolyle, quinolyle caractérisé en ce que l'on fait réagir un dérivé de formule : 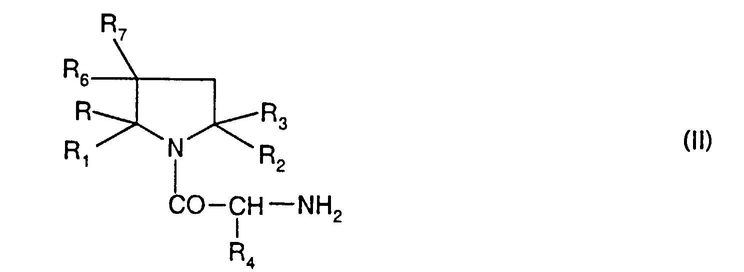 dans laquelle R, R₁, R₂, R₃, R₄, R₆ et R₇ ont les mêmes significations que dans la revendication 1, sur un acide de formule HOOC-R₅ dans laquelle R₅ a les mêmes significations que précédemment ou un dérivé réactif de cet acide, isole le produit et le transforme éventuellement en sel.

10. Procédé de préparation des composés de formule (I) pour lesquels R₅ représente un radical phénylamino dont le noyau phényle est substitué par un radical carboxy, -alk-COOX, -O-alk-COOX, -alk'-COOX, -CH=CH-COOX, -CO-COOX, -S-alk-COOX, -SO-alk-COOX, -SO₂-alk-COOX, -C(=NOH)-COOX, -O-CH₂-alk'-COOX, -CX=N-O-alk-COOX et X représente un atome d'hydrogène caractérisé en ce que l'on hydrolyse ou hydrogénolyse un composé de formule (I) correspondant pour lequel X est un radical alkyle ou phénylalkyle, isole le produit et le transforme éventuellement en sel.

11. Procédé de préparation des composés de formule (I) selon la revendication 1 pour lesquels R₅ représente un radical phénylamino dont le noyau phényle est substitué par un radical hydroxyiminoalkyle ou alcoxyiminoalkyle caractérisé en ce que l'on fait réagir un composé de formule (I) correspondant pour lequel R₅ représente un radical phénylamino dont le noyau phényle est substitué par un radical acyle, sur un dérivé de formule :
H₂N-OR₂₀ (IX)
dans laquelle R₂₀ représente un atome d'hydrogène ou un radical alkyle, isole le produit et le transforme éventuellement en sel.

12. Procédé de préparation des composés de formule (I) selon la revendication 1 pour lesquels R₂ représente une chaîne -(CH₂)ₙ-CO-R₁₀, R₁₀ représente un radical alcoxy, cycloalkyloxy, cycloalkylalkyloxy, phényle ou -NR₁₂R₁₃,_{,} R₇ représente un radical alkylsulfonyle, -SO₂-NR₈R₉ ou phénylsulfonyle dont le noyau phényle est substitué par un ou plusieurs substituants choisis parmi les atomes d'halogène et les radicaux alkyle, alcoxy, hydroxy, nitro, amino, monoalkylamino, dialkylamino, acylamino, trifluorométhyle, trifluorométhoxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X représente un radical alkyle ou phénylalkyle caractérisé en ce que l'on fait réagir un dérivé de formule : 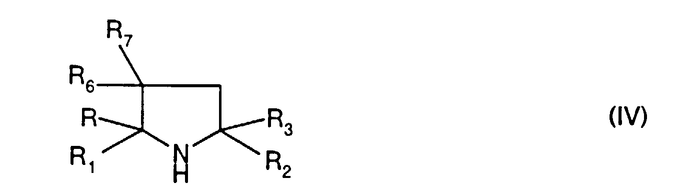 dans laquelle R₂ et R₇ ont les mêmes significations que précédemment et R, R₁, R₃ et R₆ ont les mêmes significations que dans la revendication 1, sur un acide de formule : 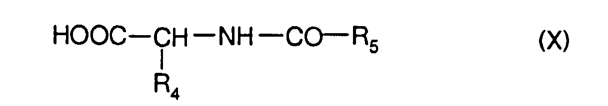 dans laquelle R₅ a les mêmes significations que ci-dessus ou un dérivé réactif de cet acide et R₄ a les mêmes significations que dans la revendication 1, isole le produit et le transforme éventuellement en sel.

13. Procédé de préparation des composés de formule (I) selon la revendication 1 pour lesquels R₇ représente un radical phénylsulfonyle dont le phényle est substitué par un radical amino caractérisé en ce que l'on réduit un composé de formule (I) correspondant pour lequel R₇ représente un radical phénylsulfonyle dont le phényle est substitué par un radical nitro, isole le produit et le transforme éventuellement en sel.

14. Procédé de préparation des composés de formule (I) selon la revendication 1 pour lesquels R₇ représente un radical phénylsulfonyle dont le phényle est substitué par un radical monométhylamino caractérisé en ce que l'on méthyle un composé de formule (I) correspondant pour lesuel R₇ représente un radical phénylsulfonyle dont le phényle est substitué par un radical amino, isole le produit et le transforme éventuellement en sel.

15. Médicaments contenant en tant que principe actif au moins un composé de formule (I) selon l'une des revendications 1 à 6.

16. Médicaments selon la revendication 15 utilisables pour le traitement ou la prévention des désordres liés à la CCK et à la gastrine.

## Patentansprüche

1. Verbindungen der Formel: 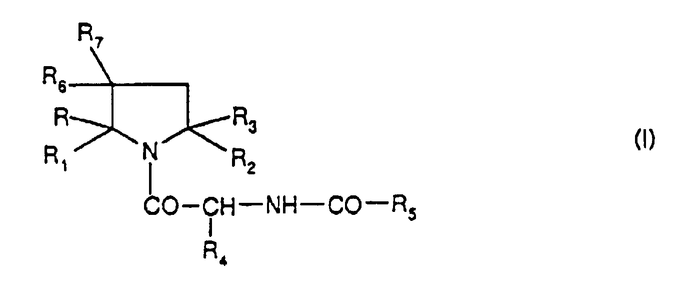 worin
R einen Alkylrest mit 1 bis 12 Kohlenstoffatomen in gerader oder verzweigter Kette und gegebenenfalls einfach oder mehrfach ungesättigt, einen Cycloalkylrest mit 3 bis 12 Kohlenstoffatomen und gegebenenfalls einfach oder mehrfach ungesättigt, einen Polycycloalkylrest mit 6 bis 12 Kohlenstoffatomen und gegebenenfalls einfach oder mehrfach ungesättigt, einen Phenylalkylrest, dessen Phenylkern gegebenenfalls substituiert ist (durch einen oder mehrere Substituenten, ausgewählt aus Alkyl-, Alkoxyresten oder Halogenatomen), einen Diphenylalkylrest, einen Cinnamylrest, einen Pyridylrest, der gegebenenfalls durch einen oder mehrere Alkylrest(e) substituiert ist, einen Furylrest, der gegebenenfalls durch einen oder mehrere Alkylrest(e) substituiert ist, einen Thienylrest, der gegebenenfalls durch einen oder mehrere Alkylrest(e) substituiert ist, einen Chinolylrest, der gegebenenfalls durch einen oder mehrere Alkylrest(e) substituiert ist, einen Naphthylrest, der gegebenenfalls durch einen oder mehrere Alkylrest(e) substituiert ist, einen Indolylrest, der gegebenenfalls durch ein oder mehrere Alkylreste substituiert ist, oder einen Phenylrest, der gegebenenfalls durch einen oder mehrere Substituenten, ausgewählt aus Halogenatomen, Alkyl-, Alkoxy-, Hydroxy-, Nitro-, Amino-, Monoalkylamino-, Dialkylamino-, Alkoxycarbonyl-, -CO-NR₈R₉, -NH-CO-CH₃, Trifluormethyl- oder Trifluormethoxyresten, substituiert ist, bedeutet,
R₁ ein Wasserstoffatom oder einen Alkylrest bedeutet,
R₂ eine Kette -(CH₂)ₙ-CO-R₁₀, -(CH₂)ₘ-O-CO-R₁₁, -(CH₂)ₘ-NR₁₂R₁₃ oder einen Oxazolinylrest, der gegebenenfalls durch ein oder mehrere Alkylrest(e) substituiert ist, oder einen 3-Alkyloxadiazolylrest bedeutet,
R₃ ein Wasserstoffatom oder einen Alkylrest bedeutet,
R₄ ein Wasserstoffatom oder einen Alkylrest bedeutet,
R₅ einen Phenylrest (gegebenenfalls substituiert durch einen oder mehrere Substituenten, ausgewählt aus Halogenatomen und Alkyl-, Alkoxy und Alkylthioresten), einen Naphthylrest, einen Indolylrest, einen Chinolylrest oder einen Phenylaminorest bedeutet, dessen Phenylkern gegebenenfalls durch einen oder mehrere Substituenten, ausgewählt aus Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H (in Form eines Salzes), -CH=CH-alk', -C(=NOH)-COOX, -S-alk-COOX, -SO-alk-COOX, -SO₂-alk-COOX, -O-CH₂-alk'-COOx, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 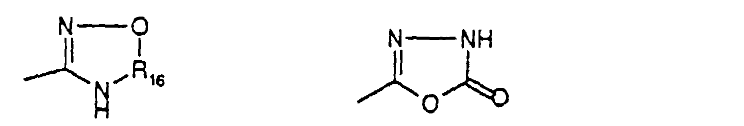 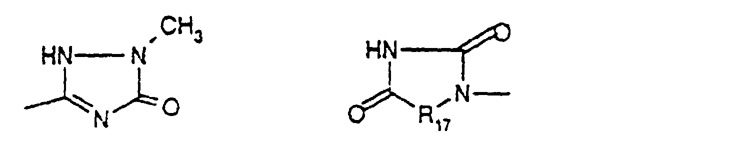 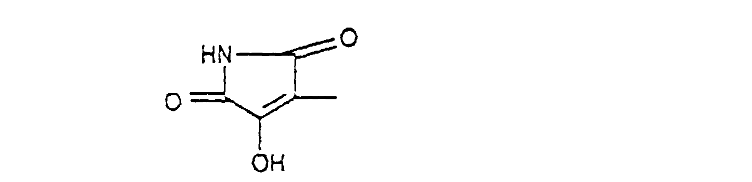 oder 2,2-Dimethyl-4,6-dioxo-1,3-dioxan-5-yl, substituiert ist,
R₆ ein Wasserstoffatom oder einen Alkyl- oder Phenylalkylrest bedeutet,
R₇ einen Alkylsulfonylrest, -SO₂-NR₈R₉- oder Phenylsulfonylrest bedeutet, dessen Phenylkern durch einen oder mehrere Substituenten, ausgewählt aus Halogenatomen, und den Resten Alkyl, Alkoxy, Hydroxy, Nitro, Amino, Monoalkylamino, Dialkylamino, Acylamino, Trifluormethyl, Trifluormethoxy, substituiert ist,
R₈ ein Wasserstoffatom oder ein Alkyl-, Phenylalkyl- oder Phenylrest, der gegebenenfalls durch einen oder mehrere Substituenten, ausgewählt aus Halogenatomen und den Resten Alkyl, Alkoxy und Alkylthio, substituiert ist, bedeutet,
R₉ einen Alkyl-, Phenylalkyl- oder Phenylrest bedeutet, der gegebenenfalls durch einen oder mehrere Substituenten, ausgewählt aus Halogenatomen und den Resten Alkyl, Alkoxy und Alkylthio, substituiert ist,
oder R₈ und R₉ mit dem Stickstoffatom, an das sie gebunden sind, einen gesättigten oder ungesättigten mono- oder polycyclischen Heterozyklus mit 4 bis 9 Kohlenstoffatomen und einem oder mehreren Heteroatom(en) (O, N) und gegebenenfalls substituiert durch einen oder mehrere Alkylrest(e) bilden,
R₁₀ einen Hydroxy-, Alkoxy-, Cycloalkyloxy-, Cycloalkylalkyloxy-, Phenyl- oder -NR₁₂R₁₃-Rest bedeutet,
R₁₁ einen Alkoxy-, Cycloalkyloxy-, Cycloalkylalkyloxy-, Phenyl- oder -NR₁₂R₁₃-Rest bedeutet,
R₁₂ ein Wasserstoffatom oder einen Alkyl-, Cycloalkylalkyl-, Cycloalkyl-, Phenylalkylrest- oder Phenylrest bedeutet, der gegebenenfalls durch einen oder mehrere Substituenten, ausgewählt aus Halogenatomen und den Resten Alkyl, Alkoxy und Alkylthio, substituiert ist,
R₁₃ einen Alkyl-, Cycloalkylalkyl-, Cycloalkyl-, Phenylalkyl- oder Phenylrest bedeutet, der gegebenenfalls durch einen oder mehrere Substituenten, ausgewählt aus Halogenatomen und Alkyl-, Alkoxy- und Alkylthioresten, substituiert ist,
oder R₁₂ und R₁₃ zusammen mit dem Stickstoffatom, an das sie gebunden sind, einen gesättigten oder ungesättigten mono-oder polycyclischen Heterozyklus bilden, der 4 bis 9 Kohlenstoffatome und ein oder mehrere Heteroatom(e) (O, N, S) enthält und gegebenenfalls durch ein oder mehrere Alkylreste substituiert ist,
R₁₄ einen Alkyl-, Cycloalkyl-, Trifluormethyl-, Phenylrest bedeutet, der gegebenenfalls durch einen oder mehrere Substituenten, ausgewählt aus den Resten Cyano, Alkoxy, Nitro, Amino und Halogenatomen, substituiert ist,
R₁₅ einen 5-Tetrazolylrest bedeutet,
R₁₆ C=O oder S=O bedeutet,
R₁₇ O oder C=O bedeutet,
n 0, 1 oder 2 bedeutet,
m 1 oder 2 bedeutet,
X ein Wasserstoffatom, einen Alkyl- oder Phenylalkylrest bedeutet,
alk einen Alkyl- oder einen Alkylenrest bedeutet,
alk' einen Hydroxyalkyl-, Hydroxyalkylen-, Alkoxyalkyl- oder Alkoxyalkylenrest bedeutet,
mit der Maßgabe, daß, außer anders angegeben, die Alkyl-, Alkylen- und Alkoxyreste und die Alkyl-, Alkylen- und Alkoxyanteile 1 bis 4 Kohlenstoffatome in gerader oder verzweigter Kette enthalten, die Acylreste oder -anteile 2 bis 4 Kohlenstoffatome enthalten, und die Cycloalkylreste und-anteile 3 bis 6 Kohlenstoffatome enthalten,
sowie die Racemate und Enantiomeren dieser Verbindungen und ihre Salze.

2. Verbindungen der Formel (I) nach Anspruch 1, in denen R einen Isopropyliden-, Cyclohexyl-, Tetrahydrophenyl-, Cyclopentadien-, Dihydrophenyl-, Norbornyl-, Adamantyl- oder Norbornenylrest bedeutet, die Racemate und Enantiomeren dieser Verbindungen und ihre Salze.

3. Verbindungen der Formel (I) nach Anspruch 1, in denen, wenn R₈ und R₉ mit dem Stickstoffatom, an das sie gebunden sind, einen Heterozyklus bilden, dieser ein gegebenenfalls durch einen oder mehrere Alkyl-, Morpholino- oder 1,2,3,4-Tetrahydrochinoleinrest(e) substituierter Piperidinring ist, die Racemate und Enantiomeren dieser Verbindungen und ihre Salze.

4. Verbindungen der Formel (I) nach Anspruch 1, in denen, wenn R₁₂ und R₁₃ mit dem Stickstoffatom, an das sie gebunden sind, einen Heterozyklus bilden, dieser ein Piperidin-, 1-Perhydroazepinyl-1,2,3,6-tetrahydro-1-pyridyl-, 1,2,3-4-Tetrahydro-1-chinolyl-, 1-Pyrrolidinyl-, 1,2,3,4-Tetrahydro-2-isochinolyl-, Thiomorpholino- oder 1-Indolinylring ist, wobei diese Ringe gegebenenfalls durch mindestens einen oder mehrere Alkylrest(e) substituiert sein können, die Racemate und Enantiomeren dieser Verbindungen und ihrer Salze.

5. Verbindungen der Formel (I) nach Anspruch 1, in denen
R einen gegebenenfalls durch einen oder mehrere Substituenten, ausgewählt aus Halogenatomen, Alkyl-, Alkoxy-, Hydroxy-, Nitro-, Amino-, Monoalkylamino-, Dialkylamino-, Alkoxycarbonyl-, -CO-NR₈R₉-, -NH-CO-CH₃-, Trifluormethyl-oder Trifluormethoxyreste substituierten Phenylrest bedeutet,
R₁ ein Wasserstoffatom bedeutet,
R₂ eine Kette -(CH₂)ₙ-CO-R₁₀ bedeutet,
R₃ ein Wasserstoffatom bedeutet,
R₄ ein Wasserstoffatom bedeutet,
R₅ einen Phenylaminorest bedeutet, dessen Phenylring gegebenenfalls durch einen oder mehrere Substituenten, ausgewählt aus Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H (in Form eines Salzes), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 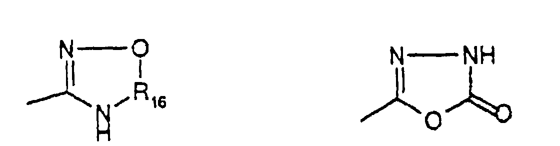 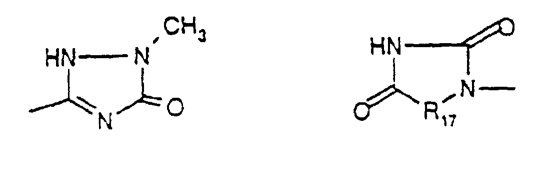 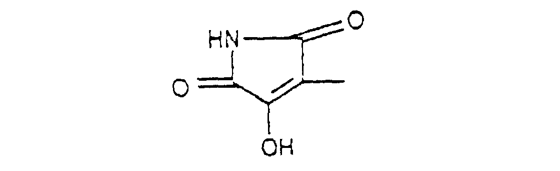 oder 2,2-Dimethyl-4,6-dioxo-1,3-dioxan-5-yl, substituiert ist,
R₆ ein Wasserstoffatom bedeutet,
R₇ einen Alkylsulfonyl-, -SO₂-NR₈R₉- oder Phenylsulfonylrest bedeutet, dessen Phenylkern durch einen oder mehrere Substituenten, ausgewählt aus Halogenatomen und den Resten Alkyl, Alkoxy, Hydroxy, Nitro, Amino, Monoalkylamino, Dialkylamino, Acylamino, Trifluormethyl, Trifluormethoxy, substituiert ist,
R₈ ein Wasserstoffatom oder einen Alkyl-, Phenylalkyl- oder Phenylrest, der gegebenenfalls durch einen oder mehrere Substituenten, ausgewählt aus Halogenatomen und Alkyl-, Alkoxy- oder Thioresten, substituiert ist, bedeutet,
R₉ einen Alkyl-, Phenylalkyl- oder Phenylrest, der gegebenenfalls durch einen oder mehrere Substituenten, ausgewählt aus Halogenatomen und Alkyl-, Alkoxy- oder Alkylthioresten, substituiert ist, bedeutet,
oder R₈ und R₉ mit dem Stickstoffatom, an das sie gebunden sind, einen gesättigten oder ungesättigten mono- oder polycyclischen Heterozyklus, der 4 bis 9 Kohlenstoffatome und 1 oder mehrere Heteroatome (O, N) enthält und gegebenenfalls durch ein oder mehrere Alkylreste substituiert ist, bilden,
R₁₀ einen Hydroxy- oder Alkoxyrest bedeutet,
R₁₄ einen Alkyl-, Cycloalkyl-, Trifluormethyl-, Phenylrest bedeutet, der gegebenenfalls durch einen oder mehrere Substituenten, ausgewählt aus Cyano-, Alkoxy-, Nitro-, Aminoresten und Halogenatomen substituiert ist,
R₁₅ einen 5-Tetrazolylrest bedeutet,
R₁₆ C=O oder S=O bedeutet,
R₁₇ O oder C=O bedeutet,
n 0, 1 oder 2 ist,
X ein Wasserstoffatom, einen Alkyl- oder Phenylalkylrest bedeutet,
alk einen Alkyl- oder Alkylenrest bedeutet,
alk' einen Hydroxyalkyl-, Hydroxyalkylen- Alkoxyalkyl- oder Alkoxyalkylenrest bedeutet,
die Racemate und Enantiomeren dieser Verbindungen sowie ihre Salze.

6. Verbindungen, ausgewählt aus
(2RS,4SR,5RS)-3-(3-{2-[2-tert.-Butoxycarbonyl-5-(2-fluorphenyl)-4-(4-chlorphenyl)sulfonyl-1-pyrrolidinyl]-2-oxo-ethyl}ureido)phenylessigsäure,
(2RS,4SR,5RS)-3-(3-{2-[2-tert.-Butoxycarbonyl-5-(2-fluorphenyl)-4-(4-fluorphenyl)sulfonyl-1-pyrrolidinyl]-2-oxo-ethyl}ureido)benzoesäure,
(2RS,4SR,5RS)-3-(3-{2-[2-tert.-Butoxycarbonyl-5-(2-fluorphenyl)-4-(3-methoxyphenyl)sulfonyl-1-pyrrolidinyl]-2-oxo-ethyl}ureido)benzoesäure,
(2RS,4SR,5RS)-3-(3-{2-(2-tert.-Butoxycarbonyl-5-(2-fluorphenyl)-4-(4-methylphenyl)sulfonyl-1-pyrrolidinyl]-2-oxo-ethyl}ureido)benzoesäure,
(2RS,4SR,5RS)-3-(3-{2-(2-tert.-Butoxycarbonyl-5-(2-fluorphenyl)-4-(4-nitrophenyl)sulfonyl-1-pyrrolidinyl]-2-oxo-ethyl}ureido)benzoesäure,
(2RS,4SR,5RS)-3-(3-{2-[4-(4-Aminophenyl)sulfonyl-2-tert.-butoxycarbonyl-5-(2-fluorphenyl)-1-pyrrolidinyl]-2-oxoethyl}ureido)benzoesäure,
(2RS,4SR,5RS)-3-(3-(2-[4-(4-Acetamidophenyl)sulfonyl-2-tert.-butoxycarbonyl-5-(2-fluorphenyl)-1-pyrrolidinyl]-2-oxoethyl}ureido)benzoesäure,
(2RS,4SR,5RS)-3-(3-{2-[2-tert.-Butoxycarbonyl-4-(4-dimethyl-aminophenyl)sulfonyl-5-(2-fluorphenyl)-1-pyrrolidinyl-2-oxo-ethyl}ureido)benzoesäure,
(2RS,4SR,5RS)-3-(3-{2-[2-tert.-Butoxycarbonyl-5-(2-fluor-phenyl)-4-methylsulfonyl-1-pyrrolidinyl]-2-oxoethyl}ureido)-benzoesäure,
(2RS,4SR,5RS)-3-(3-{2-[4-(4-chlorphenyl)sulfonyl-5-(2-fluor-phenyl)-2-isobutylcarbamoyl-1-pyrrolidinyl]-2-oxoethyl}-ureido)phenylessigsäure,
(2RS,4SR,5RS)-3-(3-{2-[5-(2-Fluorphenyl)-4-(2-fluorphenyl)-sulfonyl-2-isobutylcarbamoyl-1-pyrrolidinyl]-2-oxoethyl}-ureido)benzoesäure,
(2RS,4SR,5RS) -3-(3-{2-[5-(2-Fluorphenyl)-2-isobutylcarbamoyl-4-(3-methoxyphenyl)sulfonyl-1-pyrrolidinyl]-2-oxoethyl}ureido)benzoesäure,
(2RS,4SR,5RS)-3-(3-{2-[5-(2-Fluorphenyl)-2-isobutylcarbamoyl-4-(4-methylphenyl)sulfonyl-l-pyrrolidinyl]-2-oxoethyl}-ureido)benzoesäure,
(2RS,4SR,5RS)-3-(3-{2-[5-(2-Fluorphenyl)-2-isobutylcarbamoyl-4-(4-nitrophenyl)sulfonyl-1-pyrrolidinyl]-2-oxoethyl}-ureido)benzoesäure,
(2RS,4SR,5RS)-3-(3-{2-[4-(4-Aminophenyl)sulfonyl-5-(2-fluorphenyl)-2-isobutylcarbamoyl-1-pyrrolidinyl]-2-oxoethyl}-ureido)phenylessigsäure,
(2RS,4SR,5RS)-3-(3-(2-[4-(4-Acetamidophenyl)sulfonyl-5-(2-fluorphenyl)-2-isobutylcarbamoyl-1-pyrrolidinyl]-2-oxo-ethyl}ureido)benzoesäure,
(2RS,4SR,5RS)-3-(3-(2-[2-tert.-Butoxycarbonyl-5-(2-fluor-phenyl)-4-morpholinosulfonyl-1-pyrrolidinyl]-2-oxoethyl}-ureido)benzoesäure,
sowie ihre Salze.

7. Verfahren zur Herstellung der Verbindungen der Formel (I) nach Anspruch 1, in denen R₅ einen Phenylaminorest bedeutet, dessen Phenylkern gegebenenfalls substituiert ist, dadurch **gekennzeichnet,** daß man ein reaktives Derivat der Carbaminsäure, das gegebenenfalls in situ durch Wirkung eines reaktiven Derivats der Carbonsäure, ausgewählt aus N,N'-Diimidazolcarbonyl, Phosgen, Diphosgen, Triphosgen und p-Nitrophenylchlorformiat auf ein Derivat der Formel: 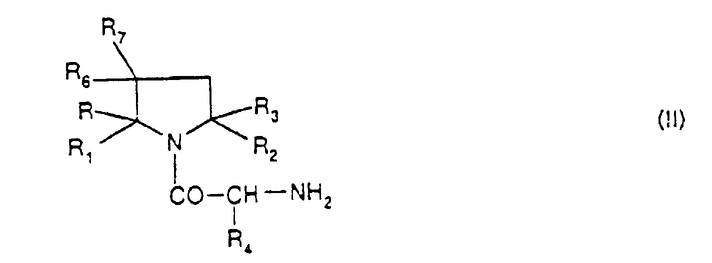 worin R, R₁, R₂, R₃, R₄, R₆ und R₇, wie in Anspruch 1 definiert sind, erhalten wurde, mit einem Anilin umsetzt, dessen Phenylkern gegebenenfalls durch einen oder mehrere Substituenten, ausgewählt aus Halogenatomen und Alkyl-, Alkoxy-, Alkylthio-, Trifluormethyl-, Carboxy-, Alkoxycarbonyl-, Hydroxy-, Nitro-, Amino-, Acyl-, Cyano-, Sulfamoyl-, Carbamoyl-, Hydroxyiminoalkyl-, Alkoxyiminoalkyl-, Hydroxyaminocarbonyl-, Alkoxyaminocarbonyl-, 5-Tetrazolyl-, 5-Tetrazolylalkyl-, Trifluormethylsulfonamido-, Alkylsulfinyl-, Mono- oder Polyhydroxyalkyl-, Sulfo-, -alk-O-CO-alk, -alk-COOX, -alk-O-alk, -alk'-COOX, -O-alk-COOX, -CH=CH-COOX, -CO-COOX, -alk-SO₃H (in Form eines Salzes), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂) =NOH, -SO₂-NH-R₁₅, -CO-NH-R_{15,} 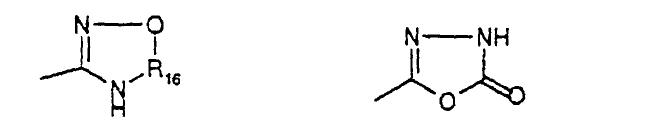 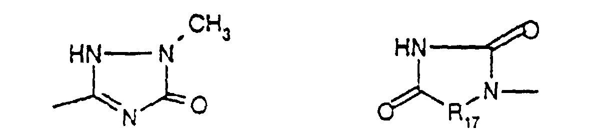 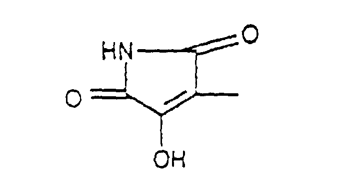 oder 2,2-Dimethyl-4,6-dioxo-1,3-dioxan-5-yl, substituiert ist, wobei alk, alk', X, R₁₄, R₁₅, R₁₆ und R₁₇ wie in Anspruch 1 definiert sind, das Produkt isoliert und gegebenenfalls in ein Salz umwandelt.

8. Verfahren zur Herstellung von Verbindungen der Formel (I) nach Anspruch 1, in denen R₅ einen Phenylaminorest bedeutet, dessen Phenylkern gegebenenfalls durch einen oder mehrere Substituenten, ausgewählt aus Halogenatomen und Alkyl-, Alkoxy-, Alkylthio-, Trifluormethyl-, Nitro-, Acyl-, Cyano-, Sulfamoyl-, Alkoxycarbonyl-, Carbamoyl-, Alkoxyiminoalkyl-, Alkoxyaminocarbonyl-, -alk-O-CO-alk, -CH=CH-alk', -alk-O-alk, Trifluormethylsulfonamido-, -alk-SO₃H (in Form eines Salzes), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X ein Alkyl- oder Phenylalkylrest ist, substituiert ist, dadurch **gekennzeichnet,**daß man eine Verbindung der Formel: 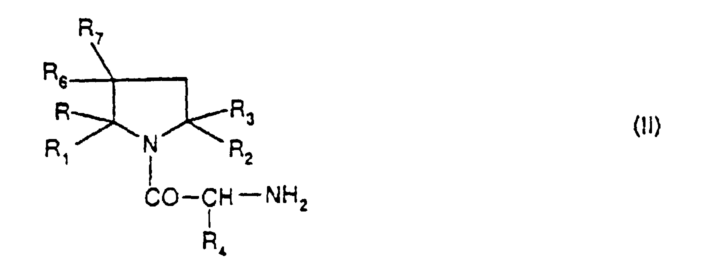 worin R, R₁, R₂, R₃, R₄, R₆ und R₇ wie in Anspruch 1 definiert sind, mit einem Phenylisocyanat umsetzt, dessen Phenylkern gegebenenfalls durch einen oder mehrere Substituenten, ausgewählt aus Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form eines Salzes), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X ein Alkyl- oder Phenylalkylrest ist und alk und alk' wie in Formel (I) definiert sind, substituiert ist, umsetzt, das Produkt isoliert, gegebenenfalls in ein Salz umwandelt.

9. Verfahren zur Herstellung von Verbindungen der Formel (I) nach Anspruch 1, in denen R₅ einen gegebenenfalls substituierten Phenylrest, Naphthylrest, Indolylrest, Chinolylrest bedeutet, dadurch **gekennzeichnet**, daß man eine Verbindung der Formel: 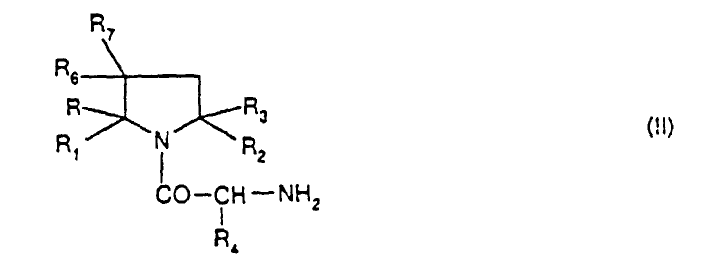 worin R, R₁, R₂, R₃, R₄, R₆ und R₇ wie in Anspruch 1 definiert sind, mit einer Säure der Formel HOOC-R₅ umsetzt, in der R₅ wie vorstehend definiert ist, oder mit einem reaktiven Derivat dieser Säure umsetzt, das Produkt isoliert und gegebenenfalls in ein Salz umwandelt.

10. Verfahren zur Herstellung von Verbindungen der Formel (I), in denen R₅ einen Phenylaminorest bedeutet, dessen Phenylkern durch einen Rest Carboxy, -alk-COOX, -O-alk-COOX, -alk'-COOX, -CH=CH-COOX, -CO-COOX, -S-alk-COOX, -SO-alk-COOX, -SO₂-alk-COOX, -C(=NOH)-COOX, -O-CH₂-alk'-COOX, -CX=N-O-alk-COOX substituiert ist, und X ein Wasserstoffatom bedeutet, dadurch **gekennzeichnet**, daß man eine entsprechende Verbindung der Formel (I), in der X einen Alkyl- oder Phenylalkylrest bedeutet, hydrolysiert oder hydrogenolysiert, das Produkt isoliert und gegebenenfalls in ein Salz überführt.

11. Verfahren zur Herstellung von Verbindungen der Formel (I) nach Anspruch 1, in denen R₅ einen Phenylaminorest bedeutet, dessen Phenylkern durch einen Hydroxyiminoalkyloder Alkoxyiminoalkylrest substituiert ist, dadurch **gekennzeichnet**, daß man eine entsprechende Verbindung der Formel (I), in der R₅ einen Phenylaminorest bedeutet, dessen Phenylkern durch einen Acylrest substituiert ist, mit einem Derivat der Formel
H₂N-OR₂₀ (IX)
umsetzt, worin R₂₀ ein Wasserstoffatom oder einen Alkylrest bedeutet, das Produkt isoliert und gegebenenfalls in ein Salz überführt.

12. Verfahren zur Herstellung von Verbindungen der Formel (I) nach Anspruch 1, in denen R₂ eine Kette -(CH₂)ₙ-CO-R₁₀ bedeutet, R₁₀ einen Alkoxy-, Cycloalkyloxy-, Cycloalkylalkyloxy-, Phenyl- oder -NR₁₂R₁₃-Rest bedeutet, R₇ einen Alkylsulfonyl-, -SO₂-NR₈R₉- oder Phenylsulfonylrest bedeutet, dessen Phenylkern durch einen oder mehrere Substituenten, ausgewählt aus Halogenatomen und den Resten Alkyl, Alkoxy, Hydroxy, Nitro, Amino, Monoalkylamino, Dialkylamino, Acylamino, Trifluormethyl, Trifluormethoxy, substituiert ist und R₅ einen gegebenenfalls substituierten Phenylrest, Naphthyl-, Indolyl-, Chinolyl- oder Phenylaminorest bedeutet, dessen Phenylkern gegebenenfalls durch einen oder mehrere Substituenten, ausgewählt aus Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido-, -alk-SO₃H (in Form eines Salzes), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X einen Alkyl- oder Phenylalkylrest bedeutet, substituiert ist, dadurch **gekennzeichnet**, daß man eine Verbindung der Formel: 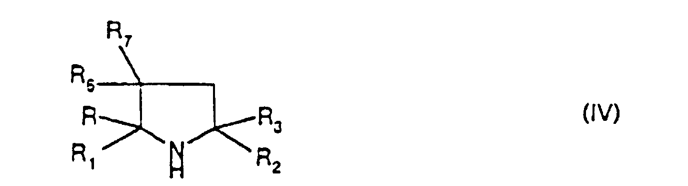 worin R₂ und R₇ wie vorstehend definiert sind und R, R₁, R₃ und R₆ wie in Anspruch 1 definiert sind, mit einer Säure der Formel: 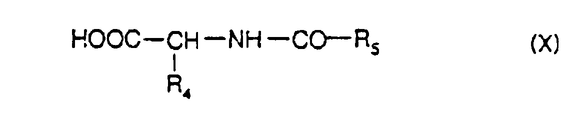 umsetzt, worin R₅ wie vorstehend definiert ist, oder mit einem reaktiven Derivat dieser Säure umsetzt, und R₄, wie in Anspruch 1 definiert ist, das Produkt isoliert und gegebenenfalls in ein Salz überführt.

13. Verfahren zur Herstellung von Verbindungen der Formel (I) nach Anspruch 1, in denen R₇ einen Phenylsulfonylrest bedeutet, dessen Phenylkern durch einen Aminorest substituiert ist, dadurch **gekennzeichnet**, daß man eine entsprechende Verbindung der Formel (I), in der R₇ einen Phenylsulfonylrest bedeutet, dessen Phenylrest durch eine Nitrogruppe substituiert ist, reduziert, das Produkt isoliert und gegebenenfalls in ein Salz überführt.

14. Verfahren zur Herstellung von Verbindungen der Formel (I) nach Anspruch 1, in denen R₇ einen Phenylsulfonylrest bedeutet, dessen Phenylkern durch eine Monomethylaminogruppe substituiert ist, dadurch **gekennzeichnet,** daß man eine entsprechende Verbindung der Formel (I), in der R₇ einen Phenylsulfonylrest bedeutet, dessen Phenylkern durch eine Aminogruppe substituiert ist, methyliert, das Produkt isoliert und gegebenenfalls in ein Salz überführt.

15. Medikamente, enthaltend als Wirkstoff mindestens eine Verbindung der Formel (I) nach einem der Ansprüche 1 bis 6.

16. Medikamente nach Anspruch 15, die zur Behandlung und Verhütung von Störungen im Zusammenhang mit CCK und Gastrin verwendbar sind.

## Claims

1. Compounds of formula: 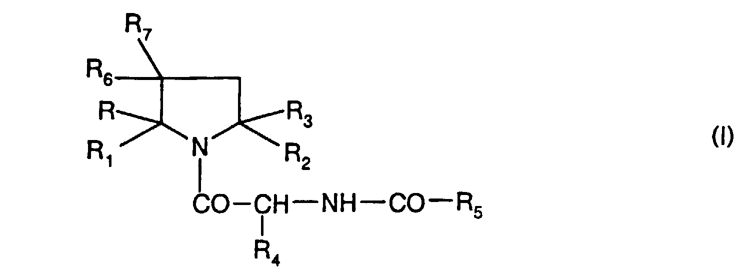 in which
R represents an alkyl radical containing 1 to 12 carbon atoms, in a straight or branched chain and optionally mono- or polyunsaturated, a cycloalkyl radical containing 3 to 12 carbon atoms and optionally mono- or polyunsaturated, a polycycloalkyl radical containing 6 to 12 carbon atoms and optionally mono- or polyunsaturated, a phenylalkyl radical in which the phenyl ring is optionally substituted (with one or more substituents chosen from alkyl and alkoxy radicals or halogen atoms), a diphenylalkyl or cinnamyl radical, a pyridyl radical optionally substituted with one or more alkyl radicals, a furyl radical optionally substituted with one or more alkyl radicals, a thienyl radical optionally substituted with one or more alkyl radicals, a quinolyl radical optionally substituted with one or more alkyl radicals, a naphthyl radical optionally substituted with one or more alkyl radicals, an indolyl radical optionally substituted with one or more alkyl radicals or a phenyl radical optionally substituted with one or more substituents chosen from halogen atoms and alkyl, alkoxy, hydroxyl, nitro, amino, monoalkylamino, dialkylamino, alkoxycarbonyl, -CO-NR₈R₉, -NH-CO-CH₃, trifluoromethyl and trifluoromethoxy radicals,
R₁ represents a hydrogen atom or an alkyl radical,
R₂ represents a chain -(CH₂)ₙ-CO-R₁₀, -(CH₂)ₙ-O-CO-R₁₁ or - (CH₂)ₘ-NR₁₂R₁₃ or an oxazolinyl radical optionally substituted with one or more alkyl radicals or a 3-alkyl-oxadiazolyl radical,
R₃ represents a hydrogen atom or an alkyl radical,
R₄ represents a hydrogen atom or an alkyl radical,
R₅ represents a phenyl radical (optionally substituted with one or more substituents chosen from halogen atoms and alkyl, alkoxy and alkylthio radicals), a naphthyl, indolyl or quinolyl radical or a phenylamino radical in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH) -CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 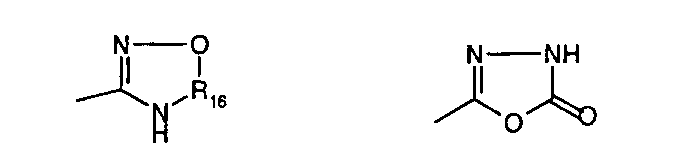 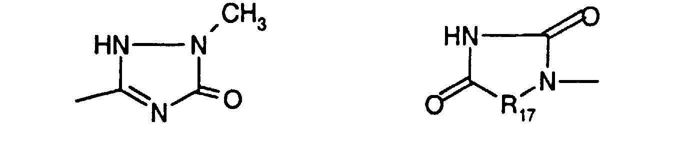 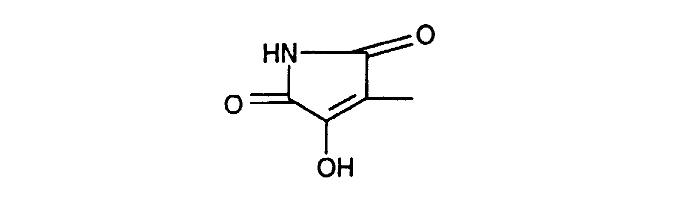 or 2,2-dimethyl-4,6-dioxo-1,3-dioxan-5-yl radicals,
R₆ represents a hydrogen atom or an alkyl or phenylalkyl radical,
R₇ represents an alkylsulphonyl radical, a radical -SO₂-NR₈R₉ or a phenylsulphonyl radical in which the phenyl ring is substituted with one or more substituents chosen from halogen atoms and alkyl, alkoxy, hydroxyl, nitro, amino, monoalkylamino, dialkylamino, acylamino, trifluoromethyl and trifluoromethoxy radicals,
R₈ represents a hydrogen atom or an alkyl or phenylalkyl radical or a phenyl radical which is optionally substituted with one or more substituents chosen from halogen atoms and alkyl, alkoxy and alkylthio radicals,
R₉ represents an alkyl or phenylalkyl radical or a phenyl radical which is optionally substituted with one or more substituents chosen from halogen atoms and alkyl, alkoxy and alkylthio radicals,
or alternatively R₈ and R₉ form, together with the nitrogen atom to which they are attached, a saturated or unsaturated mono- or polycyclic heterocycle containing 4 to 9 carbon atoms and one or more hetero atoms (O, N) and is optionally substituted with one or more alkyl radicals,
R₁₀ represents a hydroxyl, alkoxy, cycloalkyloxy, cycloalkylalkyloxy, phenyl or -NR₁₂R₁₃ radical,
R₁₁ represents an alkoxy, cycloalkyloxy, cycloalkylalkyloxy, phenyl or -NR₁₂R₁₃ radical,
R₁₂ represents a hydrogen atom or an alkyl, cycloalkylalkyl, cycloalkyl or phenylalkyl radical or a phenyl radical which is optionally substituted with one or more substituents chosen from halogen atoms and alkyl, alkoxy and alkylthio radicals,
R₁₃ represents an alkyl, cycloalkylalkyl, cycloalkyl or phenylalkyl radical or a phenyl radical which is optionally substituted with one or more substituents chosen from halogen atoms and alkyl, alkoxy and alkylthio radicals,
or alternatively R₁₂ and R₁₃ form, together with the nitrogen atom to which they are attached, a saturated or unsaturated mono- or polycyclic heterocycle containing 4 to 9 carbon atoms and one or more hetero atoms (O, N, S) and optionally substituted with one or more alkyl radicals,
R₁₄ represents an alkyl, cycloalkyl or trifluoromethyl radical or a phenyl radical which is optionally substituted with one or more substituents chosen from cyano, alkoxy, nitro and amino radicals and halogen atoms,
R₁₅ represents a 5-tetrazolyl radical,
R₁₆ represents C=O or S=O,
R₁₇ represents O or C=O,
n is equal to 0, 1 or 2,
m is equal to 1 or 2,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
it being understood that, except where otherwise mentioned, the alkyl, alkylene and alkoxy radicals and the alkyl, alkylene and alkoxy portions contain 1 to 4 carbon atoms in a straight or branched chain, the acyl radicals or portions contain 2 to 4 carbon atoms and the cycloalkyl radicals and portions contain 3 to 6 carbon atoms,
the racemic mixtures and the enantiomers of these compounds and the salts thereof.

2. Compounds of formula (I) according to Claim 1, for which R represents an isopropylidene, cyclohexyl, tetrahydrophenyl, cyclopentadienyl, dihydrophenyl, norbornyl, adamantyl or norbornenyl radical, the racemic mixtures and the enantiomers of these compounds and the salts thereof.

3. Compounds of formula (I) according to Claim 1, for which, when R₈ and R₉ form, together with the nitrogen atom to which they are attached, a heterocycle, this heterocycle is a piperidino ring optionally substituted with one or more alkyl radicals, a morpholino ring or a 1,2,3,4-tetrahydroquinoline ring system, the racemic mixtures and the enantiomers of these compounds and the salts thereof.

4. Compounds of formula (I) according to Claim 1, for which, when R₁₂ and R₁₃ form, together with the nitrogen atom to which they are attached, a heterocycle, this heterocycle is a piperidino, perhydro-1-azepinyl, 1,2,3,6-tetrahydro-1-pyridyl, 1,2,3,4-tetrahydro-1-quinolyl, 1-pyrrolidinyl, 1,2,3,4-tetrahydro-2-isoquinolyl, thiomorpholino or 1-indolinyl ring system, it being possible for these ring systems to be optionally substituted with at least one alkyl radical, the racemic mixtures and the enantiomers of these compounds and the salts thereof.

5. Compounds of formula (I) according to Claim 1, for which
R represents phenyl, optionally substituted with one or more substituents chosen from halogen atoms and alkyl, alkoxy, hydroxyl, nitro, amino, monoalkylamino, dialkylamino, alkoxycarbonyl, -CO-NR₈R₉, -NH-CO-CH₃, trifluoromethyl or trifluoromethoxy radicals,
R₁ represents a hydrogen atom,
R₂ represents a chain -(CH₂)ₙ-CO-R₁₀,
R₃ represents a hydrogen atom,
R₄ represents a hydrogen atom,
R₅ represents a phenylamino radical in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=NO-alk-COOX, -alk-N(OH)-CO-alk, -alk-SO₂H, - SO₂-NH-CO-R₁₄, -SO₂- NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 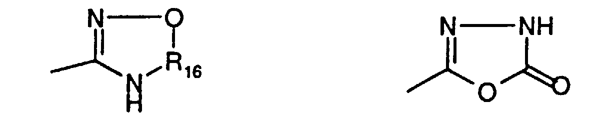 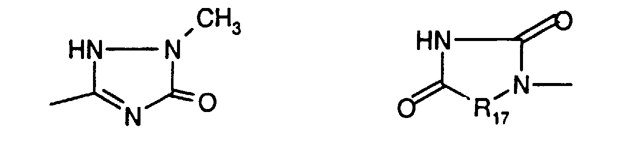 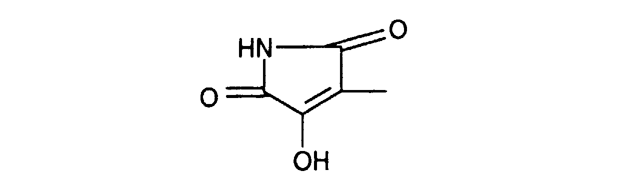 or 2,2-dimethyl-4,6-dioxo-1,3-dioxan-5-yl radicals,
R₆ represents a hydrogen atom,
R₇ represents an alkylsulphonyl radical, a radical -SO₂-NR₈R₉ or a phenylsulphonyl radical in which the phenyl ring is substituted with one or more substituents chosen from halogen atoms and alkyl, alkoxy, hydroxyl, nitro, amino, monoalkylamino, dialkylamino, acylamino, trifluoromethyl and trifluoromethoxy radicals,
R₈ represents a hydrogen atom or an alkyl or phenylalkyl radical or a phenyl radical which is optionally substituted with one or more substituents chosen from halogen atoms and alkyl, alkoxy and alkylthio radicals,
R₉ represents an alkyl or phenylalkyl radical or a phenyl radical which is optionally substituted with one or more substituents chosen from halogen atoms and alkyl, alkoxy and alkylthio radicals,
or alternatively R₈ and R₉ form, together with the nitrogen atom to which they are attached, a saturated or unsaturated mono- or polycyclic heterocycle containing 4 to 9 carbon atoms and one or more hetero atoms (O, N) and optionally substituted with one or more alkyl radicals,
R₁₀ represents a hydroxyl or alkoxy radical,
R₁₄ represents an alkyl, cycloalkyl or trifluoromethyl radical or a phenyl radical which is optionally substituted with one or more substituents chosen from cyano, alkoxy, nitro and amino radicals and halogen atoms,
R₁₅ represents a 5-tetrazolyl radical,
R₁₆ represents C=O or S=O,
R₁₇ represents 0 or C=O,
n is equal to 0, 1 or 2,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
the racemic mixtures and the enantiomers of these compounds, as well as the salts thereof.

6. Compounds chosen from
(2RS,4SR,5RS)-3-(3-{2-[2-tert-butoxycarbonyl-5-(2-fluorophenyl)-4-(4-chlorophenyl)sulphonyl-1-pyrrolidinyl]-2-oxoethyl}ureido)phenylacetic acid,
(2RS,4SR,5RS)-3-(3-{2-[2-tert-butoxycarbonyl-5-(2-fluorophenyl)-4-(2-fluorophenyl)sulphonyl-1-pyrrolidinyl]-2-oxoethyl}ureido)benzoic acid,
(2RS,4SR,5RS)-3-(3-{2-[2-tert-butoxycarbonyl-5-(2-fluorophenyl)-4-(3-methoxyphenyl)sulphonyl-1-pyrrolidinyl]-2 -oxoethyl}ureido) benzoic acid,
(2RS,4SR,5RS)-3-(3-{2-[2-tert-butoxycarbonyl-5-(2-fluorophenyl)-4-(4-methylphenyl)sulphonyl-1-pyrrolidinyl]-2-oxoethyl}ureido)benzoic acid,
(2RS,4SR,5RS)-3-(3-{2-[2-tert-butoxycarbonyl-5-(2-fluorophenyl)-4-(4-nitrophenyl)sulphonyl-1-pyrrolidinyl]-2-oxoethyl}ureido)benzoic acid,
(2RS,4SR,5RS)-3-(3-{2-[4-(4-aminophenyl)sulphonyl-2-tert-butoxycarbonyl-5-(2-fluorophenyl)-1-pyrrolidinyl]-2-oxoethyl}ureido)benzoic acid,
(2RS,4SR,5RS)-3-(3-{2-[4-(4-acetamidophenyl)sulphonyl-2-tert-butoxycarbonyl-5-(2-fluorophenyl)-1-pyrrolidinyl]-2-oxoethyl}ureido)benzoic acid,
(2RS,45R,5RS)-3-(3-{2-[2-tert-butoxycarbonyl-4-(4-dimethylaminophenyl)sulphonyl-5-(2-fluorophenyl)-1-pyrrolidinyl]-2-oxoethyl}ureido)benzoic acid,
(2RS,4SR,5RS)-3-(3-{2-[2-tert-butoxycarbonyl-5-(2-fluorophenyl)-4-methylsulphonyl-1-pyrrolidinyl]-2-oxoethyl}ureido)benzoic acid,
(2RS,4SR,5RS)-3-(3-{2-[4-(4-chlorophenyl)sulphonyl-5-(2-fluorophenyl)-2-isobutylcarbamoyl-1-pyrrolidinyl]-2-oxoethyl}ureido)phenylacetic acid,
(2RS,4SR,5RS)-3-(3 (2-[5-(2-fluorophenyl)-4-(2-fluorophenyl)sulphonyl-2-isobutylcarbamoyl-1-pyrrolidinyl]-2-oxoethyl}ureido)benzoic acid,
(2RS,4SR,5RS)-3-(3-{2-[5-(2-fluorophenyl)-2-isobutylcarbamoyl-4-(3-methoxyphenyl)sulphonyl-1-pyrrolidinyl]-2-oxoethyl}ureido)benzoic acid,
(2RS,4SR,5RS)-3-(3-{2-[5-(2-fluorophenyl)-2-isobutylcarbamoyl-4-(4-methylphenyl)sulphonyl-1-pyrrolidinyl]-2-oxoethyl}ureido)benzoic acid,
(2RS,4SR,5RS)-3-(3-{2-[5-(2-fluorophenyl)-2-isobutylcarbamoyl-4-(4-nitrophenyl)sulphonyl-1-pyrrolidinyl]-2-oxoethyl}ureido)benzoic acid,
(2RS,4SR,5RS)-3-(3-{2-[4-(4-aminophenyl)sulphonyl-5-(2-fluorophenyl)-2-isobutylcarbamoyl-1-pyrrolidinyl]-2-oxoethyl}ureido)phenylacetic acid,
(2RS,4SR,5RS)-3-(3-{2-[4-(4-acetamidophenyl)sulphonyl-5-(2-fluorophenyl)-2-isobutylcarbamoyl-1-pyrrolidinyl]-2-oxoethyl}ureido)benzoic acid,
(2RS,4SR,5RS)-3-(3-{2-[2-tert-butoxycarbonyl-5-(2-fluorophenyl)-4-morpholinosulphonyl-1-pyrrolidinyl]-2-oxoethyl}ureido)benzoic acid,
as well as the salts thereof.

7. Process for the preparation of the compounds of formula (I) according to Claim 1, for which R₅ represents a phenylamino radical in which the phenyl ring is optionally substituted, characterized in that a reactive derivative of carbamic acid, optionally obtained in situ by the action of a reactive derivative of carbonic acid chosen from N,N'-carbonyldiimidazole, phosgene, diphosgene, triphosgene and p-nitrophenyl chloroformate on a derivative of formula: 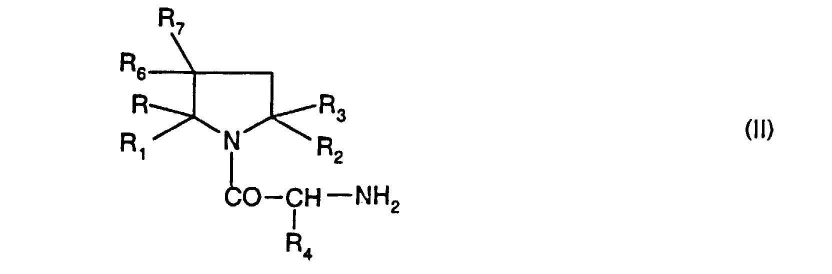 in which R, R₁, R₂, R₃, R₄, R₆ and R₇ have the same meanings as in Claim 1, is reacted with an aniline in which the phenyl ring is optionally substituted with one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H (in salt form), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₄, -SO₂-NH-SO₂-R₁₄, -CO-NH-CO-R₁₄, -CO-NH-SO₂-R₁₄, -B(OH)₂, -C(NH₂)=NOH, -SO₂-NH-R₁₅, -CO-NH-R₁₅, 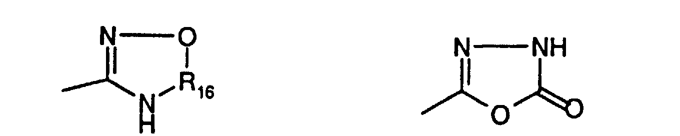 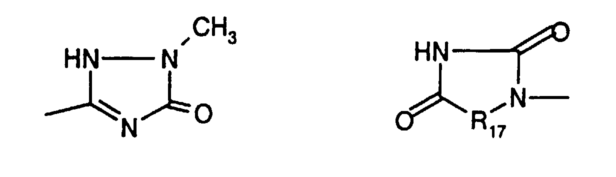 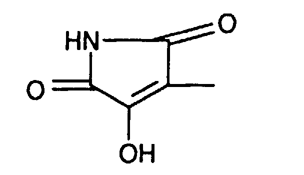 or 2,2-dimethyl-4,6-dioxo-1,3-dioxan-5-yl radicals, alk, alk', X, R₁₄, R₁₅, R₁₆ and R₁₇ having the same meanings as in Claim 1, and the product is isolated and optionally converted into a salt.

8. Process for the preparation of the compounds of formula (I) according to Claim 1, for which R₅ represents a phenylamino radical in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is an alkyl or phenylalkyl radical, characterized in that a derivative of formula: 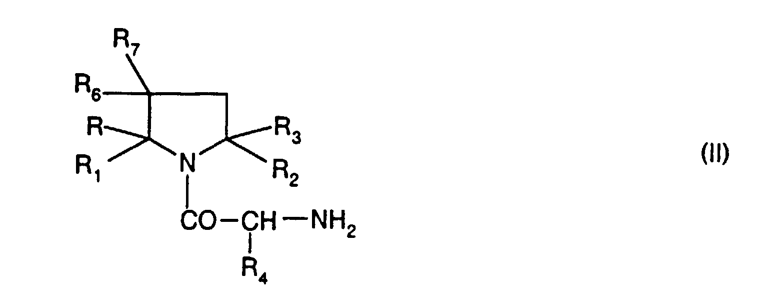 in which R, R₁, R₂, R₃, R₄, R₆ and R₇ have the same meanings as in Claim 1, is reacted with a phenyl isocyanate in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is an alkyl or phenylalkyl radical, and alk and alk' have the same meanings as in formula (I), and the product is isolated and optionally converted into a salt.

9. Process for the preparation of the compounds of formula (I) according to Claim 1, for which R₅ represents a phenyl radical which is optionally substituted or a naphthyl, indolyl or quinolyl radical, characterized in that a derivative of formula: 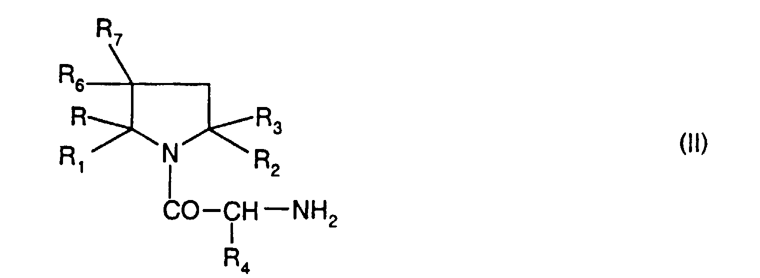 in which R, R₁, R₂, R₃, R₄, R₆ and R₇ have the same meanings as in Claim 1, is reacted with an acid of formula HOOC-R₅ in which R₅ has the same meanings as above, or a reactive derivative of this acid, and the product is isolated and optionally converted into a salt.

10. Process for the preparation of the compounds of formula (I) for which R₅ represents a phenylamino radical in which the phenyl ring is substituted with a carboxyl radical or an -alk-COOX, -O-alk-COOX, -alk'-COOX, -CH=CH-COOX, -CO-COOX, -S-alk-COOX, -SO-alk-COOX, -SO₂-alk-COOX, -C(=NOH)-COOX, -O-CH₂-alk'-COOX or -CX=N-O-alk-COOX radical and X represents a hydrogen atom, characterized in that a corresponding compound of formula (I), for which X is an alkyl or phenylalkyl radical, is hydrolysed or hydrogenolysed, and the product is isolated and optionally converted into a salt.

11. Process for the preparation of the compounds of formula (I) according to Claim 1 for which R₅ represents a phenylamino radical in which the phenyl ring is substituted with a hydroxyiminoalkyl or alkoxyiminoalkyl radical, characterized in that a corresponding compound of formula (I), for which R₅ represents a phenylamino radical in which the phenyl ring is substituted with an acyl radical, is reacted with a derivative of formula:
H₂N-OR₂₀ (IX)
in which R₂₀ represents a hydrogen atom or an alkyl radical, and the product is isolated and optionally converted into a salt.

12. Process for the preparation of the compounds of formula (I) according to Claim 1, for which R₂ represents a chain -(CH₂)ₙ-CO-R₁₀, R₁₀ represents an alkoxy, cycloalkyloxy, cycloalkylalkyloxy or phenyl radical or a radical -NR₁₂R₁₃, R₇ represents an alkylsulphonyl radical, a radical -SO₂-NR₈R₉ or a phenylsulphonyl radical in which the phenyl ring is substituted with one or more substituents chosen from halogen atoms and alkyl, alkoxy, hydroxyl, nitro, amino, monoalkylamino, dialkylamino, acylamino, trifluoromethyl and trifluoromethoxy radicals and R₅ represents a phenyl radical which is optionally substituted, a naphthyl, indolyl or quinolyl radical or phenylamino radical in which the phenyl ring is optionally substituted with one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, tri-fluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is an alkyl or phenylalkyl radical, characterized in that a derivative of formula: 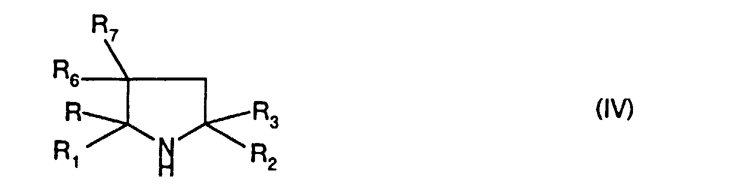 in which R₂ and R₇ have the same meanings as above and R, R₁, R₃ and R₆ have the same meanings as in Claim 1, is reacted with an acid of formula 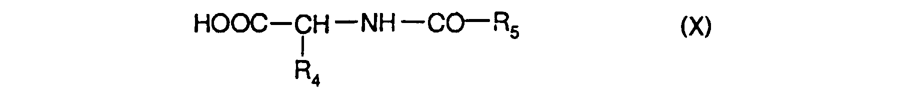 in which R₅ has the same meanings as above, or a reactive derivative of this acid, and R₄ has the same meanings as in Claim 1, and the product is isolated and optionally converted into a salt.

13. Process for the preparation of the compounds of formula (I) according to Claim 1, for which R₇ represents a phenylsulphonyl radical in which the phenyl is substituted with an amino radical, characterized in that a corresponding compound of formula (I), for which R₇ represents a phenylsulphonyl radical in which the phenyl is substituted with a nitro radical, is reduced, and the product is isolated and optionally converted into a salt.

14. Process for the preparation of the compounds of formula (I) according to Claim 1, for which R₇ represents a phenylsulphonyl radical in which the phenyl is substituted with a monomethylamino radical, characterized in that a corresponding compound of formula (I), for which R₇ represents a phenylsulphonyl radical in which the phenyl is substituted with an amino radical, is methylated, and the product is isolated and optionally converted into a salt.

15. Medicinal products containing at least one compound of formula (I) according to one of Claims 1 to 6 as active principle.

16. Medicinal products according to Claim 15, which may be used for the treatment or prevention of disorders linked to CCK and to gastrin.
